# EUROPÄISCHE PATENTSCHRIFT

(11) **EP 3 380 618 B1**
(45) Veröffentlichungstag und Bekanntmachung des Hinweises auf die Patenterteilung: **24.08.2022**
(21) Anmeldenummer: 16804741.3
(22) Anmeldetag: 26.11.2016
(51) Int. Cl.: C12N 15/82, C12Q 1/6895, C07K 14/415, A01H 5/10

(54) **KÜHLETOLERANTE PFLANZE**
PLANT WITH TOLERANCE OF THE COLD
PLANTE RUSTIQUE

(30) Priorität: 27.11.2015 EP 15196721
(43) Veröffentlichungstag der Anmeldung: 03.10.2018
(73) Patentinhaber: KWS SAAT SE & Co. KGaA, 37574 Einbeck (DE)
(72) Erfinder: OUZUNOVA, Milena, 37077 Göttingen (DE); PRESTERL, Thomas, 37574 Einbeck (DE); KNAAK, Carsten, 37079 Göttingen (DE); SCHEUERMANN, Daniela, 37574 Einbeck (DE); URBANY, Claude, 37574 Einbeck (DE); WESTHOFF, Peter, 41468 Neuss (DE); PESTSOVA, Elena, 42115 Wuppertal (DE); ERNST, Karin, 40225 Düsseldorf (DE)
(86) Internationale Anmeldenummer: PCT/EP2016/078920
(87) Internationale Veröffentlichungsnummer: WO 2017/089601

(56) Entgegenhaltungen:
- WO-A1-2008/034648
- WO-A1-2014/160304
- US-A1- 2007 044 171
- Nino Baliashvili: "Feinkartierung eines QTL (Quantitative Trait Locus) für Kühletoleranz auf Chromosom 4 in Mais und dessen molekularbiologische und phänotypische Charakterisierung. Inaugural-Dissertation zur Erlangung des Doktorgrades der Mathematisch-Naturwissenschaftlichen Fakultät der Heinrich-Heine-Universität", , Januar 2011 (2011-01), Seiten I-V, 1-96, XP55273945, Düsseldorf, Germany Gefunden im Internet: URL:http://docserv.uni-duesseldorf.de/serv lets/DerivateServlet/Derivate-22792/Doktor arbeit_Nino Baliashvili.pdf [gefunden am 2016-05-20]
- VICTOR M. RODRIGUEZ ET AL: "Identification of quantitative trait loci involved in the response to cold stress in maize (Zea mays L.)", MOLECULAR BREEDING: NEW STRATEGIES IN PLANT IMPROVEMENT., Bd. 33, Nr. 2, 20. September 2013 (2013-09-20), Seiten 363-371, XP55274060, NL ISSN: 1380-3743, DOI: 10.1007/s11032-013-9955-4
- THOMAS PRESTERL ET AL: "Quantitative trait loci for early plant vigour of maize grown in chilly environments", THEORETICAL AND APPLIED GENETICS ; INTERNATIONAL JOURNAL OF PLANT BREEDING RESEARCH, SPRINGER, BERLIN, DE, Bd. 114, Nr. 6, 6. März 2007 (2007-03-06), Seiten 1059-1070, XP019510483, ISSN: 1432-2242, DOI: 10.1007/S00122-006-0499-4
- SOBKOWIAK ALICJA ET AL: "Genome-wide transcriptomic analysis of response to low temperature reveals candidate genes determining divergent cold-sensitivity of maize inbred lines", PLANT MOLECULAR BIOLOGY, SPRINGER, DORDRECHT, NL, Bd. 85, Nr. 3, 13. März 2014 (2014-03-13), Seiten 317-331, XP035314577, ISSN: 0167-4412, DOI: 10.1007/S11103-014-0187-8 [gefunden am 2014-03-13]
- PEDRO REVILLA ET AL: "Cold Tolerance in Two Large Maize Inbred Panels Adapted to European Climates", JOURNAL OF PLANT REGISTRATIONS, Bd. 54, Nr. 5, 30. Juni 2014 (2014-06-30), Seiten 1981-1991, XP55599939, US ISSN: 1936-5209, DOI: 10.2135/cropsci2013.11.0733
- DATABASE EMBL [Online] 31. Oktober 2008 (2008-10-31), "Zea mays clone 213309 SAUR31 - auxin-responsive SAUR family member mRNA, complete cds.", XP002757915, gefunden im EBI accession no. EMBL:EU959260 Database accession no. EU959260 -& NICKOLAI N ALEXANDROV ET AL: "Insights into corn genes derived from large-scale cDNA sequencing", PLANT MOLECULAR BIOLOGY, KLUWER ACADEMIC PUBLISHERS, DORDRECHT, NL, Bd. 69, Nr. 1-2, 21. Oktober 2008 (2008-10-21), Seiten 179-194, XP019648179, ISSN: 1573-5028
- Nino Baliashvili: "Feinkartierung eines QTL (Quantitative Trait Locus) für Kühletoleranz auf Chromosom 4 in Mais und dessen molekularbiologische und phänotypische Charakterisierung. Inaugural-Dissertation zur Erlangung des Doktorgrades der Mathematisch-Naturwissenschaftlichen Fakultät der Heinrich-Heine-Universität", , Januar 2011 (2011-01), Seiten I-V, 1-96, XP055273945, Düsseldorf, Germany Gefunden im Internet: URL:http://docserv.uni-duesseldorf.de/serv lets/DerivateServlet/Derivate-22792 [gefunden am 2016-05-20] in der Anmeldung erwähnt
- VÍCTOR M. RODRÍGUEZ ET AL: "Identification of quantitative trait loci involved in the response to cold stress in maize (Zea mays L.)", MOLECULAR BREEDING: NEW STRATEGIES IN PLANT IMPROVEMENT., Bd. 33, Nr. 2, 20. September 2013 (2013-09-20), Seiten 363-371, XP055274060, NL ISSN: 1380-3743, DOI: 10.1007/s11032-013-9955-4
- PHILIPP WECKWERTH ET AL: "Zm CPK1, a calcium-independent kinase member of the Zea mays CDPK gene family, functions as a negative regulator in cold stress signalling", PLANT CELL AND ENVIRONMENT, Bd. 38, Nr. 3, 22. August 2014 (2014-08-22), Seiten 544-558, XP055273960, GB ISSN: 0140-7791, DOI: 10.1111/pce.12414
- SOBKOWIAK ALICJA ET AL: "Genome-wide transcriptomic analysis of response to low temperature reveals candidate genes determining divergent cold-sensitivity of maize inbred lines", PLANT MOLECULAR BIOLOGY, SPRINGER, DORDRECHT, NL, Bd. 85, Nr. 3, 13. März 2014 (2014-03-13), Seiten 317-331, XP035314577, ISSN: 0167-4412, DOI: 10.1007/S11103-014-0187-8 [gefunden am 2014-03-13]
- ALEXANDER STRIGENS ET AL: "Association mapping for chilling tolerance in elite flint and dent maize inbred lines evaluated in growth chamber and field experiments", PLANT CELL AND ENVIRONMENT, Bd. 36, Nr. 10, 13. Mai 2013 (2013-05-13), Seiten 1871-1887, XP055274058, GB ISSN: 0140-7791, DOI: 10.1111/pce.12096
- YUZHU CHEN ET AL: "Small auxin upregulated RNA ( SAUR ) gene family in maize: Identification, evolution, and its phylogenetic comparison with Arabidopsis , rice, and sorghum", JOURNAL OF INTEGRATIVE PLANT BIOLOGY, Bd. 56, Nr. 2, 8. November 2013 (2013-11-08), Seiten 133-150, XP055274021, GB ISSN: 1672-9072, DOI: 10.1111/jipb.12127
- A. MAROCCO ET AL: "CHILLING STRESS IN MAIZE", MAYDICA, Bd. 50, 2005, Seiten 571-580, XP055270513,
- JOHN A GREAVES: "Improving suboptimal temperature tolerance in maize-the search for variation", JOURNAL OF EXPERIMENTAL BOTANY, Bd. 47, Nr. 296, März 1996 (1996-03), Seiten 307-323, XP055273961, GB ISSN: 0022-0957
- CAI GUOHUA ET AL: "ZmMKK1, a novel group A mitogen-activated protein kinase kinase gene in maize, conferred chilling stress tolerance and was involved in pathogen defense in transgenic tobacco", PLANT SCIENCE, Bd. 214, 4. Oktober 2013 (2013-10-04), Seiten 57-73, XP028780024, ISSN: 0168-9452, DOI: 10.1016/J.PLANTSCI.2013.09.014
- Nino Baliashvili: "Feinkartierung eines QTL (Quantitative Trait Locus) für Kühletoleranz auf Chromosom 4 in Mais und dessen molekularbiologische und phänotypische Charakterisierung. Inaugural-Dissertation zur Erlangung des Doktorgrades der Mathematisch-Naturwissenschaftlichen Fakultät der Heinrich-Heine-Universität", , January 2011 (2011-01), pages I-V, 1-96, XP55273945, Düsseldorf, Germany Retrieved from the Internet: URL:http://docserv.uni-duesseldorf.de/serv lets/DerivateServlet/Derivate-22792/Doktor arbeit_Nino Baliashvili.pdf [retrieved on 2016-05-20]
- VICTOR M. RODRIGUEZ ET AL: "Identification of quantitative trait loci involved in the response to cold stress in maize (Zea mays L.)", MOLECULAR BREEDING: NEW STRATEGIES IN PLANT IMPROVEMENT., vol. 33, no. 2, 20 September 2013 (2013-09-20), pages 363-371, XP55274060, NL ISSN: 1380-3743, DOI: 10.1007/s11032-013-9955-4
- THOMAS PRESTERL ET AL: "Quantitative trait loci for early plant vigour of maize grown in chilly environments", THEORETICAL AND APPLIED GENETICS ; INTERNATIONAL JOURNAL OF PLANT BREEDING RESEARCH, SPRINGER, BERLIN, DE, vol. 114, no. 6, 6 March 2007 (2007-03-06) , pages 1059-1070, XP019510483, ISSN: 1432-2242, DOI: 10.1007/S00122-006-0499-4
- SOBKOWIAK ALICJA ET AL: "Genome-wide transcriptomic analysis of response to low temperature reveals candidate genes determining divergent cold-sensitivity of maize inbred lines", PLANT MOLECULAR BIOLOGY, SPRINGER, DORDRECHT, NL, vol. 85, no. 3, 13 March 2014 (2014-03-13) , pages 317-331, XP035314577, ISSN: 0167-4412, DOI: 10.1007/S11103-014-0187-8 [retrieved on 2014-03-13]
- PEDRO REVILLA ET AL: "Cold Tolerance in Two Large Maize Inbred Panels Adapted to European Climates", JOURNAL OF PLANT REGISTRATIONS, vol. 54, no. 5, 30 June 2014 (2014-06-30), pages 1981-1991, XP55599939, US ISSN: 1936-5209, DOI: 10.2135/cropsci2013.11.0733
- Pedro Revilla ET AL: "Cold Tolerance in Two Large Maize Inbred Panels Adapted to European Climates", Journal of Plant Registrations, vol. 54, no. 5, 1 January 2014 (2014-01-01), page 1981, XP055599939, ISSN: 1936-5209, DOI: 10.2135/cropsci2013.11.0733

## Beschreibung

### Gebiet der Erfindung

Die vorliegende Erfindung betrifft das Gebiet der Modifikation von Pflanzen mittels molekularbiologischer Methoden und Markertechnologie und der Gentechnik. Sie betrifft eine neue kühletolerante Pflanze, insbesondere eine Maispflanze, sowie die Identifizierung und molekulare Charakterisierung sowie die Verwendung von Genen und Markern aus einem chromosomalen Intervall von 25,7 kb, der in Maislinien einen Lokus zur Kälte/Kühletoleranz enthält. Ein weiterer Aspekt der Erfindung betrifft die Entwicklung von molekularen Markern zur Unterstützung bei der Züchtung, insbesondere zur Vermeidung einer Fixierung eines "selective sweep" in einer Region mit geringer Rekombinationsrate.

### Hintergrund der Erfindung

Der Ausdruck "Kühle" betrifft Temperaturen bei denen die Maispflanze überleben kann, jedoch das Wachstum beeinträchtigt oder sogar erheblich beeinträchtigt ist. Die optimale Wachstumstemperatur für die Keimung von Maissamen und die Entwicklung von Maispflanzen liegt zwischen 21-27 °C (Greaves JA (1996) Improving suboptimal temperature tolerance in maizethe search for variation. J Exp Bot 47: 307-323, 1996).

Daher tritt Stress bereits bei Temperaturen von unterhalb von 20 °C auf, was eine typische bei der Pflanzzeit gemessene Temperatur in Nordeuropa ist. Milder Kühlestress mit verringerter Photosynthese im Licht und verringertem Wachstum wird bei 12-17 °C gefunden, und starker Kühlestress tritt zusammen mit Kühle-induziertem Wasserstress, einer Art von Trockenstress, in Licht bei 2-10 °C auf (Marocco A., Lorenzoni C and Fracheboud Y, 2005. Chilling stress in maize. Maydica, 50: 571-580).

Kühlestress geht entweder mit Photoinhibierung und oxidativem Stress im Licht oder Genexpressionsveränderungen im Dunklen einher (zusammengefasst in Marocco et al., 2005). Am empfindlichsten ist die heterotrophe Phase (Aussaat bis zum dritten Blatt), jedoch beeinträchtigt Kühlestress auch die frühe autotrophe Phase (Bhosale et al., 2007. Chilling Tolerance of Central European Maize Lines and their Factorial Crosses, Annals of Botany 100: 1315-1321). Ausgedehntes Einwirken von geringeren Temperaturen führt zu einer irreversiblen Verletzung der Zellen und des Gewebes (Greaves, 1996) und anschließendem verringertem Wachstum und Ertrag.

Frühes kräftiges Pflanzenwachstum wird als wichtiger Indikator für hohe und stabile Erträge beispielsweise im Mais angesehen, insbesondere im kühlen Klima von Zentral- und Nordeuropa. Zusätzlich führen auch Maissorten mit verbessertem frühem Pflanzenwachstum zu einer besseren Bodenabdeckung und helfen daher dabei, die Erosion und Nitratauswaschung am Anfang der Wachstumsphase zu verringern.

Mehrere QTL (Quantitative Trait Locus)-Untersuchungen wurden bereits durchgeführt, um eine genetische Kühletoleranz in Mais zu identifizieren. Die meisten der Studien analysierten Maispflanzen, die in Wachstumskammern unter optimalen (25/22 °C) und suboptimalen (15/13 °C) Bedingungen angezogen wurden. Dabei wurden Parameter, wie etwa die Quanteneffizienz des Photosystems II, die maximale Quanteneffizienz des Photosystems II, die Chlorophyllfluoreszenz, der Chlorophyllgehalt des dritten Blatts (SPAD), Blattfläche und Trockengewicht des Keimlings gemessen (Fracheboud, Y., et al. "Identification of quantitative trait loci for cold-tolerance of photosynthesis in maize (Zea mays L.)." Journal of experimental botany 53.376 (2002): 1967-1977; Fracheboud, Y., et al. "Genetic analysis of cold-tolerance of photosynthesis in maize." Plant molecular biology 56.2 (2004): 241-253; Hund, A., et al. "QTL controlling root and shoot traits of maize seedlings under cold stress." Theoretical and applied genetics 109.3 (2004): 618-629; Hund, Andreas, et al. "Cold tolerance of the photosynthetic apparatus: pleiotropic relationship between photosynthetic performance and specific leaf area of maize seedlings." Molecular Breeding 16.4 (2005): 321-331; Guerra-Peraza, Orlene, et al. "Temperature at night affects the genetic control of acclimation to cold in maize seedlings. "Maydica 56.4 (2012)), jedoch wurde in allen dieser Experimente kein QTL in der Nähe des hier auf Chromosom 4 beschriebenen und klonierten QTL dokumentiert. Leipner et al. (QTL studies reveal little relevance of chilling-related seedling traits for yield in maize. Theor Appl Genet (2008) 116:555-562) berichteten über ein QTL Kartierungsexperiment bei der Blüte- und Erntephase in einem Feldexperiment mit zwei zeitlich verschiedenen Aussaaten. Die gemessenen Parameter waren Blütezeit, Pflanzenhöhe, Stroh- und Ähren-Trockenmassegewicht, und die Autoren verglichen ihre identifizierten QTLs mit QTLs, die mittels Wachstumskammer-Experimenten in der Keimphase identifiziert wurden, wie durch Jompuk et al. (Mapping of quantitative trait loci associated with chilling tolerance in maize (Zea mays L.) seedlings grown under field conditions. Journal of experimental botany, 2005, 56. Jg., Nr. 414, S. 1153-1163.) veröffentlicht. Nur wenige gemeinsame QTLs wurden nachgewiesen. Leipner et al. folgerten daraus, dass die Kühletoleranz der Keimlinge scheinbar keinen signifikanten Effekt auf den Ertrag hat.

Jompuk et al. (2005) bestimmten den Kohlenstoffaustausch und die Chlorophyllfluoreszenz, die Betriebs-Quanteneffizienz von Photosystem II, die Grünfärbung des dritten Blattes (SPAD), Fläche des dritten Blattes und Trockenmasse des Keimlings in der gleichen Population, und kartierten einen QTL für SPAD bei früher Aussaat und die Betriebs-Quanteneffizienz von Photosystem II auf Chromosom 4 bei 31,1 Mb, was ungefähr 6 Mb von dem QTL der vorliegenden Erfindung an Position 37 Mb entfernt liegt.

Unter Verwendung von SSR-Markern kartierten Presterl et al. 2007 ("Quantitative trait loci for early plant vigour of maize grown in chilly environments." Theoretical and Applied Genetics 114.6 (2007): 1059-1070) auf Chromosom 4 einen QTL von 4 cM, welcher jedoch eine physische Größe von ca. 155 Mb umfasst, was nicht weniger als etwa 7 % des Gesamtgenoms im Mais ausmacht. Zwar wird von einer fortgesetzten Feinkartierungsstudie in Baliashvili in 2011 (Feinkartierung eines QTL (Quantitative Trait Locus) für Kühletoleranz auf Chromosom 4 in Mais und dessen molekularbiologische und phänotypische Charakterisierung. Diss. Universitäts-und Landesbibliothek der Heinrich-Heine-Universität Düsseldorf, 2011) berichtet, jedoch werden weder die Marker noch andere Sequenzinformationen dort offenbart.

Eine kürzlich durchgeführte QTL Studie wurde durch Rodriguez et al. (Effects of selection for color intensity on antioxidant capacity in maize (Zea mays L.). Euphytica, 2013, 193. Jg., Nr. 3, S. 339-345.) veröffentlicht. Die Nachkommen einer Kreuzung von Flint-Mais und Dent-Mais wurden unter kontrollierten Bedingungen evaluiert, wobei die Dent-Maislinie empfindlich gegenüber niedrigen Temperaturen reagierte und eine drastische Verringerung des Chlorophyllgehalts zeigte. Die Kontrolltemperaturen waren dabei auf 25/20 °C, und die kühlen Temperaturen auf 14/8 °C eingestellt. Die gemessenen Parameter waren Zahl an überlebenden Pflanzen unter Kontroll- und Kühlebedingungen, Trockengewicht des Keimlings unter Kontrollbedingungen, Quantenausbeute von Photosystem II bei Kontrollbedingungen und Gesamt-Anthocyaningehalt. Zwar überlappten vier von 10 nachgewiesenen QTL Regionen mit dem von Presterl et al 2007 kartierten QTL auf Chromosom 4, jedoch wurden keine weiteren Untersuchungen hinsichtlich der genetischen Grundlagen wie z.B. die Feinkartierung der Regionen oder das Identifizieren von Kandidatengenen durchgeführt.

Bei solchen Untersuchungen gilt grundsätzlich, dass durch die Analyse eines parenteralen Ursprungs von Allelen bei Markern, die einen Ziellokus flankieren, man Individuen mit einem kurzen intakten Donor-Chromosomensegment um das Zielgen herum selektieren und so den "linkage drag" reduzieren kann. Stam und Zeven (The theoretical proportion of the donor genome in near-isogenic lines of self-fertilizers bred by backcrossing. Euphytica, 1981, 30. Jg., Nr. 2, S. 227-238) zeigten jedoch, dass die zu erwartende Länge eines Donor-Chromosomsegments, das mit einem Zielgen gekoppelt ist, sogar nach sechs Generationen von Rückkreuzung und kombiniert mit Selektion auf das Zielgen noch 32 cM auf einem 100 cM Chromosom ist. Es finden sich Beispiele von negativen genetisch kodierten Eigenschaften, die mit einem Zielgen unter Selektion gekoppelt bleiben (Zeven, AC; Knott, DR; Johnson, R. Investigation of linkage drag in near isogenic lines of wheat by testing for seedling reaction to races of stem rust, leaf rust and yellow rust. Euphytica, 1983, 32. Jg., Nr. 2, S. 319-327).

Grundsätzlich ist Kühletoleranz in der Weiterentwicklung von Nutzpflanzen ein bedeutendes Merkmal. So sind bereits für andere Kulturarten Kühletoleranz-vermittelnde Gene bekannt. So beschrieben Ma et al. (in: COLD1 confers chilling tolerance in rice. Cell. 2015 Mar 12; 160(6):1209-21) beispielsweise den QTL COLD1 in Reis. Eine Überexpression von COLD1(jap) erhöht die Kühletoleranz signifikant. COLD1 kodiert für einen Regulator der G-Protein-Signalkaskade.

Weiterhin wird auch ein SNP in COLD1 beschrieben, genannt SNP2. Ebenso gilt die Kühletoleranz auch als ein wichtiges Ziel für die Silo-, Körner- und Energie-Maiszüchtung in zahlreichen Maisanbauregionen, insbesondere in den kühlen Bereichen Zentral- und Nordeuropas, aber auch Südeuropas, wo Landwirte Maispflanzen gerne früher aussäen würden, um die im Boden befindliche Feuchtigkeit des Winters besser auszunutzen. Die Auffindung und Charakterisierung von Kühletoleranz-vermittelnden Genen und die Bereitstellung von neuen Markern für Kühletoleranz in Pflanzen allgemein und besonders in Nutzpflanzen, sowie die Bereitstellung von Pflanzen mit einer gesteigerten Kühletoleranz, ohne dass diese Pflanzen zudem weitere agronomische oder züchterische Nachteile aufweisen, ist daher von besonderem Interesse und eine Aufgabe dieser Erfindung. Weitere Aspekte werden dem Fachmann bei Studium der folgenden Beschreibung und der Beispiele ersichtlich werden.

In einem ersten Aspekt der vorliegenden Anmeldung eine Nukleinsäure bereitgestellt, die eine Nukleinsäuresequenz aufweist, ausgewählt aus der Gruppe bestehend aus a) einer Nukleinsäuresequenz gemäß einer der SEQ ID NOs 29, 3, 7, 11, 15, 25 oder 35, oder einem funktionellen Fragment davon, b) einer Nukleinsäuresequenz, die zu einer Sequenz aus a) komplementär ist, c) einer Nukleinsäuresequenz, die zu mindestens 80%, 85%, 90% oder 95% oder bevorzugt zu mindestens 96%, 97%, 98%, oder 99% identisch ist mit einer Sequenz aus a) oder b), d) einer Nukleinsäuresequenz, die sich entsprechend des degenerierten genetischen Codes von einer Nukleinsäuresequenz gemäß a), b) oder c) ableitet, e) einer Nukleinsäuresequenz, die mit einer Nukleinsäuresequenz gemäß a), b) oder c) unter stringenten Bedingungen hybridisiert, f) einer Nukleinsäuresequenz, die für ein Protein gemäß einer der SEQ ID NOs 30, 4, 8, 12, 16 oder 26 oder ein Homolog, Analog oder Ortholog davon kodiert, oder g) einer Nukleinsäuresequenz, die für eine oder mehrere RNAs kodiert, die in der Lage ist/sind zumindest partiell mit sich selbst oder untereinander zu hybridisieren und dadurch einen doppelsträngigen Teil auszubilden, wobei diese Nukleinsäuresequenz über mindestens 19, 20, 21, 22, 23, 24 oder 25, bevorzugt mindestens 30, 32, 34, 36, 38 oder 40, besonders bevorzugt mindestens 50, 60, 70, 80, 90 oder 100, oder ganz besonders bevorzugt mindestens 150, 200, 250, 300, 400, 500, 750 oder 1000 aufeinanderfolgende Nukleotide übereinstimmt mit einer der Nukleinsäuresequenzen ausgewählt aus der Gruppe bestehend aus (i) einer Nukleinsäuresequenz gemäß einer der SEQ ID NOs: 27, 17, 19, 5, 7, 23 oder 25, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, (vi) einer Nukleinsäure sequenz, die für ein Protein gemäß einer der SEQ ID NOs: 28, 18, 20, 6, 8, 24 oder 26, oder ein Homolog, Analog oder Ortholog davon kodiert, oder vii) einer Nukleinsäuresequenz in Antisinnorientierung zu einer Nukleinsäuresequenz gemäß (i) bis (vi).

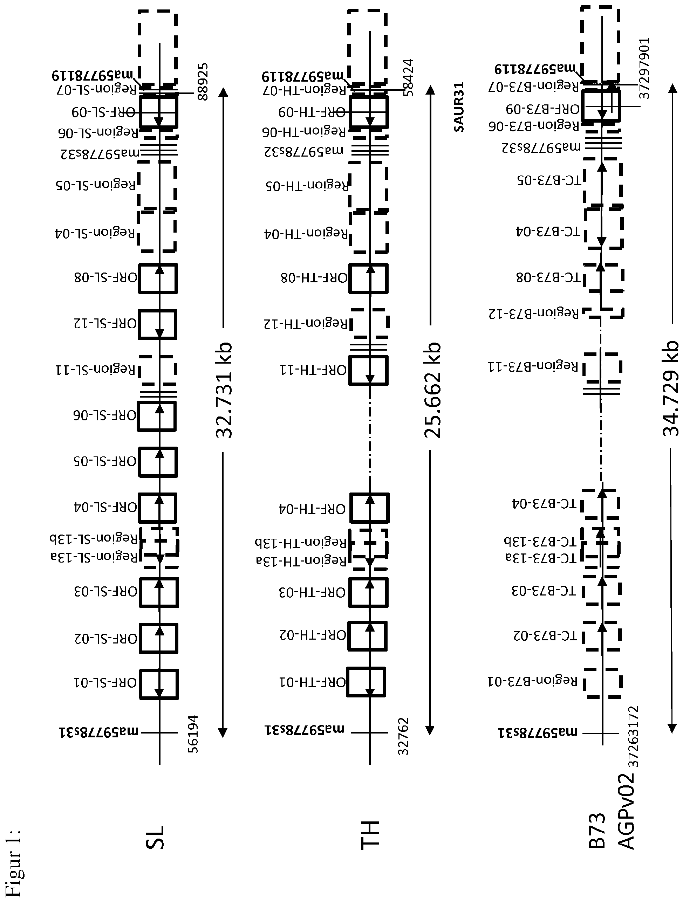

Die vorliegende Erfindung ist das Ergebnis von Bemühungen der Erfinder, einen Kühletoleranzquantitativen Markerlocus (quantitative trait locus (QTL)) auf Chromosom 4 von Mais präzise zu identifizieren und zu lokalisieren, diesen QTL zu klonieren und zu sequenzieren und die/das Gen(e) zu identifizieren, die für den Kühletoleranz-Phänotyp verantwortlich sind. Die identifizierte Region von insgesamt 25,7 kb (Donorgenotyp) wurde molekular untersucht, und geeignete Kandidatengene identifiziert (siehe Figur 1). Die durchgeführte funktionelle Validierung umfasste die

Identifizierung von Tilling-Mutanten und Genexpressionsstudien. Hierdurch wird die identifizierte Kühletoleranz züchterisch und gentechnisch erst nutzbar. Im Zuge der Kartierungs- und Feinkartierungarbeiten wurden spezielle molekulare Marker entwickelt, welche diagnostisch zur Selektion von Pflanzen mit gesteigerter Kühletoleranz einsetzbar sind. Weiterhin können die entwickelten Marker auch eingesetzt werden, um die genetische Quelle der gesteigerten Kühletoleranz in bereits vorhandenes Züchtungsmaterial (z.B. bestehende Sorten, Elitelinien etc.) gezielt einzukreuzen und dabei die Länge der erzeugten Introgression gering zu halten. Somit ist es erstmals möglich das Kühletoleranzmerkmal zu übertragen, ohne einen ausgedehnten genetischen Bereich der Zentromerregion von Chromosom 4 des Donors mitzuführen. Dies ist besonders vorteilhaft, da festgestellt wurde, dass dieser genetische Bereich stark fixiert ist und somit nur ein sehr geringes Maß an genetischer Diversität und Rekombinationsfrequenz zulässt. Würde man diesen genetischen Bereich, der immerhin mehr als 5% des Gesamtgenoms von Mais ausmacht, mitführen, würde dies die weitere züchterische Nutzbarkeit von Sorten mit diesem wichtigen agronomischen Merkmal drastisch einschränken. Die vorliegende Erfindung ermöglicht somit in vorteilhafter Weise erstmals die züchterische Nutzung des identifizierten Kühletoleranzmerkmals und reduziert gleichzeitig den Verlust der genetischen Diversität (selective sweep) auf ein Minimum.

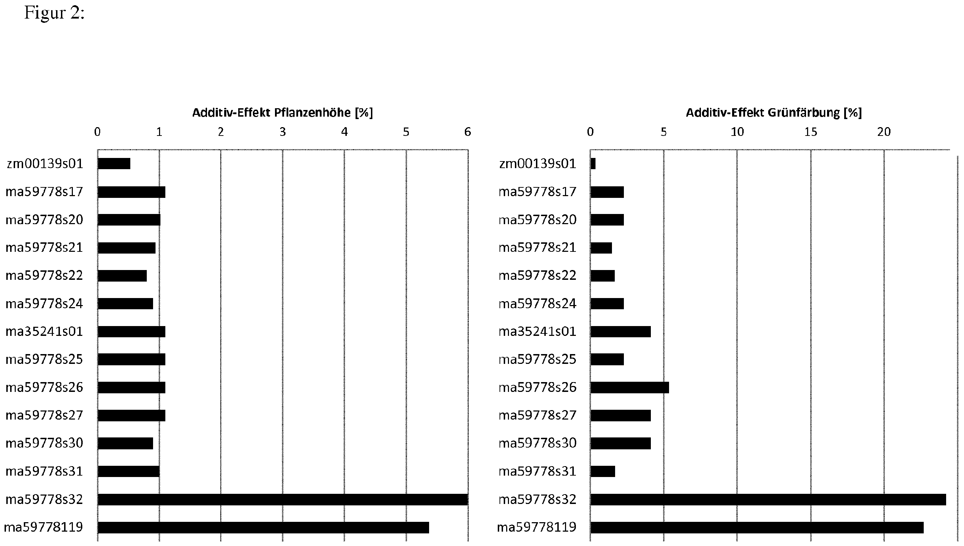

Als ein Ergebnis der durchgeführten Analysen im Rahmen der Erfindung konnte auch eine signifikante Korrelation phänotypischer Marker für Kühletoleranz mit der Region zwischen den Markern ma59778s31 und ma59778119 gefunden werden (siehe z.B. Figur 2). Das Auffinden eines QTL auf Chromosom 4 im Dentpool der 35% der phänotypischen Varianz für Kühletoleranz erklärt, stellt eine entscheidende Ressource zur züchterischen Nutzung dar.

Auf Grund der geringen Rekombinationsrate und der perizentromeren Lage des QTL war es vorliegend besonders schwierig, den ursächlichen Bereich auf das enge Intervall einzugrenzen und statt eines halben Chromosoms von 122 MB, auf dem noch weitere negative Merkmale weitervererbt wurden und die zu einem Verlust genetischer Diversität geführt hätten, in der Züchtung nur wenige kb einzukreuzen. Mit dem Wissen, um die genetischen Grundlagen des Kühletoleranzmerkmals ist nun auch deren alleinige Nutzung unter Vermeidung von jeglichem Linkage Drag und reduzierter genetischer Diversität möglich geworden.

In der Region um den besagten QTL konnten durch Vergleiche zwischen den verschiedenen Pflanzen und anschließende Datenbankvergleiche mehrere funktionelle Elemente, bzw. Gene, identifiziert werden (siehe Beispiele), die entsprechend allein oder in Kombination an der Kühletoleranz beteiligt sind. Die folgende Tabellen la, 1b und 1c führen diese auf.

**Tabelle la: Genetische Elemente und Gene in der 25,7 kb-Zielregion (TH Genotyp; ORF - Leseraster (open reading frame) wie identifiziert; SL - sensitive Linie; TH - tolerante Linie)**

| SEQ ID NO: | Bezeichnung | Position (SL) | Annotation |
|---|---|---|---|
| 29 | ORF TH-09 | 88663 | Auxin responsives SAUR Protein (SAUR31) |
| 3 | ORF TH-01 | 61977 | Retrotransposon gag Protein |
| 7 | ORF TH-02 | 63012 | Transposon Sb07g001920 aus *Sorghum bicolor* |
| 11 | ORF TH-03 | 65848 | Transposon Sb07g001880 aus *Sorghum bicolor* |
| 15 | ORF TH-04 | 70255 | Transposon Sb07g001880 aus *Sorghum bicolor* |
| 25 | ORF TH-08 | 80491 | Transposon Sb07g001900 aus *Sorghum bicolor* |
| 35 | ORF TH-11 | 79716 | Putatives Polyprotein, *Oryza sativa* ssp. japonica |
| | Region TH-12 | | Transposon Sb07g001920 aus *Sorghum bicolor* |

**Tabelle 1b: Genetische Elemente und Gene in der 25,7 kb-Zielregion, welche im TH Genotyp nicht vorhanden sind oder welche im TH Genotyp eine geänderte, vorzugsweise reduzierte Expression aufweisen (ORF - Leseraster (open reading frame) wie identifiziert; SL - sensitive Linie; TH - tolerante Linie).**

| SEQ ID NO: | Bezeichnung | Position (SL) | Annotation |
|---|---|---|---|
| 17 | ORF SL-05 | 71918 | Transponierbares Element, mögliches nicht charakterisiertes Protein, *Oryza sativa subsp. Japonica* |
| 19 | ORF SL-06 | 74307 | Retrotransposon, mögliches nicht charakterisiertes Protein OSJNBb0006B22.8, *Oryza sativa subsp. japonica* |
| 7 | ORF TH-02 | 63012 | Transposon Sb07g001920 aus *Sorghum bicolor* |
| 25 | ORF TH-08 | 80491 | Transposon Sb07g001900 aus *Sorghum bicolor* |
| 29 | ORF TH-09 | 88663 | Auxin responsives SAUR Protein (SAUR31) |

**Tabelle 1c: Genetische Elemente und Gene in der 25,7 kb-Zielregion (SL Genotyp; ORF - Leseraster (open reading frame) wie identifiziert; SL - sensitive Linie; TH - tolerante Linie)**

| SEQ ID NO: | Bezeichnung | Position (SL) | Annotation |
|---|---|---|---|
| 27 | ORF SL-09 | 88663 | Auxin responsives SAUR Protein (SAUR31) |
| 1 | ORF SL-01 | 61977 | Retrotransposon gag Protein |
| 5 | ORF SL-02 | 63012 | Transposon Sb07g001920 aus *Sorghum bicolor* |
| 9 | ORF SL-03 | 65848 | Transposon Sb07g001880 aus *Sorghum bicolor* |
| 13 | ORF SL-04 | 70255 | Transposon Sb07g001880 aus *Sorghum bicolor* |
| 17 | ORF SL-05 | 71918 | Transponierbares Element, mögliches nicht |
| | | | charakterisiertes Protein, *Oryza sativa subsp. Japonica* |
| 19 | ORF SL-06 | 74307 | Retrotransposon, mögliches nicht charakterisiertes Protein OSJNBb0006B22.8, *Oryza sativa subsp. japonica* |
| 23 | ORF SL-08 | 80491 | Transposon Sb07g001900 aus *Sorghum bicolor* |
| 27 | ORF SL-09 | 88663 | Auxin responsives SAUR Protein (SAUR31) |
| | Region SL-11 | 79716 | Putatives Polyprotein, *Oryza sativa* ssp. japonica |
| 21 | ORF SL-12 | | Transposon Sb07g001920 aus *Sorghum bicolor* |

Von besonderem Interesse im Rahmen der vorliegenden Erfindung und daher bevorzugt sind ORF-09 (SAUR31), ORF-08 und ORF-02, die unterschiedliche Expression zwischen den Linien SL und TH zeigten (siehe Tabelle 1b). Beispielsweise war die Expressionrate von ORF-09 unter Kühlestress höher in den kühlesensitiven Linien (SL) als in den kühletoleranten Linien (TH) und nahm über die Zeit hinweg ab.

Daten aus Analysen zu dem Gen ORF-09 bzw. die zugehörige Annotation SAUR31 gemäß SEQ ID NO: 27 (Nukleotidsequenz des SL-Genotyps), SEQ ID NO: 29 (Nukleotidsequenz des TH-Genotyps) und SEQ ID NO: 31 (Nukleotidsequenz des Maisgenom-Referenzlinie B73) erlaubten mit Hilfe von Markeranalysen und der Auswertung von Rekombinationshäufigkeiten in den unterschiedlichen Linien SL, TH und B73 (Figur 1) eine eindeutige Positionierung von SAUR31 innerhalb eines Abschnitts auf Chromosom 4 zwischen den Markern ma59778s31 und ma59778119, vorzugsweise zwischen ma59778s32 und ma59778119. Dabei ist SAUR31 ein Gen, welches für das gleichnamige Auxin-responsive Protein kodiert (vgl. SEQ ID NOs: 28, 30 und 32). Von SAUR Genen ist bekannt, dass sie an der Zellexpansion, der Auxin-vermittelten Signaltransduktion und der Wurzelmeristem-Entwicklung beteiligt sind und auch positive Regulatoren der Blattseneszenz sind (Xu, N.; Hagen, G; Guilfoyle, T. Multiple auxin response modules in the soybean SAUR 15A promoter. Plant Science, 1997, 126. Jg., Nr. 2, S. 193-201; Jain, M; Tyagi, AK; Khurana, JP. Genome-wide analysis, evolutionary expansion, and expression of early auxin-responsive SAUR gene family in rice (Oryza sativa). Genomics, 2006, 88. Jg., Nr. 3, S. 360-371; Jain, M; Khurana, JP. Transcript profiling reveals diverse roles of auxin-responsive genes during reproductive development and abiotic stress in rice. Febs Journal, 2009, 276. Jg., Nr. 11, S. 3148-3162; Spartz, AK, et al. The SAUR19 subfamily of SMALL AUXIN UP RNA genes promote cell expansion. The Plant Journal, 2012, 70. Jg., Nr. 6, S. 978-990; Hou et al.. SAUR36, a small auxin up RNA gene, is involved in the promotion of leaf senescence in Arabidopsis. Plant physiology, 2013, 161. Jg., Nr. 2, S. 1002-1009). SAUR Gene sind primäre Auxin-Antwortgene, die am Auxin-Signalweg beteiligt sind (Chen et al. Jun. Small auxin upregulated RNA (SAUR) gene family in maize: Identification, evolution, and its phylogenetic comparison with Arabidopsis, rice, and sorghum. Journal of integrative plant biology, 2014, 56. Jg., Nr. 2, S. 133-150). Sie können in verschiedene Gruppen aufgeteilt werden und wurden bisher in A. *thaliana*, Reis und Soja in unterschiedlichen Funktionen gefunden. In Mais wurden im B73 Genom 79 putative SAUR Gene identifiziert (Chen et al. Small auxin upregulated RNA (SAUR) gene family in maize: Identification, evolution, and its phylogenetic comparison with Arabidopsis, rice, and sorghum. Journal of integrative plant biology, 2014, 56. Jg., Nr. 2, S. 133-150). Das Kandidatengen ORF-09 wurde als ZmSAUR37 erwähnt, die Bedeutung bei der Vermittlung von Kühletoleranz ist jedoch nicht vorbeschrieben und durchaus überraschend. In den der Erfindung zugrundeliegenden Studien wurde eine unterschiedlich starke Expression von SAUR31 in den sensitiven (SL) im Vergleich mit den toleranten Linien (TH) während Kühlestress festgestellt (Tabelle lb). Die Promotorsequenz zeigte eine hohe Zahl an Polymorphismen zwischen den untersuchten Linien, während die Aminosäuresequenz des kodierten Proteins weitgehend unverändert war.

Weiterhin offenbart die vorliegende Anmeldung das Bereitstellen einer Expressionskassette, die eine Nukleinsäure mit einer Nukleinsäuresequenz umfasst, welche ausgewählt ist aus der Gruppe bestehend aus a) einer Nukleinsäuresequenz gemäß einer der SEQ ID NOs 29, 3, 7, 11, 15, 25 oder 35, oder einem funktionellen Fragment davon, b) einer Nukleinsäuresequenz, die zu einer Sequenz aus a) komplementär ist, c) einer Nukleinsäuresequenz, die zu mindestens 80%, 85%, 90% oder 95% oder bevorzugt zu mindestens 96%, 97%, 98%, oder 99% identisch ist mit einer Sequenz aus a) oder b), d) einer Nukleinsäuresequenz, die sich entsprechend des degenerierten genetischen Codes von einer Nukleinsäuresequenz gemäß a), b) oder c) ableitet, e) einer Nukleinsäuresequenz, die mit einer Nukleinsäuresequenz gemäß a), b) oder c) unter stringenten Bedingungen hybridisiert, f) einer Nukleinsäuresequenz, die für ein Protein gemäß einer der SEQ ID NOs 30, 4, 8, 12, 16 oder 26 oder ein Homolog, Analog oder Ortholog davon kodiert, oder g) einer Nukleinsäuresequenz, die für eine oder mehrere RNAs kodiert, die in der Lage ist/sind zumindest partiell mit sich selbst oder untereinander zu hybridisieren und dadurch einen doppelsträngigen Teil auszubilden, wobei diese Nukleinsäuresequenz über mindestens 19, 20, 21, 22, 23, 24 oder 25, bevorzugt mindestens 30, 32, 34, 36, 38 oder 40, besonders bevorzugt mindestens 50, 60, 70, 80, 90 oder 100, oder ganz besonders bevorzugt mindestens 150, 200, 250, 300, 400, 500, 750 oder 1000 aufeinanderfolgende Nukleotide übereinstimmt mit einer der Nukleinsäuresequenzen ausgewählt aus der Gruppe bestehend aus (i) einer Nukleinsäuresequenz gemäß einer der SEQ ID NOs: 27, 17, 19, 5, 7, 23 oder 25, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, (vi) einer Nukleinsäuresequenz, die für ein Protein gemäß einer der SEQ ID NOs: 28, 18, 20, 6, 8, 24 oder 26, oder ein Homolog, Analog oder Ortholog davon kodiert, oder vii) einer Nukleinsäuresequenz in Antisinnorientierung zu einer Nukleinsäuresequenz gemäß (i) bis (vi).

Eine Nukleinsäure oder Expressionskassette ist vorzugsweise geeignet, nach Transkription oder nach Expression in einer Pflanze, die Eigenschaft einer Kühletoleranz zu vermitteln oder die Kühletoleranz der Pflanze zu steigern.

Dem Prinzip nach sind Expressionskassetten, deren Aufbau und deren Bestandteile dem Fachmann bekannt und in der Literatur beschrieben (Sambrook et al. 2001, Molecular cloning: a laboratory manual (3-volume set) (Vol. 999). Cold Spring Harbor, New York:: Cold spring harbor laboratory press). Solche Kassetten bestehen mindestens aus einem zu exprimierenden Gen und einem operativ damit verknüpften Promotor. Solche Promotoren können in der Lage sein, die transgene Expression bestimmter Gene pflanzenentwicklungs- oder gewebespezifische zu vermitteln, wie z.B. in WO 2003/006660 oder WO 2000/026388. Zum Beispiel beschreibt DE 10 2005 021365 eine blütenspezifische Expressionskassette. Weiter enthalten sein kann mindestens eine in pflanzlichen Zellen oder pflanzlichen Organismen funktionelle Terminatorsequenz (z.B. wie in WO 2003/008596). Weiterhin können ein oder mehrere Resistenzgene in der Expressionskassette enthalten sein, welche eine Selektion erfolgreich transformierter oder transfizierter Zellen erlauben. Dem Fachmann sind auch aus dem Stand der Technik verschiedene zur Selektion geeignete Resistenzgene (Selektionsmarker) bekannt (Miki, B; McHugh, S. Selectable marker genes in transgenic plants: applications, alternatives and biosafety. Journal of Biotechnology, 2004, 107. Jg., Nr. 3, S. 193-232). Diese können beispielsweise eine Resistenz gegen Kanamycin, Streptomycin oder Ampicilin umfassen. Expressionskassetten können als lineare Nukleinsäure oder in einem Vektor oder Plasmid vorliegen.

In einer Ausgestaltung der Expressionskassette der vorliegenden Anmeldung ist die oben beschriebene Nukleinsäure operativ verknüpft mit einem konstitutiven Promotor wie beispielsweise dem 35S-Promotor (Odell, JT; Nagy, F; Chua, NH. Identification of DNA sequences required for activity of the cauliflower mosaic virus 35S Promotor. 1985, US 5,352,605 A), einem Kühle/Kälteinduzierbaren Promotor wie beispielsweise BN115 (US 5,847,102 A) oder einem Promotor wie beispielsweise p63 (EP 2 116 606 B1), der insbesondere in der Frühentwicklung von Pflanzen oder in jungen Geweben von Pflanzen aktiv ist, wobei Frühentwicklung die ersten 12 Wochen nach Keimung, besonders die ersten 8 Wochen nach Keimung und insbesondere die ersten 4 Wochen nach Keimung meinen.

In einer bevorzugten Ausgestaltung der vorliegenden Anmeldung umfasst die Expressionskassette eine Nukleinsäure, die eine Nukleinsäuresequenz aufweist, ausgewählt aus der Gruppe bestehend aus a) einer Nukleinsäuresequenz gemäß der SEQ ID NOs 29, oder einem funktionellen Fragment davon, b) einer Nukleinsäuresequenz, die zu einer Sequenz aus a) komplementär ist, c) einer Nukleinsäuresequenz, die zu mindestens 80%, 85%, 90% oder 95% oder bevorzugt zu mindestens 96%, 97%, 98%, oder 99% identisch ist mit einer Sequenz aus a) oder b), d) einer Nukleinsäuresequenz, die sich entsprechend des degenerierten genetischen Codes von einer Nukleinsäuresequenz gemäß a), b) oder c) ableitet, e) einer Nukleinsäuresequenz, die mit einer Nukleinsäuresequenz gemäß a), b) oder c) unter stringenten Bedingungen hybridisiert, oder f) einer Nukleinsäuresequenz, die für ein Protein gemäß einer der SEQ ID NOs 30 oder ein Homolog, Analog oder Ortholog davon kodiert, vorzugsweise operativ verknüpft mit einem Promotor, der die Nukleotidsequenz gemäß SEQ ID NO: 33 umfasst, oder mit einer allelischen Variante oder einer modifizierte Form eines Promotor, der die Nukleotidsequenz gemäß SEQ ID NO: 34 umfasst, wobei die allelische Variante oder die modifizierte Form eine vergleichbar Expressionsrate oder Expressionshöhe wie der Promotor, der die Nukleotidsequenz gemäß SEQ ID NO: 33 umfasst, erzeugt. Eine allelische Variante oder eine modifizierte Form des Promotors meint einen Promotor, der eine um mehr als 5%, 10%, 15%, 20%, 25%, 30%, 40% oder 50% reduzierte Expressionsrate oder Expressionshöhe im Vergleich mit der Expressionsrate bzw. Expressionshöhe, bewirkt durch den Promotor, der die Nukleotidsequenz gemäß SEQ ID NO: 34 umfasst, verursacht. Eine vergleichbare Expressionsrate oder Expressionshöhe bedeutet, das die allelische Variante oder die modifizierte Form des Promotors, der die Nukleotidsequenz gemäß SEQ ID NO: 34 umfasst, im Wesentlichen eine Expressionsrate oder Expressionshöhe aufweist, welche um nicht mehr als 20%, 18%, 16%, 14% oder 12%, bevorzugt um nicht mehr als 10%, 9%, 8%, 7% oder 6%, oder besonders bevorzugt nicht mehr als 5%, 4%, 3%, 2%, 1%, 0,5% oder 0% von der Expressionsrate oder Expressionshöhe des Promotor, der die Nukleotidsequenz gemäß SEQ ID NO: 33 umfasst, abweicht.

Ferner offenbart die vorliegende Anmeldung auch einen Kühlestress-responsiven Promotor, umfassend eine Nukleotidsequenz aus der SEQ ID NO: 33 oder 34 oder eine zu der Nukleotidsequenz der SEQ ID NO: 33 oder 34 komplementäre Nukleotidsequenz oder eine Nukleotidsequenz, die mit der Nukleotidsequenz der SEQ ID NO: 33 oder 34 oder einer zu der Nukleotidsequenz der SEQ ID NO: 33 oder 34 komplementären Nukleotidsequenz hybridisiert, sowie eine Expressionskassette, umfassend den Kühlestress-responsiven Promotor, einen Vektor, umfassend den Kühlestress-responsiven Promotor oder die Expressionskassette, die den Kühlestress-responsiven Promotor umfasst, eine Wirtszelle oder eine Pflanzen oder Teile davon, umfassend den Kühlestress-responsiven Promotor als Transgen, die Expressionskassette, die den Kühlestress-responsiven Promotor umfasst, oder den Vektor, umfassend den Kühlestress-responsiven Promotor oder die Expressionskassette, die den Kühlestress-responsiven Promotor umfasst.

In einer weiteren bevorzugten Ausgestaltung der vorliegenden Anmeldung umfasst die Expressionskassette eine Nukleinsäure, die Nukleotidsequenz aufweist, die für eine oder mehrere RNAs kodiert, die in der Lage ist/sind zumindest partiell mit sich selbst oder untereinander zu hybridisieren und dadurch einen doppelsträngigen Teil auszubilden, wobei diese Nukleinsäuresequenz über mindestens 19, 20, 21, 22, 23, 24 oder 25, bevorzugt mindestens 30, 32, 34, 36, 38 oder 40, besonders bevorzugt mindestens 50, 60, 70, 80, 90 oder 100, oder ganz besonders bevorzugt mindestens 150, 200, 250, 300, 400, 500, 750 oder 1000 aufeinanderfolgende Nukleotide übereinstimmt mit einer der Nukleinsäuresequenzen ausgewählt aus der Gruppe bestehend aus (i) einer Nukleinsäuresequenz gemäß einer der SEQ ID NOs: 27, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, (vi) einer Nukleinsäuresequenz, die für ein Protein gemäß einer der SEQ ID NOs: 28 oder ein Homolog, Analog oder Ortholog davon kodiert, oder vii) einer Nukleinsäuresequenz in Antisinnorientierung zu einer Nukleinsäuresequenz gemäß (i) bis (vi).

In einem weiteren Aspekt beschreibt die vorliegende Anmeldung einen Vektor, welcher die oben beschriebene Nukleinsäure oder die oben beschriebene Expressionskassette umfasst. Bei dem Vektor kann es sich um ein Plasmid, ein Cosmid, eine Phage oder einen Expressionsvektor, einen Transformationsvektor, Shuttle-Vektor oder Klonierungsvektor handeln, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist die oben beschriebene Nukleinsäure oder Expressionskassette in einem Vektor mit einer oder mehreren regulatorischen Sequenzen, welche die Transkription und optional die Expression in einer prokaryotischen oder eukaryotischen Wirtszelle erlauben, operativ verknüpft. Eine regulatorische Sequenz, vorzugsweise DNA, kann homolog oder heterolog zu der Nukleinsäure sein. Beispielsweise steht die Nukleinsäure unter der Kontrolle eines geeigneten Promotors oder eines Terminators. Geeignete Promotoren können Promotoren sein, welche konstitutiv induziert werden (Bsp.: 35S-Promotor aus dem "Cauliflower mosaic virus" (Odell et al. 1985), besonders geeignet sind solche Promotoren, welche gewebespezifisch, stressspezifisch (z.B. Kühle-repsonsiv, BN115 (US 5,847,102 A)) oder entwicklungsspezifisch sind (Bsp.: Blühspezifische Promotoren z.B. Promotor Region des Gens GTCHS1; Kobayashi, H et al. Flower-specific gene expression directed by the promoter of a chalcone synthase gene from Gentiana triflora in Petunia hybrida. Plant Science, 1998, 131. Jg., Nr. 2, S. 173-180). Geeignete Promotoren können auch synthetische bzw. chimäre Promotoren sein, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren können den gewünschten Spezifitäten nach konzipiert und werden durch unterschiedliche Faktoren induziert oder reprimiert. Beispiele für solche Promotoren finden sich in Gurr & Rushton (Gurr, SJ; Rushton, PJ. Engineering plants with increased disease resistance: what are we going to express?. TRENDS in Biotechnology, 2005, 23. Jg., Nr. 6, S. 275-282) oder Venter (Synthetic promoters: genetic control through cis engineering. Trends in Plant Science, 2007, 12. Jg., Nr. 3, S. 118-124). Ein geeigneter Terminator ist beispielsweise der nos-Terminator (Depicker, A, Stachel, S, Dhaese, P, Zambryski ,P and Goodman, H (1982) J. Mol. Appl. Genet., 1, 561-575).

Zusätzlich zu den oben beschriebenen Vektoren, offenbart die vorliegende Anmeldung auch ein Verfahren bereit, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook et al. 2001, Molecular cloning: a laboratory manual (3-volume set) (Vol. 999). Cold Spring Harbor, New York:: Cold spring harbor laboratory press).

In einem weiteren Aspekt beschreibt die vorliegende Anmeldung eine Wirtszelle, welche die oben beschriebene Nukleinsäure, die Expressionskassette oder den Vektor umfasst. Eine Wirtszelle im Sinne der vorliegenden Anmeldung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche die oben beschriebene Nukleinsäure, die Expressionskassette oder den Vektor umfassen. Dem Fachmann sind sowohl zahlreiche Methoden wie Konjugation oder Elektroporation bekannt, mit denen er die oben beschriebene Nukleinsäure, die Expressionskassette oder den Vektor in ein Agrobakterium einbringen kann, als auch Methoden wie diverse Transformationsverfahren (biolistische Transformation, Agrobakterium-vermittelte Transformation), mit denen er die oben beschriebene Nukleinsäure, die Expressionskassette oder den Vektor in eine Pflanzenzelle einbringen kann (Sambrook et al. 2001).

In einem weiteren Aspekt beschreibt die vorliegende Anmeldung eine transgene Pflanzenzelle, welche die oben beschriebene Nukleinsäure als Transgen oder die Expressionskassette oder den Vektor umfasst, und eine transgene Pflanze oder einen Teil davon, welche die transgene Pflanzenzelle umfassen. Eine solche transgene Pflanzenzelle oder Pflanze ist beispielsweise eine Pflanzenzelle oder Pflanze, welche mit der oben beschriebenen Nukleinsäure, mit der Expressionskassette oder mit dem Vektor, vorzugsweise stabil, transformiert ist. Eine transgene Pflanze oder Zelle weist vorzugsweise eine neu-vermittelte Kühletoleranz oder eine gesteigerte Kühletoleranz im Vergleich zu einer Wildtyp-Pflanze, welche isogen ist, jedoch nicht mit der oben beschriebenen Nukleinsäure, mit der

Expressionskassette oder mit dem Vektor, vorzugsweise stabil, transformiert ist, auf.

In einer bevorzugten Ausgestaltung der transgenen Pflanze ist die Nukleinsäure mit einer oder mehreren regulatorischen Sequenzen, welche die Transkription und optional die Expression in der Pflanzenzelle erlauben, operativ verknüpft. Eine regulatorische Sequenz, vorzugsweise DNA, kann homolog oder heterolog zu der oben beschriebenen Nukleinsäure sein. Das Gesamtkonstrukt aus der Nukleinsäure und der/den regulatorischen Sequenz(en) können dann in Form der Expressionskassette das Transgen darstellen. Ein Teil einer Pflanze kann ein befruchteter oder unbefruchteter Samen, ein Embryo, ein Pollen, ein Gewebe, ein Organ oder eine pflanzliche Zelle sein, wobei der befruchtete oder unbefruchtete Samen, der Embryo oder der Pollen auf der transgenen Pflanze erzeugt werden und in deren Genom die oben beschriebenen Nukleinsäure als Transgen oder die Expressionskassette oder der Vektor integriert ist. Ebenso offenbart die vorliegende Anmeldung auch einen Nachkommen der transgenen Pflanze ein, in dessen Genom die oben beschriebene Nukleinsäure als Transgen, die Expressionskassette oder der Vektor integriert ist und der eine vermittelte Kühletoleranz oder eine gesteigerte Kühletoleranz im Vergleich zu einer Wildtyp-Pflanze, welche isogen ist, jedoch nicht mit der oben beschriebenen Nukleinsäure, mit der Expressionskassette oder mit dem Vektor, vorzugsweise stabil, transformiert ist, aufweist.

Eine neu-vermittelte oder gesteigerte Kühletoleranz kann spezies-spezifisch und experimentell bestimmt werden. Hierbei bietet sich eine Blattbild-Analyse an, welche im Wesentlichen folgende Verfahrensschritte umfasst: a) zwei bis vier wöchige Auszucht der Pflanzen unter Nicht-Stress-Bedingungen mit Bezug auf die Außentemperatur, b) Aussetzen der Pflanzen einem signifikanten Kühlestress über einen Zeitraum von mindestens einer Woche, c) Ausführung einer Regenerationsphase wieder unter Nicht-Stress-Bedingungen über einen Zeitraum von mindestens einer Woche und d) Messung des Blattgrüneverlustes an einem oder mehreren Blättern, deren Wachstum im Zeitraum der Kühlestressapplikation stattfand. Beispielhaft ist dies im Folgenden für Mais (*Zea mays*) beschrieben: Die Pflanzen werden zwei Wochen unter optimalen Bedingungen (ohne Stress) im Gewächshaus bei 25°C (Tagestemperatur) bzw. 22°C (Nachttemperatur) herangezogen. Anschließend erfolgt für eine Woche eine Überführung in eine Klimakammer mit 8°C bzw. 6°C. Es folgt eine einwöchige Regenerationsphase im Gewächshaus bei 25°C bzw. 22°C. Anschließend wurde vorzugsweise das 4. bzw. 5. Blatt hinsichtlich seiner Farbe untersucht. Hierbei steht ein Wert von 100% für vollständige Erhaltung der grünen Blattfarbe und ein Wert von 0% für eine vollständige Gelbfärbung (Chlorose). Im Rahmen der vorliegenden Erfindung konnte somit gezeigt werden, dass die TH-Variante der SL-Variante bei der Erhaltung des Chlorophylls unter Kühlestress überlegen war (s. Tab. 5). Insgesamt wurden Werte von 10% bis 85% Blattgrüne gemessen. Der Blattgrünverlust der TH-Varianten war dabei um 19% bis zu 75% gegenüber den SL-Varianten reduziert, d.h. die Kühletoleranz signifikant gesteigert. Somit meint der Begriff Kühletoleranz - ohne darauf beschränkt zu werden - eine Reduktion des Blattgrünverlustes unter Kühlestress um 5%, 10%, 15%, 20%, bevorzugt 30%, 40% oder 50%, besonders bevorzugt 60%, 70%, 80% oder 90% gemessen mit oben beschriebener Blattbild-Analyse.

Alternativ kann die neu-vermittelte oder gesteigerte Kühletoleranz in einer Pflanze auch durch Messung der Pflanzenhöhe zum Zeitpunkt des Einsetzens des Streckungswachstums im Sprossbereich quantifiziert werden. Hierzu werden Kühletolerante und sensitive Pflanzen unter Kühlestressbedingungen in Vergleichstests angebaut. Im Rahmen der vorliegenden Erfindung konnte so gezeigt werden, dass beispielsweise die Mais-TH-Variante eine um etwa 35% gesteigerte Pflanzenlänge, welche absolut betrachtet etwa zusätzliche 21 cm im Vergleich mit der sensitiven SL-Variante ausmachte. Somit kann der Begriff Kühletoleranz auch meinen, dass eine Pflanze mit neuvermittelter oder gesteigerter Kühletoleranz eine um mindestens 5%, 10%, 15%, 20%, 25%, 30% oder 35% gesteigerte Pflanzenhöhe im Vergleich mit einer Kontrollpflanze zum Zeitpunkt des Einsetzens des Streckungswachstums aufweist.

Ein weiterer Aspekt wird ein Verfahren zur Herstellung einer kühletoleranten Pflanze beschrieben. Ein solches Verfahren umfasst die folgenden Schritte: A) Mutagenisieren von Pflanzenzellen oder von Teilen einer Pflanze und anschließendem Regenerieren von Pflanzen aus den mutagenisierten Pflanzenzellen oder mutagenisierten Teilen oder Mutagenisieren von Pflanzen, und B) Identifizieren einer Pflanze aus A), welche in einer endogenen DNA-Sequenz, welche mit einer Nukleinsäuresequenz, ausgewählt aus der Gruppe bestehend aus (i) einer Nukleinsäuresequenz gemäß einer der SEQ ID NOs: 27, 17, 19, 5, 7, 23 oder 25, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, oder (vi) einer Nukleinsäuresequenz, die für ein Protein gemäß einer der SEQ ID NOs: 28, 18, 20, 6, 8, 24 oder 26, oder ein Homolog, Analog oder Ortholog davon kodiert, identisch ist, oder in einer regulatorischen Sequenz der endogenen DNA-Sequenz mindestens eine Mutation aufweist, die eine Änderung der Transkriptions- oder Expressionsrate oder Transkriptions- oder Expressionshöhe der endogenen DNA-Sequenz in der identifizierten Pflanze im Vergleich mit einer nicht-mutagenisierten Wildtyp-Pflanze oder eine Änderung der Aktivität oder Stabilität eines durch die endogene DNA-Sequenz kodierten Proteins oder Polypeptids in der identifizierten Pflanze im Vergleich mit einer nicht-mutagenisierten Wildtyp-Pflanze bewirkt. Bevorzugt bewirkt die mindestens eine Mutation, dass die identifizierte Pflanze kühletolerant wird oder eine bereits bestehende Kühletoleranz gesteigert wird.

Bevorzugt kodiert die endogene DNA-Sequenz aus Schritt B) für ein Auxin-responsives Protein oder ein SAUR-Protein, besonders bevorzugt für das Protein SAUR31 gemäß einer der SEQ ID NOs: 28 oder 30 oder ein Homolog, Analog oder Ortholog davon. Bevorzugt ist die regulatorische Sequenz der endogenen DNA-Sequenz aus Schritt B) ein Promotor oder ein Teil davon. Besonders bevorzugt ist der Promotor ein Promotor gemäß SEQ ID NO: 34 oder ein Promotor, welcher eine Identität von mindestens 80%, 85% oder 90%, bevorzugt von mindestens 92%, 94%, 96%oder 98% oder besonders bevorzugt von mindestens 98,5%, 99%, 99,5% oder 99,8% mit dem Promotor gemäß SEQ ID NO: 34 aufweist. Ein Beispiel für eine potentielle mutierte Form einer regulatorische Sequenz einer endogenen DNA-Sequenz ist der Promotor gemäß SEQ ID NO: 33.

Eine Mutation meint eine Modifikation auf DNA-Ebene, also eine Veränderung der Genetik und/oder der Epigenetik. Beispielsweise kann eine Veränderung der Genetik der Austausch von mindestens einer Nukleobase in der endogenen DNA-Sequenz oder in einer regulatorischen Sequenz der endogenen DNA-Sequenz sein. Findet ein solcher Nukleobasen-Austausch z.B. in einem Promotor statt, kann dies zu einer veränderten Aktivität des Promotors führen, da beispielsweise cis-regulatorische Elemente derart modifiziert werden, dass die Affinität eines Transkriptionsfaktors zu dem mutierten cis-regulatorische Elemente im Vergleich zum Wildtyp-Promotor verändert ist, sodass die Aktivität des Promotors mit dem mutierten cis-regulatorische Elemente erhöht oder erniedrigt ist, je nachdem ob es sich bei dem Transkriptionsfaktor um einen Repressor oder Induktor handelt oder ob die Affinität des Transkriptionsfaktors zu dem mutierten cis-regulatorische Elemente verstärkt oder abgeschwächt wird. Findet ein solcher Nukleobase-Austausch z.B. in einer kodierenden Region der endogenen DNA-Sequenz statt, kann dies zu einem Aminosäureaustausch im kodierten Protein führen, was eine Veränderung der Aktivität oder Stabilität des Proteins im Vergleich zum Wildtyp-Protein bewirken kann. Ein weiteres Beispiel für eine Veränderung der Genetik ist die Deletion von Nukleotiden in der regulatorische Sequenz und/oder der endogenen DNA-Sequenz sowie die Addition von Nukleotiden in der regulatorischen Sequenz und/oder der endogenen DNA-Sequenz. Ein Beispiel zur Regulation von Genen durch Insertion von Nukleotiden durch Transposonmutagenese in Mais zeigt Das & Martienssen (Das, Lekha, and Robert Martienssen. "Site-selected transposon mutagenesis at the hcf106 locus in maize." The Plant Cell 7.3 (1995): 287-294.). Eine Veränderung der Epigenetik kann beispielsweise durch ein verändertes Methylierungsmuster der DNA erfolgen.

Dem Fachmann ist bekannt, wie eine Mutation im Sinne der Anmeldung durch den Prozess einer Mutagenisierung im Schritt A) des Verfahrens zur Herstellung einer kühletoleranten Pflanze erreicht werden kann. Die Mutagenisierung schließt hierbei sowohl die konventionelle Mutagenese als auch die ortspezifische Mutagenese beziehungsweise das ,Genome Editing' ein. Bei der konventionellen Mutagenese wird die Modifikation auf DNA-Ebene nicht gezielt herbeigeführt. Die Pflanzenzelle oder die Pflanze wird mutagenen Bedingungen wie z.B. TILLING durch UV-Licht-Bestrahlung oder den Einsatz chemischer Stoffe ausgesetzt (Till, Bradley J., et al. "Discovery of induced point mutations in maize genes by TILLING." BMC Plant Biology 4.1 (2004): 12.). Eine weitere Methode der Zufallsmutagenese ist die Mutagenese mit Hilfe eines Transposons. Eine umfangreiche Mutantesammlung steht über das UniformMU-Projekt frei zur Verfügung. Die Sammlung und die Methode sind beschrieben in McCarty et al. (McCarty, Donald R., et al. "Steady-state transposon mutagenesis in inbred maize." The Plant Journal 44.1 (2005): 52-61.). Die ortspezifische Mutagenese ermöglicht die Einbringung von Modifikation auf DNA-Ebene zielgerichtet an vordefinierten Stellen der DNA. Hierfür können beispielsweise, TALENS (WO 2010/079430, WO 2011/072246), Meganukleasen (Silva, George, et al. "Meganucleases and other tools for targeted genome engineering: perspectives and challenges for gene therapy." Current gene therapy 11.1 (2011): 11.), Homing-Endonukleasen (Stoddard, Barry L. "Homing endonucleases: from microbial genetic invaders to reagents for targeted DNA modification." Structure 19.1 (2011): 7-15.), Zinkfmgernukleasen (Lloyd, Alan, et al. "Targeted mutagenesis using zinc-finger nucleases in Arabidopsis." Proceedings of the National Academy of Sciences of the United States of America 102.6 (2005): 2232-2237.) oder ein CRISPR/Cas-System (Gaj, Thomas, Charles A. Gersbach, and Carlos F. Barbas. "ZFN, TALEN, and CRISPR/Cas-based methods for genome engineering." Trends in biotechnology 31.7 (2013): 397-405.) verwendet werden. Vorzugsweise wird die Mutation an allen Kopien bzw. Allelen oder wo sinnvoll an allen Homologen der entsprechenden endogenen DNA-Sequenzen durchgeführt. Dies bedeutet in Bezug auf einen diploiden Organismus wie beispielsweise *Zea mays* typischerweise mindestens zwei Veränderungen.

Das Identifizieren einer Pflanze im Schritt B) kann beispielsweise mit Hilfe von molekularen Markern oder Sonden erfolgen. DNA-Sonden sind beispielsweise Primer oder Primerpaare, welche in einer PCR-Reaktion eingesetzt werden können. Beispielsweise können Tillingmutanten durch Sequenzierung des Zielgens in einer Tilling-Population oder weitere Methoden, die Fehlpaarungen in der DNA nachweisen, wie z.B. Schmelzpunktanalysen oder Einsatz fehlpaarungsspezifischer Nukleasen nachgewiesen beziehungsweise identifiziert werden. Vorliegende Anmeldung schließt hierfür einsetzbare Primer/Primerpaare ebenfalls mit ein, wie z.B. Primer zum Nachweis von SAUR31 oder einer mutierten Form des Promotors von SAUR31. Ferner können Mutanten, erzeugt mittels Transposons, durch Einsatz von Transposon-spezfischen Primern und Zielgen-spezifischen Primern in der PCR über die gesamte Population und anschließende Sequenzierung von PCR-Produkten nachgewiesen werden. Auch solche Primer sind in der vorliegenden Anmeldung offenbart. Änderung der Expressionsrate oder Expressionshöhe können beispielsweise mit RT-PCR in pflanzlichen Geweben bestimmt werden, die Änderung der Stabilität beispielsweise durch Untersuchung von Ubiquitin-Bindestellen und Vorhersagen über Änderungen der Teritärstruktur. Weiterhin sind auch rekombinante Expression der Wildtyp-Proteine und der enstprechenden mutierten Proteine und nachfolgende biochemische Aktivitätstests geeignet. Dem Fachmann sind aus dem Stand der Technik weitere Mittel und Verfahren bekannt, welche er zum Identifizieren einer Pflanze oder Pflanzenzelle im Schritt B) verwenden kann.

Die vorliegende Anmeldung beschreibt auch molekulare Marker, welche die Anwesenheit oder Abwesenheit einer Mutation in der endogenen DNA-Sequenz oder in einer regulatorischen Sequenz der endogenen DNA-Sequenz nachweisen. Solche Marker basieren beispielsweise auf einem SNP und sind spezifisch für die Mutation (Beispiele: KASPar oder TaqMan Marker).

Die vorliegende Anmeldung offenbart weiterhin auch eine Pflanze, welche mit dem vorstehenden Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die mindestens eine Mutation vorhanden ist. Ebenso beschreibt die vorliegende Anmeldung auch ein Nachkomme der Pflanze, welcher die mindestens eine Mutation aufweist und kühletolerant ist.

Ferner betrifft die vorliegende Anmeldung auch ein Verfahren zur Isolierung einer Nukleinsäure, die in einer Pflanze oder Pflanzenzelle die Kühletoleranz vermitteln oder steigern, umfassend die folgenden Schritte:
A) Herstellung einer Pflanze nach vorstehend beschriebenen Verfahren oder Bereitstellung einer Pflanze oder einer Zelle einer Pflanze, welche mit vorstehend beschriebenen Verfahren herstellbar ist oder hergestellt ist, und B) Isolieren einer Nukleinsäure, welche die endogene DNA-Sequenz mit der mindestens einen Mutation umfasst, aus dem Genom der Pflanze oder Pflanzenzelle aus A). Das Isolieren der Nukleinsäure in Schritt B) kann durch CTAB-Extraktion oder über DNA-bindende Säulen erfolgen, der Nachweis der Mutation über Sequenzierung oder molekulare Marker, wie SNP basierte KASPar oder TaqMan Marker, oder bei Insertions- oder Deletionsmutanten über Marker auf der Basis von Längenpolymorphismen.

Die vorliegende Anmeldung beschreibt auch eine Nukleinsäure, welche durch das vorstehend beschriebenen Verfahren zur Isolierung erhalten wurde oder durch das vorstehend beschriebenen Verfahren zur Isolierung erhältlich ist, sowie eine Expressionkassette oder einen Vektor, welche/welcher die isolierte Nukleinsäure umfasst.

Ein weiterer Aspekt der vorliegenden Anmeldung ist ein Verfahren zur Herstellung einer transgenen, kühletoleranten Pflanze. Das Verfahren kann die folgenden Schritte umfassen: A) Bereitstellen der oben beschriebenen Nukleinsäure oder Expressionskassette, oder Bereitstellen des oben beschriebenen Vektors, B) Transformieren, vorzugsweise stabil Transformieren, von mindestens einer Pflanzenzellen durch Einbringen der Nukleinsäure, der Expressionskassette oder des Vektors aus A), C) Regenerieren transgener Pflanzen aus der mindestens einen transformierten Pflanzenzellen aus B), und D) Identifizieren einer transgenen, kühletoleranten Pflanze aus C). Das Verfahren zur Herstellung der transgenen, kühletoleranten Pflanze schließt auch die Bereitstellung von zwei oder mehr der oben beschriebenen Nukleinsäure, wahlweise auch unterschiedliche Ausgestaltungen der oben beschriebenen Nukleinsäure und optional in einem oder mehreren Expressionskassetten oder Vektoren, und das Transformieren von Pflanzenzellen durch Einbringen der zwei oder mehr Nukleinsäuren ein. Ergänzend können neben der oben beschriebenen Nukleinsäure auch eine oder mehrere weitere Nukleinsäuren, welche bekanntermaßen einsetzbar sind zur Vermittlung oder

Steigerung von Kühletoleranz (z.B. WO 2002/048378 A2, WO 2008/148298 A1) als Transgen eingesetzt werden.

In einer bevorzugten Ausführungsform des Herstellungsverfahrens weist eine in D) identifizierte Pflanze im Vergleich zu einer Wildtyp-Pflanze, welche beispielsweise aus einer isogenen, nicht-transformierten Pflanzenzelle regeneriert wurde, vorzugsweise ein geändertes Expressionsmuster auf, gekennzeichnet dadurch, dass aufgrund von post-transkriptionellem Gene Silencing die Expressionsrate oder Expressionshöhe einer endogenen DNA-Sequenz mit einer Nukleinsäuresequenz, ausgewählt aus der Gruppe bestehend aus (i) einer Nukleinsäuresequenz gemäß einer der SEQ ID NOs: 27, 17, 19, 5, 7, 23 oder 25, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, oder (vi) einer Nukleinsäuresequenz, die für ein Protein gemäß einer der SEQ ID NOs: 28, 18, 20, 6, 8, 24 oder 26, oder ein Homolog, Analog oder Ortholog davon kodiert, reduziert ist.

Die vorliegende Anmeldung betrifft weiterhin auch eine transgene, kühletolerante Pflanze, welche mit genanntem Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die erfindungsgemäße Nukleinsäure als Transgen, die Expressionskassette oder der Vektor integriert ist. Ebenso schließt die vorliegende Erfindung auch ein Nachkomme der transgenen Pflanze ein, welcher kühletolerant ist, ein.

In einem weiteren Aspekt betrifft die vorliegende Anmeldung ein Verfahren zur Vermittlung oder Steigerung der Kühletoleranz in einer Pflanzenzelle oder einer Pflanze. Ein solches Verfahren kann die folgenden Schritte umfassen: A) Transformieren, vorzugsweise stabil Transformieren, vorzugsweise von mindestens einer Pflanzenzellen durch Einbringen der oben beschriebenen Nukleinsäure oder Expressionskassette, oder des oben beschriebenen Vektors, optional B) Regenerieren transgener Pflanzen aus der mindestens einen transformierten Pflanzenzellen aus A). Das Verfahren zur Herstellung der transgenen, kühletoleranten Pflanze schließt auch die Transformieren von zwei oder mehr der oben beschriebenen Nukleinsäuren, wahlweise auch unterschiedliche Ausgestaltungen der Nukleinsäuren und optional von einem oder mehreren oben beschriebenen Expressionskassetten oder Vektoren ein. In einer bevorzugten Ausführungsform des Verfahren führt das Transformieren in Schritt A) zu einer Pflanzenzelle oder Pflanze, die im Vergleich mit einer Wildtyp-Pflanzenzelle, welche beispielsweise eine isogenen, nicht-transformierten Pflanzenzelle ist, oder mit einer Pflanze, welche beispielsweise aus einer isogenen, nicht-transformierten Pflanzenzelle regeneriert wurde, vorzugsweise ein geändertes Expressionsmuster aufweist, gekennzeichnet dadurch, dass aufgrund von post-transkriptionellem Gene Silencing die Expressionsrate oder Expressionshöhe einer endogenen DNA-Sequenz mit einer Nukleinsäuresequenz, ausgewählt aus der Gruppe bestehend aus (i) einer Nukleinsäuresequenz gemäß einer der SEQ ID NOs: 27, 17, 19, 5, 7, 23 oder 25, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, oder (vi) einer Nukleinsäuresequenz, die für ein Protein gemäß einer der SEQ ID NOs: 28, 18, 20, 6, 8, 24 oder 26, oder ein Homolog, Analog oder Ortholog davon kodiert, reduziert ist.

Ferner offenbart in einem alternativen Aspekt die Anmeldung auch die Verwendung der oben beschriebenen Nukleinsäure, der Expressionskassette oder des Vektors in einem Verfahren zur Herstellung einer transgenen, kühletoleranten Pflanzezelle oder Pflanze oder in einem Verfahren zur Vermittlung oder Steigerung der Kühletoleranz in einer Pflanzenzelle oder Pflanzen.

In einem weiteren Aspekt beschreibt die vorliegende Anmeldung ein Mittel zur äußeren Applikation bei Pflanzen. Dieses Mittel wird zur äußeren Applikation bei Pflanzen bereitgestellt. Es enthält doppelsträngige RNA, wobei ein Strang dieser RNA eine Nukleinsäuresequenz aufweist, die über mindestens 19, 20, 21, 22, 23, 24 oder 25, bevorzugt mindestens 30, 32, 34, 36, 38 oder 40, besonders bevorzugt mindestens 50, 60, 70, 80, 90 oder 100, oder ganz besonders bevorzugt mindestens 150, 200, 250, 300, 400, 500, 750 oder 1000 aufeinanderfolgende Nukleotide übereinstimmt mit einer der Nukleinsäuresequenzen ausgewählt aus der Gruppe bestehend aus (i) einer Nukleinsäuresequenz gemäß einer der SEQ ID NOs: 27, 17, 19, 5, 7, 23 oder 25, (ii) einer Nukleinsäuresequenz, die zu einer Sequenz aus i) komplementär ist, (iii) einer Nukleinsäuresequenz, die zu mindestens 80%, 85%, 90% oder 95% oder bevorzugt zu mindestens 96%, 97%, 98%, oder 99% identisch ist mit einer Sequenz aus (i) oder (ii), (iv) einer Nukleinsäuresequenz, die sich entsprechend des degenerierten genetischen Codes von einer Nukleinsäuresequenz gemäß (i), (ii) oder (iii) ableitet, (v) einer Nukleinsäuresequenz, die mit einer Nukleinsäuresequenz gemäß (i), (ii) oder (iii) unter stringenten Bedingungen hybridisiert, (vi) einer Nukleinsäuresequenz, die für ein Protein gemäß einer der SEQ ID NOs: 28, 18, 20, 6, 8, 24 oder 26, oder ein Homolog, Analog oder Ortholog davon kodiert, oder vii) einer Nukleinsäuresequenz in Antisinnorientierung zu einer Nukleinsäuresequenz gemäß (i) bis (vi). Doppelsträngige RNA zur Herstellung des Mittels kann *in vitro* über dem Fachmann bekannte Methoden produziert werden. Beispielsweise kann die Synthese der doppelsträngigen RNA synthetisch erfolgen, wobei die RNA *in vitro* direkt gebildet wird. Die doppelsträngige RNA kann auch ausgehend von einer doppelsträngigen DNA über die Bildung eines mRNA-Transkriptes, welches dann z. B. eine hairpin-Struktur ausbildet, synthetisiert wird.

Das oben beschriebene Mittel kann als Beimischung in einer Saatguthülle oder in der Frühentwicklung durch Aufsprühen in Form eines Sprays zum Einsatz kommen. Weiterhin kann das Mittel auch durch Mischen mit dem Aufzuchtsubstrat vor oder nach Aufgang der Pflanzen eingesetzt werden. In jedem Fall ist das Mittel geeignet, einer Zelle des Samens oder Pflanze, oder dem Samen oder der Pflanze an sich Kühletoleranz zu vermitteln oder die Kühletoleranz zu steigern. In der Anwendung zur Vorbehandlung des Saatguts kann das Mittel zunächst mit einer Trägersubstanz verbunden und in einer Kombination, welche die doppelsträngige RNA und die Trägersubstanz umfasst, auf das Saatgut aufgebracht werden, wobei die Trägersubstanz beispielsweise RNAstabilisierende Wirkung hat. Als RNA-Stabilisatoren können beispielsweise Liposomen, welche die RNA-Moleküle verkapseln, eingesetzt werden.

Des Weiteren offenbart die vorliegende Anmeldung auch ein Verfahren zur Vermittlung oder Steigerung der Kühletoleranz in einer Pflanzenzelle oder einer Pflanze ein, welches den Schritt der äußeren Applikation des oben beschriebenen Mittels umfasst. Vorzugsweise wird das Mittel der Saatguthülle oder dem Saatgutfilm beigemengt oder direkt auf das Saatgut oder die Pflanze aufgesprüht. Auch beschreibt die vorliegende Anmeldung die Verwendung des oben beschriebenen Mittels zur Vermittlung oder Steigerung der Kühletoleranz in einer Pflanzenzelle oder einer Pflanze.

In einem Aspekt wird die der Erfindung zugrunde liegende Aufgabe gelöst durch das Bereitstellen einer kühletoleranten Maispflanze oder einem Teil davon umfassend ein erstes chromosomales Intervall eines Donors auf Chromosom 4 zwischen den Markerpositionen ma59778s31 und ma59778119, welches eine endogene Kühletoleranz-vermittelnde Nukleinsäure aufweist, wobei die Kühletoleranz-vermittelnde Nukleinsäure eine Nukleinsäuresequenz umfasst, ausgewählt aus der Gruppe bestehend aus a) einer Nukleinsäuresequenz gemäß einer der SEQ ID NO 29, b) einer Nukleinsäuresequenz, die zu mindestens 98%, 99% oder 99,5% identisch ist mit einer Sequenz aus a), c) einer Nukleinsäuresequenz, die sich entsprechend des degenerierten genetischen Codes von einer Nukleinsäuresequenz gemäß a) ableitet, d) einer Nukleinsäuresequenz, die für ein Protein gemäß einer der SEQ ID NO 30 kodiert, wobei an der Markerposition ma59778s31 ein T, an einer Markerposition ma59778s32 ein T, an der Markerposition ma59778119 ein C, an einer Markerposition ma52594s01 ein A und einer Markerposition ma20205s01 ein G ist, oder wobei an der Markerposition ma59778s31 ein C, an einer Markerposition ma59778s32 ein T, an der Markerposition ma59778119 ein C und an einer Markerposition ma52594s01 ein A ist; und wobei die Markerpositionen mit Referenz zur Maislinie B73 AGPv2 die folgenden sind: ma59778s31 = 37263172 bp, ma59778s32 = 37296672 bp, ma59778119 = 37297901 bp, ma52594s01 = 58033711 bp und ma20205s01 = 156.998.152 bp. In einem bevorzugten Ausführungsbeispiel ist das chromosomale Intervall auf Chromosom 4, welches eine Kühletoleranz-vermittelnde Nukleinsäure aufweist, ein Intervall zwischen den Markerpositionen ma59778s32 und ma59778119 und/oder umfasst die Kühletoleranz-vermittelnde Nukleinsäure eine Nukleinsäuresequenz, ausgewählt aus der Gruppe bestehend aus a) einer Nukleinsäuresequenz gemäß der SEQ ID NOs 29, b) einer Nukleinsäuresequenz, die zu mindestens 98%, 99% oder 99,5% identisch ist mit einer Sequenz aus a), c) einer Nukleinsäuresequenz, die sich entsprechend des degenerierten genetischen Codes von einer Nukleinsäuresequenz gemäß a) ableitet, oder d) einer Nukleinsäuresequenz, die für ein Protein gemäß einer der SEQ ID NO 30 kodiert, vorzugsweise ist die Nukleinsäuresequenz operativ verknüpft mit einem Promotor, der die Nukleotidsequenz gemäß SEQ ID NO: 33 umfasst, oder mit einer allelischen Variante oder einer modifizierte Form eines Promotor, der die Nukleotidsequenz gemäß SEQ ID NO: 34 umfasst, wobei die allelische Variante oder die modifizierte Form eine vergleichbar Expressionsrate oder Expressionshöhe wie der Promotor, der die Nukleotidsequenz gemäß SEQ ID NO: 33 umfasst, erzeugt. Eine allelischen Variante oder einer modifizierte Form des Promotor meint einen Promotor, der eine um mehr als 5%, 10%, 15%, 20%, 25%, 30%, 40% oder 50% reduzierte Expressionsrate oder Expressionshöhe im Vergleich mit der Expressionsrate bzw. Expressionshöhe, erzeugt durch den Promotor, der die Nukleotidsequenz gemäß SEQ ID NO: 34 umfasst, verursacht. Eine vergleichbare Expressionsrate oder Expressionshöhe bedeutet, das die allelische Variante oder die modifizierte Form des Promotors, der die Nukleotidsequenz gemäß SEQ ID NO: 34 umfasst, im Wesentlichen eine Expressionsrate oder Expressionshöhe aufweist, welche um nicht mehr als 20%, 18%, 16%, 14% oder 12%, bevorzugt um nicht mehr als 10%, 9%, 8%, 7% oder 6%, oder besonders bevorzugt nicht mehr als 5%, 4%, 3%, 2%, 1%, 0,5% oder 0% von der Expressionsrate oder Expressionshöhe des Promotor, der die Nukleotidsequenz gemäß SEQ ID NO: 33 umfasst, abweicht.

Ferner bedeutet die Anwesenheit von dem mindestens einen chromosomalen Intervall, das nicht vom Donor stammt sind, in dem Bereich auf Chromosom 4 flankiert durch die Markerpositionen ma59778119 und ma20205s01, dass beispielsweise aufgrund eines oder mehrerer Rekombinationsereignisse im Kreuzungsprozess mit einer Maispflanze, die nicht Träger der Donorintervalle ist, ein entsprechendes Donor-Intervall bzw. entsprechende Donorintervalle durch das mindestens einen chromosomalen Intervall, das nicht vom Donor stammt sind, ersetzt wurde. Anders ausgedrückt, das ursprünglich vom Donor stammende chromosomale Intervall flankiert durch die Markerpositionen ma59778119 und ma20205s01 ist um Donorallele verkürzt bzw. liegt in trunkierter Form vor.

Alternativ stellt die moderne Biotechnologie dem Fachmann diverse weitere Werkzeuge zur Verfügung, welche ein präzises Genom-Engineering ermöglichen: Gentechnische Ansätze, mit deren Hilfe die gezielt Donorabschnitte durch Nicht-Donorabschnitte ersetzt werden können und so ein ,selective sweep' in einem pflanzlichen Genom reduziert oder eliminiert werden kann, umfassen die Verwendung von TALE-Nukleasen (TALENs) oder Zinkfinger-Nukleasen (ZFNs) sowie CRISPR/Cas Systeme, die u.a. beispielhaft in der deutschen Patentanmeldung DE 10 2013 014 637 zur Eliminierung von Linkage Drag-tragenden Nukleotidsequenzen aus dem Genom von *Helminthosporium turcicum* resistenten (Hybrid)Mais beschrieben sind; siehe die DE 10 2013 014 637 auf Seite 13 und 14 in Paragraphen [0038] bis [0042] und die dort zitierten Referenzen. Diese Techniken, die auch in der internationalen Patentanmeldung WO 2014/104878 beschrieben sind, können equivalent in der Herstellung der vorliegenden erfindungsgemäßen Pflanzen verwendet werden.

Die vorliegende Erfindung schließt weiterhin auch eine Kombination von der konventionellen Züchtungstechnik und der modernen Biotechnologie ein. So können beispielsweise mit Hilfe dieser neuartigen Genom-Editing Ansätze Rekombinations-"Hot spots" in einer Pflanze erzeugt werden, die an geeigneten Stellen stattfinden, um den Austausch von Donor-Abschnitten in dem Bereich auf Chromosom 4 flankiert durch die Markerpositionen ma59778119 und ma20205s01 durch Nicht-Donor-Abschnitte unmittelbar zu begünstigen. Die vorliegende Erfindung stellt dem Fachmann hierfür die notwendigen Informationen über Lokalisation des ,selective sweeps', sowie der Position der Kühletoleranz-vermittelnden Nukleinsäure(n) zu Verfügung.

In einem bevorzugten Ausführungsbeispiel weist die Pflanze in dem Bereich auf Chromosom 4 flankiert durch die Markerpositionen ma59778119 und ma20205s01 mindestens ein weiteres chromosomales Intervall desselben Donors und mindestens ein chromosomales Intervall, das nicht vom Donor stammt, auf, wobei das mindestens eine weitere chromosomale Intervall desselben Donors weniger als 90%, weniger als 80%, weniger als 70%, bevorzugt weniger als 60%, weniger als 50%, weniger als 40%, oder besonders bevorzugt weniger als 30%, weniger als 20%, weniger als 10%, weniger als 5%, weniger als 2% oder weniger als 1% des Bereichs auf Chromosom 4 flankiert durch die Markerpositionen ma59778119 und ma20205s01 darstellt, oder wobei das mindestens eine chromosomale Intervall, das nicht vom Donor stammt, mehr als 5%, mehr als 10%, mehr als 20%, mehr als 30%, bevorzugt mehr als 40%, mehr als 50%, mehr als 60%, oder besonders bevorzugt mehr als 70%, mehr als 80%, mehr als 90%, mehr als 95%, mehr als 98% oder mehr als 99% des Bereichs auf Chromosom 4 flankiert durch die Markerpositionen ma59778119 und ma20205s01 darstellt.

In einem anderen bevorzugten Ausführungsbeispiel weist die Pflanze in dem Bereich auf Chromosom 4 flankiert durch die Markerpositionen ma59778119 und ma20205s01 mindestens ein weiteres chromosomales Intervall desselben Donors und mindestens ein chromosomales Intervall, das nicht vom Donor stammt, auf, wobei das mindestens eine weitere chromosomale Intervall desselben Donors weniger als 100 Mb, weniger als 90Mb, weniger als 80Mb, bevorzugt weniger als 70 Mb, weniger als 60 Mb, weniger als 50 Mb, oder besonders bevorzugt weniger als 40 Mb, weniger als 30 Mb, weniger als 20 Mb, weniger als 15 Mb, weniger als 10 Mb oder weniger als 5 Mb des Bereichs auf Chromosom 4 flankiert durch die Markerpositionen ma59778119 und ma20205s01 darstellt, oder wobei das mindestens eine chromosomale Intervall, das nicht vom Donor stammt, mehr als 5 Mb, mehr als 10 Mb, mehr als 15 Mb, mehr als 20 Mb, bevorzugt mehr als 30 Mb, mehr als 40 Mb, mehr als 50 Mb, oder besonders bevorzugt mehr als 60 Mb, mehr als 70 Mb, mehr als 80 Mb, mehr als 90 Mb oder mehr als 100 Mb des Bereichs auf Chromosom 4 flankiert durch die Markerpositionen ma59778119 und ma20205s01 darstellt.

In einem besonders bevorzugten Ausführungsform weist die Pflanze in dem Bereich auf Chromosom 4 flankiert durch die Markerpositionen ma59778119 und ma20205s01 ein chromosomales Intervall desselben Donors von Markerposition ma59778119 bis ma52594s01 und ein chromosomales Intervall, das nicht vom Donor stammt, von ma52594s01 bis ma20205s01 auf.

In einer weiteren Ausführungsform ist der Bereich auf Chromosom 4 flankiert durch die Markerpositionen ma59778119 und ma20205s01 dadurch charakterisiert, dass er zumindest in Teilen eine erhöhte Allelfrequenz aufweist.

Vorzugsweise ist das chromosomale Intervall zwischen den Markerpositionen ma59778s31 und ma59778119 und das chromosomale Intervall zwischen den Markerpositionen ma59778119 und ma20205s01 auf Chromosom 4 im Maisgenom lokalisiert. Das chromosomale Intervall zwischen den Markerpositionen ma59778119 und ma20205s01 kann das Centromer des Chromsom 4 enthalten.

In einem bevorzugten Beispiel ist das chromosomale Intervall, welches eine Kühletoleranz-vermittelnde Nukleinsäure aufweist, ein chromosomales Intervall zwischen den Markerpositionen ma59778s32 und ma59778119. In einem weiteren bevorzugten Ausführungsbeispiel umfasst das chromosomale Intervall zwischen den Markerpositionen ma59778s31 und ma59778119 ein chromosomales Intervall flankiert durch Markerpositionen ma59778s32 und ma59778119 Vorzugsweise stammt das chromosomale Intervall zwischen den Markerpositionen ma59778s31 und ma59778119 wie auch die darin enthaltende Kühletoleranz-vermittelnde Nukleinsäure aus einer Maislinie des Dent-Pools bzw. ist charakteristisch für den Dent-Pool, d.h. der Fachmann ist in der Lage den chromosomalen Abschnitt eindeutig als aus dem Dent-Pool stammend beispielsweise mit Hilfe von molekularen Markern zu identifizieren.

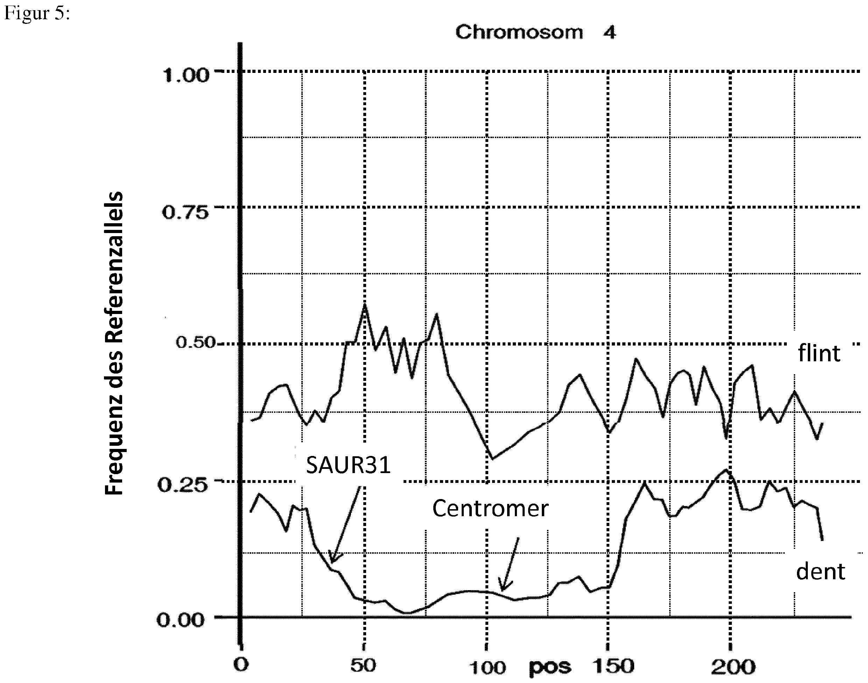

Eine Selektion auf Gene mit starken Effekten oder chromosomale Intervalle enthaltend ein Gen mit starkem Effekt, wie beispielsweise im oben beschriebenen Genomintervall bzw. kühletoleranz vermittelnden QTL (insbesondere das identifizierte Gen SAUR31) führt zu einer Veränderung der Allelfrequenzen. Abhängig vom Grad der Rekombination und der Selektionsintensität ist von dieser Allelfrequenzveränderung nicht nur der an das Gen bzw. das Intervall angrenzende Bereich betroffen, sondern auch benachbarte Chromosomenregionen. Dies kann zu einer eingeschränkten genetischen Diversität führen, welche als "Selective Sweep" bezeichnet wird. Für einen Fachmann aus dem Bereich der Pflanzenzüchtung ist dieser "Selective Sweep" außerordentlich nachteilig, da das erzeugte Pflanzenmaterial bei der weiteren züchterischen Bearbeitung nicht mehr sein ursprünglich vorhandenes Potential entfalten kann. Die genetische Verarmung lässt den klassischen Ansatz der Züchtung aus Neurekombination und Selektion ins Leere laufen. Dies wird durch Fig. 5 verdeutlicht. Die Abbildung zeigt eine deutlich reduzierte genetische Diversität im Dent-Genpool im Vergleich zum Flint-Genpool im Bereich des Kühletoleranz vermittelnden QTL, umfassend beispielsweise das Gen SAUR31. Sämtliche hierin befindlichen ORFs des QTLs und die entsprechenden Gene stammen aus dem Dent-Genpool. Unkontrollierte Einkreuzung des Kühletoleranz vermittelnden QTL in einen anderen genetischen Hintergrund (beispielsweise den Flint-Pool) würde auch in diesem Pool zu einem drastischen Rückgang an Allelfrequenzen führen. Im Rahmen der Erfindung wurde offensichtlich, dass das Einkreuzen des chromosomalen Intervalls zwischen den Markerpositionen ma59778s31 und ma59778119, welches eine Kühletoleranz-vermittelnde Nukleinsäure aufweist, ohne adäquate Gegenmaßnahmen in jedem Fall zu einer deutlichen Herabsetzung der Allel-Frequenz auf Chromosom 4 führen würde. Die Übertragung des identifizierten QTLs bzw. der identifizierten Kühletoleranz-vermittelnden Nukleinsäure ohne eine Reduzierung der Diversität des Zuchtmaterials stellte eine enorme Herausforderung dar und konnte letztendlich durch eine sehr aufwendige markergestützte Feinkartierung des Bereichs angegangen werden. In diesem Zusammenhang wurde auch die Identifizierung der Single-Nucleotide-Polymorphismen (SNPs; Abfolge der SNPs = Haplotyp) im chromosomalen Intervall und angrenzenden Bereichen erforderlich. Durch die Identifikation des mit gesteigerter Kühletoleranz ausgestatten TH-Haplotypen und die Entwicklung neuer Marker war es erfindungsgemäß jedoch überraschenderweise möglich, durch Anwendung neu entwickelter molekularer Marker für eine markergestützte Selektion das entsprechende oben beschriebene chromosomale Intervall mit der Kühletoleranz-vermittelnde Nukleinsäure innerhalb eines stark eingegrenzten Intervalls einzukreuzen. Die auf diese Weise erzeugten Pflanzen zeigten eine gesteigerte Kühletoleranz bei gleichzeitig weitestgehend erhaltener genetischer Diversität. Aus bekannten Züchtungslinien, wie zum Beispiel B73, ließ sich eine Lösung für das Problem des selective sweep aufgrund der unterschiedlichen Struktur der Region nicht ableiten.

"Eine zumindest in Teilen" erhöhte Allelfrequenz bedeutet beispielsweise, dass der Bereich auf Chromosom 4 flankiert durch die Markerpositionen ma59778119 und ma20205s01in einen Bereich von mindestens 5 Megabasen (Mb), mindestens 10 Mb, mindestens 15 Mb, mindestens 20 Mb oder mindestens 25 Mb, bevorzugt mindestens 30 Mb, mindestens 40 Mb, mindestens 50 Mb oder mindestens 60 Mb, oder besonders bevorzugt mindestens 70 Mb, mindestens 80 Mb, mindestens 90 Mb oder mindestens 100 Mb eine erhöhte Allelfrequenz aufweist. Ferner kann "eine zumindest in Teilen" erhöhte Allelfrequenz bedeuten, dass der Bereich auf Chromosom 4 flankiert durch die Markerpositionen ma59778119 und ma20205s01 in einen Bereich von mindestens 1 Mb, mindestens 2 Mb, mindestens 3 Mb, mindestens 4 Mb oder mindestens 5 Mb, bevorzugt mindestens 10 Mb, mindestens 15 Mb, mindestens 20 Mb oder mindestens 25 Mb, oder besonders bevorzugt mindestens 30 Mb, mindestens 35 Mb, mindestens 40 Mb oder mindestens 50 Mb zu beiden Seiten des Centromers vozugsweise des Chromsoms 4 eine erhöhte Allelfrequenz aufweist.

Eine "erhöhte Allelfrequenz" bedeutet beispielsweise eine Abweichung von der Allelfrequenz von 0,5 um nicht mehr als 0,4, 0,375 oder 0,35, bevorzugt nicht mehr als 0,325, 0,3oder 0,275, oder besonderes bevorzugt nicht mehr als 0,25. Ferner kann eine "erhöhte Allelfrequenz" auch bedeuten, dass die Allelfrequenz nicht kleiner als 0,1, 0,125 oder 0,15, bevorzugt nicht kleiner als 0,175, 0,2 oder 0,225, oder besonders bevorzugt nicht kleiner als 0,25 ist. Weiterhin kann eine "erhöhte Allelfrequenz" bedeuten, dass mindestens 10%, 15% oder 20%, bevorzugt 25%, 30% oder 40% oder besonders bevorzugt 45% oder 50% eines chromosomalen Abschnitts aus dem Flint-Pool stammen. Umgekehrt kann eine "niedrige Allelfrequenz" bedeutet eine Abweichung von der Allelfrequenz von 0,5 um mehr als 0,4 oder 0,425. Ferner kann eine "niedrige Allelfrequenz" auch bedeuten, dass die Allelfrequenz kleiner als 0,1 oder 0,075 ist. Weiterhin kann eine "niedrige Allelfrequenz" auch bedeuten, dass weniger als 5%, 6%, 7%, 8%, 9% oder 10% eines chromosomalen Abschnitts aus dem Flint-Pool stammen. Im Zusammenhang mit der vorliegenden Erfindung kann eine "erhöhte Allelfrequenz" auch bedeuten, dass das chromosomale Intervall, welches die erhöhte Allelfrequenz aufweisen soll, vorzugsweise proximal oder distal zu der Kühletoleranz-vermittelnde Nukleinsäure, trunkiert bzw. verkürzt ist, beispielsweise um mindestens 5 Megabasen (Mb), mindestens 10 Mb, mindestens 15 Mb, mindestens 20 Mb oder mindestens 25 Mb, bevorzugt mindestens 30 Mb, mindestens 40 Mb, mindestens 50 Mb oder mindestens 60 Mb, oder besonders bevorzugt mindestens 70 Mb, mindestens 80 Mb, mindestens 90 Mb oder mindestens 100 Mb.

In einem weiteren Aspekt offenbart die vorliegende Anmeldung molekulare Marker, welche geeignet sind, in einem chromosomalen Intervall flankiert durch die Markerpositionen ma59778s31 und ma20205s01 oder durch die Markerpositionen ma59778s31 und ma52594s01 oder durch die Markerpositionen ma59778s31 und ma59778119 zwischen einem kühle-toleranten und einem kühle-sensitiven Haplotyp zu unterscheiden. Bevorzugt entspricht der Kühle-tolerante Haplotyp der TH-Linie mit einem Haplotyp gemäß der Tabelle 2, und/oder der kühle-sensitive Haplotyp der SL-Linie mit einem Haplotyp gemäß der Tabelle 2. Bei einem molekularen Marker der vorliegenden Erfindung kann sich beispielsweise um einen molekularen Marker handeln, welcher geeignet ist, an einer der Markerpositionen ma59778s31, ma59778s32, ma59778119, ma52594s01 und ma20205s01 zwischen einem kühle-toleranten und einem kühle-sensitiven Haplotyp zu unterscheiden. Ein molekularer Marker kann ein Oligonukleotid, insbesondere ein Primer-Oligonukleotid sein, oder kann in isolierter Form vorliegen. In einer besonders bevorzugten Ausführungsform ist der molekulare Marker der vorliegenden Erfindung allein oder in Kombination mit anderen molekularen Markern geeignet, die die Kühletoleranz-vermittelnde Nukleinsäure nachzuweisen. Ferner beschreibt die vorliegende Anmeldung die Verwendung von mindestens einem der molekularen Marker der vorliegenden Erfindung zur Identifizierung oder Selektion einer erfindungsgemäßen kühletoleranten Maispflanze oder eines Teils davon.

In einem weiteren Aspekt betrifft die vorliegende Erfindung ein Verfahren zur Identifikation einer oben beschriebenen erfindungsgemäßen kühletoleranten Maispflanze oder Teile davon, umfassend die Schritte A) Isolierung von DNA aus dem Genom einer Maispflanze, und B) Nachweis der Allele an Marker-Positionen ma59778s31, ma59778s32 und ma59778119, welches diagnostisch für die Kühletoleranz-vermittelnde Nukleinsäure ist, in einem chromosomalen Intervall flankiert durch die Markerpositionen ma59778s31 und ma59778119, wobei an Markerposition ma59778s31 ein T, an Markerposition ma59778s32 ein T und an Markerposition ma59778119 ein C ist, oder wobei an Markerposition ma59778s31 ein C, an Markerposition ma59778s32 ein T und an Markerposition ma59778119 ein C ist, optional erweitert um einen Schritt C) Nachweis von mindestens einem chromosomalen Intervall, das nicht vom Donor stammt, oder von einer zumindest in Teilen erhöhten

Allelfrequenz in einem chromosomalen Intervall flankiert durch die Markerpositionen ma59778119 und ma20205s01 oder durch die Markerpositionen ma59778119 und ma52594s01. Vorzugsweise befindet sich das Allel aus Schritt B) in einem chromosomalen Interval flankiert durch die Markerpositionen ma59778s31 und ma59778119 oder ma59778s32 und ma59778119. In einer besonders bevorzugten Ausführungsform ist das Allel aus Schritt B) diagnostisch für die die Kühletoleranz-vermittelnde Nukleinsäure. Diagnostisch bedeutet, dass das Allel entweder direkt auf der Kühletoleranz-vermittelnden Nukleinsäure liegt bzw. die Kühletoleranz-vermittelnde Nukleinsäure ist, oder eng-gekoppelt ist zu der Kühletoleranz-vermittelnden Nukleinsäure.

In einer weiteren besonders bevorzugten Ausführungsform wird in dem Schritt B) zusätzlich zu einem ersten Allel auch ein zweites Allel nachgewiesen, wobei das eine Allel und das zweites Allel Markerpositionen darstellen, die ein chromosomales Intervall flankieren, das die Kühletoleranz-vermittelnde Nukleinsäure umfasst. Hierbei liegt vorzugsweise das erste Allel distal zu der Kühletoleranz-vermittelnden Nukleinsäure, bevorzugt in einem chromosomalen Intervall zwischen ma11840s01 und ma59778s31 oder ma11840s01 und ma59778s32, und das zweites Allel proximal zu der Kühletoleranz-vermittelnden Nukleinsäure, bevorzugt in einem chromosomalen Intervall zwischen ma59778119 und ma20205s01 oder ma59778119 und ma52594s01.

Ein weiterer Aspekt der vorliegenden Erfindung ist ein Verfahren zur Selektion einer oben beschriebenen erfindungsgemäßen kühletoleranten Maispflanze oder Teile davon, umfassend oben beschriebenes Verfahren zur Identifikation einer oben beschriebenen erfindungsgemäßen kühletoleranten Maispflanze oder Teile davon, erweitert um einen weiteren Schritt der Selektion der kühletoleranten Maispflanze oder Teile davon auf Grundlage der Nachweise des Schrittes B) und optional des Schrittes C).

Ein weiterer Aspekt beschreibt ein Verfahren zur Herstellung einer erfindungsgemäßen Maispflanze, umfassend einen ersten Schritt der Kreuzung von zwei Maispflanzen, wobei eine Maispflanze eine kühletolerante Maispflanze umfassend ein erstes chromosomales Intervall zwischen den Markerpositionen ma59778s31 und ma59778119, welches eine Kühletoleranz-vermittelnde Nukleinsäure aufweist, und ein weiteres chromosomales Intervall flankiert durch die Markerpositionen ma59778119 und ma20205s01, welches zumindest in Teilen von demselben Donor wie das erste chromosomale Intervall stammt, und/oder zumindest in Teilen eine niedrige Allelfrequenz aufweist, und als zweiten Schritt das oben beschriebenen Verfahren zur Selektion einer erfindungsgemäßen kühletoleranten Maispflanze aus den Nachkommen der Kreuzung des ersten Schrittes. Vorzugsweise umfasst die Kühletoleranz-vermittelnde Nukleinsäure eine oder mehrere Nukleinsäuresequenzen, ausgewählt aus der Gruppe bestehend aus a) einer Nukleinsäuresequenz gemäß einer der SEQ ID NOs 29, 3, 7, 11, 15, 25 oder 35, b) einer Nukleinsäuresequenz, die zu mindestens 98%, 99% oder 99,5% identisch ist mit einer Sequenz aus a) oder b), c) einer Nukleinsäuresequenz, die sich entsprechend des degenerierten genetischen Codes von einer Nukleinsäuresequenz gemäß a) ableitet, d) einer Nukleinsäuresequenz, die für ein Protein gemäß einer der SEQ ID NOs 30, 4, 8, 12, 16 oder 26 oder ein Homolog, Analog oder Ortholog davon kodiert. In einem bevorzugten Ausführungsbeispiel ist das chromosomale Intervall auf Chromosom 4, welches eine Kühletoleranz-vermittelnde Nukleinsäure aufweist, ein Intervall zwischen den Markerpositionen ma59778s32 und ma59778119 und/oder umfasst die Kühletoleranz-vermittelnde Nukleinsäure eine Nukleinsäuresequenz, ausgewählt aus der Gruppe bestehend aus a) einer Nukleinsäuresequenz gemäß der SEQ ID NOs 29, b) einer Nukleinsäuresequenz, die zu mindestens 98%, 99% oder 99,5% identisch ist mit einer Sequenz aus a), c) einer Nukleinsäuresequenz, die sich entsprechend des degenerierten genetischen Codes von einer Nukleinsäuresequenz gemäß a) ableitet, oder d) einer Nukleinsäuresequenz, die für ein Protein gemäß einer der SEQ ID NO 30 oder ein Homolog, Analog oder Ortholog davon kodiert, vorzugsweise ist die Nukleinsäuresequenz operativ verknüpft mit einem Promotor, der die Nukleotidsequenz gemäß SEQ ID NO: 33 umfasst, oder mit einer allelischen Variante oder einer modifizierte Form eines Promotor, der die Nukleotidsequenz gemäß SEQ ID NO: 34 umfasst, wobei die allelische Variante oder die modifizierte Form eine vergleichbar Expressionsrate oder Expressionshöhe wie der Promotor, der die Nukleotidsequenz gemäß SEQ ID NO: 33 umfasst, erzeugt. Eine allelischen Variante oder einer modifizierte Form des Promotor meint einen Promotor, der eine um mehr als 5%, 10%, 15%, 20%, 25%, 30%, 40% oder 50% reduzierte Expressionsrate oder Expressionshöhe im Vergleich mit der Expressionsrate bzw. Expressionshöhe, erzeugt durch den Promotor, der die Nukleotidsequenz gemäß SEQ ID NO: 34 umfasst, verursacht. Eine vergleichbare Expressionsrate oder Expressionshöhe bedeutet, das die allelische Variante oder die modifizierte Form des Promotors, der die Nukleotidsequenz gemäß SEQ ID NO: 34 umfasst, im Wesentlichen eine Expressionsrate oder Expressionshöhe aufweist, welche um nicht mehr als 20%, 18%, 16%, 14% oder 12%, bevorzugt um nicht mehr als 10%, 9%, 8%, 7% oder 6%, oder besonders bevorzugt nicht mehr als 5%, 4%, 3%, 2%, 1%, 0,5% oder 0% von der Expressionsrate oder Expressionshöhe des Promotor, der die Nukleotidsequenz gemäß SEQ ID NO: 33 umfasst, abweicht.

Ein zusätzlicher Aspekt der vorliegenden Anmeldung offenbart ein Verfahren zur Steigerung des Ertrags von Pflanzen oder Maispflanzen, umfassend den Anbau von oben beschriebenen transgenen kühletoleranten Pflanzen, mutierten kühletoleranten Pflanzen oder erfindungsgemäßen kühletoleranten Maispflanzen sowei die Ernte eines gesteigerten Ertrags. Die heranwachsenden Pflanzen verfügen über eine gesteigerte Kühletoleranz, welche sie in gemäßigten Breiten in die Lage versetzt, während einer Kühlestressphase schneller zu wachsen als eine Pflanze vergleichbaren Genotyps, die in ihrem Genom keine erfindungsgemäße Nukleinsäure enthalten. Dies führt dazu, dass zum Zeitpunkt der Ernte oben beschriebene transgene kühletolerante Pflanzen, mutierte kühletolerante Pflanzen oder erfindungsgemäße kühletolerante Maispflanzen einen gesteigerten Ertrag aufweisen.

In einem weiteren zusätzlichen Aspekt beschreibt die vorliegende Anmeldung ein Verfahren zur Verringerung des Einsatzes an Herbiziden beim Anbau von Pflanzen bzw. Maispflanzen, insbesondere während der Frühentwicklung der Pflanzen bzw. Maispflanzen, umfassend den Anbau von oben beschriebenen transgenen kühletoleranten Pflanzen, mutierten kühletoleranten Pflanzen oder erfindungsgemäßen kühletoleranten Maispflanzen. Die heranwachsenden Pflanzen verfügen über eine gesteigerte Kühletoleranz, welche sie in gemäßigten Breiten in die Lage versetzt, während einer Kühlestressphase schneller zu wachsen als eine Pflanze vergleichbaren Genotyps, die in ihrem Genom keine erfindungsgemäße Nukleinsäure enthalten. Hierdurch sind die Pflanzen kompetitiv gegenüber heranwachsendem Unkräutern/Fremdvegetation. Vorzugsweise verfügen die angebauten Pflanzen zusätzlich über eine Herbizidresistenz.

Einige der in dieser Anmeldung verwendeten Begriffe werden nachfolgend näher erläutert:
Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65 °C und anschließendes mehrfaches Waschen in 0,1 × SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

Im Sinne der Erfindung wird unter einem "Homolog" ein Protein gleichen phylogenetischen Ursprungs verstanden, unter einem "Analog" ein Protein verstanden, welches die gleiche Funktion ausübt, jedoch einen anderen phylogenetischen Ursprung hat, und unter einem "Ortholog" ein Protein aus einer anderen Spezies verstanden, das die gleiche Funktion ausübt.

Eine "Pflanze" im Sinne der Erfindung kann, sofern nicht anders angegeben von jeder Spezies aus den dikotyledonen, monokotyledonen und gymnospermen Pflanzen sein. Hierzu zählen beispielhaft *Hordeum vulgare*, *Sorghum bicolor*, *Secale cereale*, Triticale, *Saccharum officinarium*, *Zea mays*, *Setaria italic, Oryza sativa*, *Oryza minuta*, *Oryza australiensis, Oryza alta*, *Triticum aestivum, Triticum durum, Hordeum bulbosum, Brachypodium distachyon*, *Hordeum marinum*, *Aegilops tauschii, Beta vulgaris, Helianthus annuus, Daucus glochidiatus, Daucus pusillus, Daucus muricatus*, *Daucus carota, Eucalyptus grandis, Erythranthe guttata, Genlisea aurea*, *Gossypium sp., Musa sp., Avena sp., Nicotiana sylvestris, Nicotiana tabacum, Nicotiana tomentosiformis*, *Solanum lycopersicum, Solanum tuberosum, Coffea canephora, Vitis vinifera, Cucumis sativus, Morus notabilis*, *Arabidopsis thaliana, Arabidopsis lyrata*, *Arabidopsis arenosa, Crucihimalaya himalaica*, *Crucihimalaya wallichii, Cardamine flexuosa*, *Lepidium virginicum*, *Capsella bursa-pastoris, Olmarabidopsis pumila*, *Arabis hirsuta*, *Brassica napus, Brassica oleracea, Brassica rapa, Brassica juncacea, Brassica nigra, Raphanus sativus, Eruca vesicaria sativa, Citrus sinensis, Jatropha curcas, Glycine max* und *Populus trichocarpa.* Eine erfindungsgemäße Pflanze ist vorzugsweise eine Pflanze der Gattung Zea, insbesondere der Spezies Zea mays, oder Sorghum.

"Operativ verknüpft" meint verbunden in einem gemeinsamen Nukleinsäuremolekül, in einer Weise, dass die verbundenen Elemente derart zueinander positioniert und orientiert sind, dass eine Transkription des Nukleinsäuremoleküls stattfinden kann. Eine DNA, welche operativ mit einem Promotor verknüpft ist, steht unter der transkriptionellen Kontrolle dieses Promotors.

Pflanzliche "Organe" meinen beispielsweise Blätter, Sprossachse, Stamm, Wurzeln, vegetative Knospen, Meristeme, Embryos, Antheren, Ovula oder Früchte. Pflanzliche "Teile" meinen einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe. Unter pflanzlichen "Zellen" sind beispielsweise isolierte Zellen mit einer Zellwand oder Aggregate davon oder Protoplasten zu verstehen.

Ein "funktionelles Fragment" einer Nukleotidsequenz meint einen Abschnitt einer Nukleotidsequenz, welcher die identische oder eine vergleichbare Funktionalität wie die Gesamtnukleotidsequenz, aus welcher das funktionelle Fragment stammt, aufweist. Als solches kann das funktionelle Fragment eine Nukleotidsequenz besitzen, welche über eine Länge von mindestens 50%, 55%, 60%, 65%, 70%, 75%, 80%, 85%, 90%, 92%, 94% 96%, 97%, 98% oder 99% identisch oder homolog mit der Gesamtnukleotidsequenz ist. Weiterhin kann ein "funktionelles Fragment" einer Nukleotidsequenz auch einen Abschnitt einer Nukleotidsequenz meinen, welcher die Funktionalität der Gesamtnukleotidsequenz beispielsweise im Zuge von posttranskriptionalem oder transkriptionellem Gene Silencing verändert. Als solches kann das funktionelle Fragment einer Nukleotidsequenz mindestens 14, 15, 16, 17, 18, 19, 20, 21, 22, 23, 24 oder 25, bevorzugt mindestens 30, 35, 40, 45, 50, 60, 70, 80, 90, 100, 120 oder 140, besonders bevorzugt mindestens 160, 180, 200, 250, 300, 350, 400, 450, 500, 600, 700, 800, 900 oder 1000 aufeinanderfolgende Nukleotide der Gesamtnukleotidsequenz umfassen.

Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Im Zusammenhang mit der vorliegenden Erfindung betrifft der Begriff "regulatorische Sequenz" eine Nukleotidsequenz, welche die Spezifität und/oder die Expressionsstärke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

Der Begriff "chromosomales Intervall" meint einen durchgängigen linearen Abschnitt auf einer genomischen DNA, welcher in einem einzelnen Chromosom in planta oder auf einem Chromosomfragment vorliegt. Wird das chromosomale Intervall durch die Angabe zwei flankierender Markerpositionen definiert, stellen diese die Endpunkte des Intervalls zur distalen und proximalen Seite hin dar. Dabei können die Markerpositionen, die das Intervall endständig definieren selbst auch Teil des Intervalls sein. In der Beschreibung wird ein Intervall auch durch die Angabe "zwischen Markerposition A und Markerposition B" spezifiziert. Dann stellt das chromosomale Intervall einen durchgängigen linearen DNA Abschnitt dar, welcher zwischen den spezifizierten Markerpositionen lokalisiert ist. Die Markerpositionen sind nicht die Endpunkte des Intervalls zur distalen und proximalen Seite hin. Spezifizierte Markerpositionen sind selbst nicht Teil des Intervalls.

Der Begriff "Allel" betrifft eine von zwei oder mehr Nukleotidsequenzen an einem bestimmten Locus im Genom. Ein erstes Allel findet sich auf einem Chromosom, ein zweites auf einem zweiten Chromosom an derselben Position. Unterscheiden sich die beiden Allele liegen diese heterozygot vor, sind es dieselben Allele liegen sie homozygot vor. Verschiedene Allele eines Gens (Genallele) unterscheiden sich in mindestens einem SNP. Je nach Kontext der Beschreibung meint ein Allel auch nur ein einzelnes SNP, das beispielsweise eine Unterscheidung zwischen zwei Haplotypen erlaubt. Eine Maispflanze ist eine Pflanze der Spezies *Zea mays* sowie deren Subspezies wie beispielsweise *Zea mays* ssp. *mays, Zea mays* ssp. *mexicana* oder *Zea mays* ssp. *parviglumis* verstanden.

Ein "Marker" oder "molekularer Marker" ist eine Nukleotidsequenz, die als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Für Marker finden sich diese Unterschiede auf DNA-Ebene und sind beispielsweise Polynukleotidsequenzunterschiede wie zum Beispiel SSRs (simple sequence repeats), RFLPs (restriction fragment length polymorphisms), FLPs (fragment length polymorphisms) oder SNPs (single nucleotide polymorphisms). Die Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, welche genetische Polymorphismen zwischen Teilen einer Population betreffen, können mittels etablierter Verfahren aus dem Stand der Technik nachgewiesen werden (An Introduction to Genetic Analysis. 7th Edition, Griffiths, Miller, Suzuki et al., 2000). Dazu gehören z.B.: DNA-Sequenzierung, PCR-basierte, sequenzspezifische Amplifikation, Nachweis von RFLPs, Nachweis von Polynukleotidpolymorphismen mittels Allel-spezifischer Hybridisierung (ASH), Detektion von SSRs, SNPs oder AFLPs. Darüber hinaus sind auch Verfahren zur Detektion von ESTs (expressed sequence tags) und RAPD (randomly amplified polymorphic DNA) bekannt. Je nach Kontext kann der Begriff Marker in der Beschreibung auch eine spezifische Chromosomposition in dem Genom einer Spezies, wo ein spezifischer Marker (z.B. SNP) gefunden werden kann, meinen. Ein solche Marker-Position kann genutzt werden, um die Anwesenheit eines gekoppelten Locus zu verfolgen, z.B. ein gekoppelter Locus, der zur Expression eines bestimmten phänotypischen Merkmals beiträgt. Beispielsweise kann der Marker-Locus auch genutzt werden, um die Segregation von Allelen an einem Locus (QTL oder Einzelgen), die genetisch oder physikalisch eng gekoppelt sind mit der Marker-Position, zu beobachten.

Die vorliegende Erfindung soll nun im Folgenden in den Beispielen unter Bezugnahme auf die Figuren weiter erläutert werden, jedoch ohne darauf beschränkt zu werden. In den Figuren zeigt
Figur 1: Die schematische Abfolge von Kandidatengenen im Bereich zwischen den Markerpositionen ma59778s31 und ma59778119 im Vergleich zwischen SL, TH und B73 AGPv02. Durchgezogener Rahmen: annotiertes Gen; Gepunkteter Rahmen: Information aus markergestützer Kartierung; Bereiche, in welchen ein genetischer Polymorphismus zwischen der SL- und TH-Linie bestehen, sind ORF-SL-01/ORF-TH-01, ORF-SL-02/ORF-TH-02, Region-SL-13a/Region-TH-13a, Region-SL-13b/Region-TH-13b, ORF-SL-05, ORF-SL-06, Region-SL-11/0RF-TH-11, ORF-SL-12/Region-TH-12, Region-SL-06/Region-TH-06, ORF-SL-09/ORF-TH-09 und Region-SL-07/Region-TH-07; Pfeilspitzen zeigen in 5'-3'-Richtung, je nachdem auf welchem DNA-Strang das entsprechende Gen sich befindet; SL: kühlesensitiver Genotyp; TH: kühletoleranter Genotyp; B73: Mais-Linie deren Genom sequenziert wurde und welche der Fachmann für Maiszüchtung als Referenzgenom verwendet. Mithilfe der B73-Daten ist es möglich neben der markergestützten Kandidatengenposition auch deren Relativposition (relativ zum B73-Referenzgenom) anzugeben.
Figur 2: Additive Markereffekte (halbe Differenz zwischen den Mittelwerten der beiden homozygoten Markerausprägungen) für die phänotypischen Merkmale frühe Pflanzenhöhe und Blattgrüne der untersuchten rekombinanten Pflanzen nach Kühlestress in Feld-(links) und Klimakammer-(rechts) Experimenten. Die Länge eines Balkens hinter der Bezeichnung der Markerposition gibt die Größe des Einfluss der Genetik auf die Ausprägung der besagten Merkmale nach Kühlestress wieder. Demnach entscheiden die Markerpositionen ma59778s32, ma59778116 und ma59778119 in erheblichem Maße darüber, ob eine Pflanze kühletolerant ist. Messwerte und Kalkulationen zu Figur 2 können der Tabelle 5 entnommen werden.

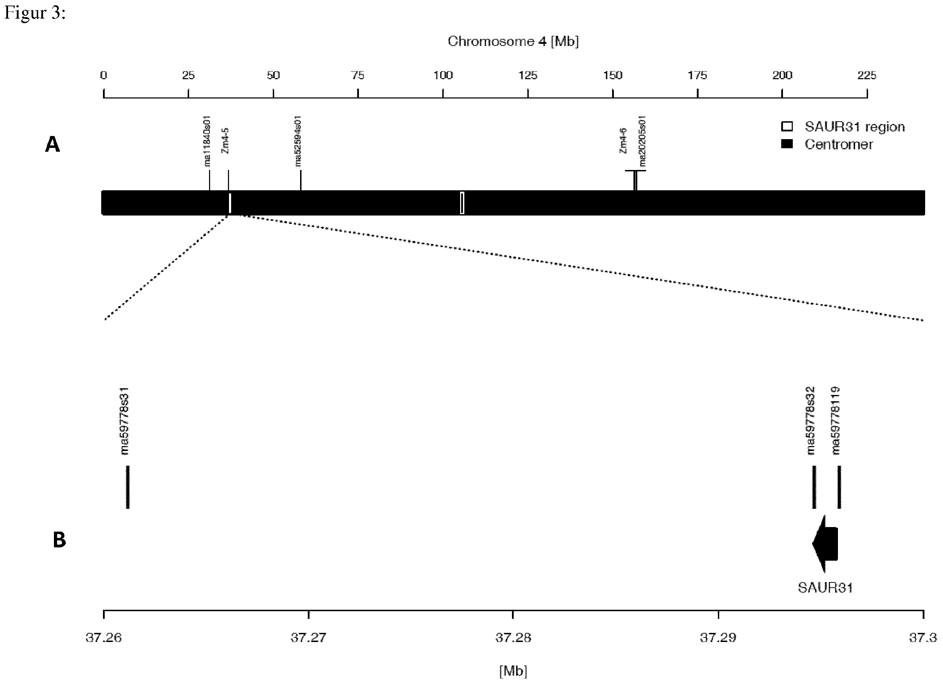

Figur 3 zeigt im oberen Bereich (A) die schematische Darstellung des Chromosoms Nr. 4 aus Zea mays mit insgesamt ca. 225 Megabasen. Die Lage des Centromers und vier wichtige Markerpositionen mit Namen sind kenntlich gemacht. Im unteren Bereich (B) ist ein Ausschnitt um den Marker Zm4-5, in welchem sich auch das Gen SAUR31 befindet, vergrößert dargestellt. Es handelt sich um eine Feinkartierung, welche mittels drei Markerpositionen die Lage von SAUR31 sehr genau wiedergibt. SAUR31 wird durch die beiden Marker ma59778s32 und ma59778119 flankiert.

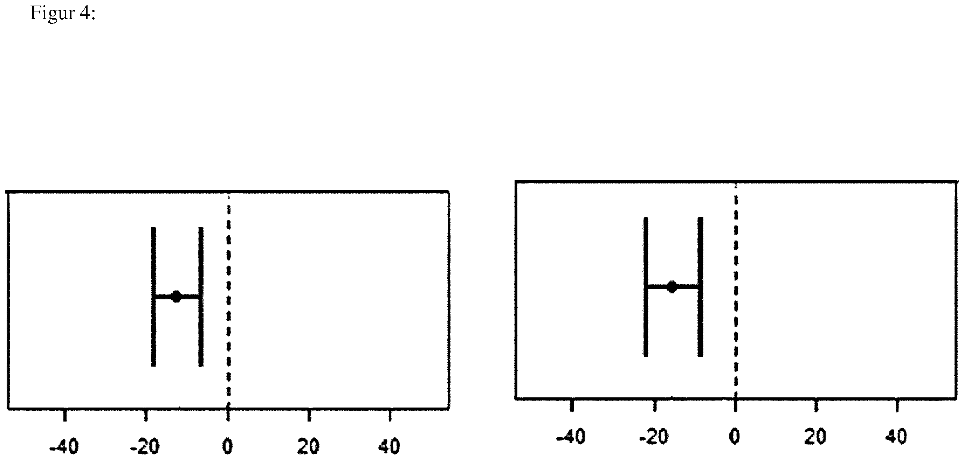

Figur 4 zeigt die Mittelwerte des Merkmals Pflanzenwuchshöhe von Pflanzen mit Mutation im 5'UTR-Bereich vor dem SAUR31-Gen im Vergleich zum Mittel der nicht-mutagenisierten Ausgangslinie. Dargestellt sind zwei unterschiedliche Messzeitpunkte (zwei unterschiedlichen Stufen der Pflanzenentwicklung): Die links dargestellte Messung erfolgte 50 Tage nach der Aussaat und die rechts dargestellte zweite Messung erfolgte 27 Tage nach der links dargestellten ersten Messung. Der Feldaufgang erfolgte unter kühlen Frühjahrsbedingungen und beide Messungen wurden vor der Maisblütc durchgeführt. Die Daten verifizieren die Bedeutung von SAUR31 an der phänotypischen Ausprägung des Merkmals Kühletoleranz.
Figur 5 zeigt das Ausmaß genetischer Diversität anhand der Allelfrequenzen im Dent-Genpool (untere Linie) und im Flint-Genpool (obere Linie) auf Chromosom 4. Maximale genetische Diversität liegt bei einer Allelfrequenz von 0,5 vor. Die Werte 0,0 und 1,0 stellen Extrama dar, welche für die vollständige Fixierung eines bestimmten genetischen Hintergrunds ohne jegliche Variabilität sprechen. Wie aus der Abbildung ersichtlich wird, findet man im Dent-Genpool eine deutliche genetische Fixierung im Vergleich zum Flint-Genpool, insbesondere angrenzend an den Bereich des Kühletoleranz vermittelnden QTL enthaltend das Gen SAUR31.

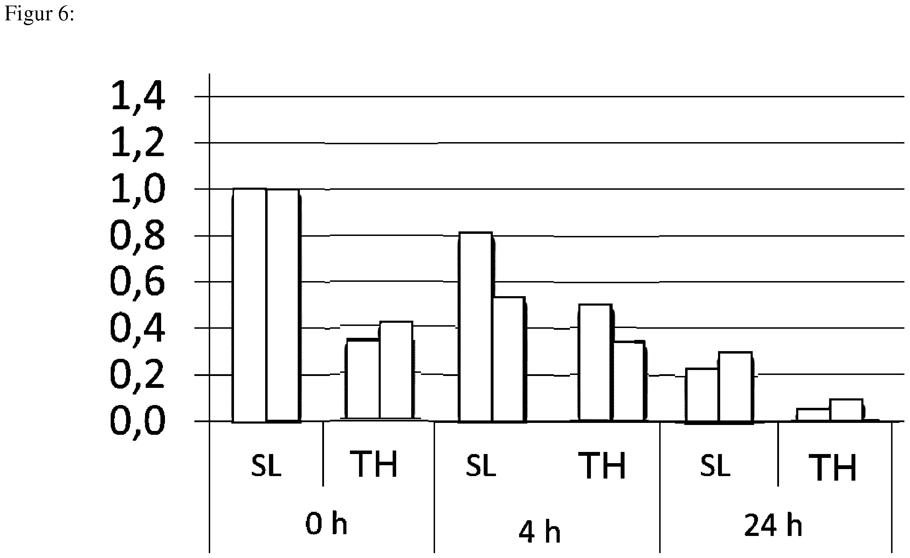

Figur 6 zeigt die relative Expression des SAUR31-Gens in kühlesensitiven und kühletoleranten Linien. Die Pflanzen wurden zwei Wochen lang bei Temperaturen von 22°C/25°C aufgezogen und anschließend einem Kühlestress von 6°C/8°C für 24h ausgesetzt. Zu Beginn der Kühlebehandlung (0h) sowie nach 4h und 24h wurden die oberirdischen Teile von acht Pflanzen zur RNA-Isolierung verwendet. Die RNA wurde mithilfe von RT-PCR untersucht. Die Versuche wurden zwei Mal durchgeführt und beide Ergebnisse sind als zwei benachbarte Balken dargestellt. Alle Werte wurden standardisiert auf den Wert SL 0h, welcher als 1 definiert wurde.

SEQ ID NOs: 1 bis 35 zeigen:
SEQ ID NO: 1 offener Leserahmen ORF-SL-01 aus der Linie SL
SEQ ID NO: 2 das von SEQ ID NO: 1 kodierte Protein
SEQ ID NO: 3 offener Leserahmen ORF-TH-01 aus der Linie TH
SEQ ID NO: 4 das von SEQ ID NO: 3 kodierte Protein
SEQ ID NO: 5 offener Leserahmen ORF-SL-02 aus der Linie SL
SEQ ID NO: 6 das von SEQ ID NO: 5 kodierte Protein
SEQ ID NO: 7 offener Leserahmen ORF-TH-02 aus der Linie TH
SEQ ID NO: 8 das von SEQ ID NO: 7 kodierte Protein
SEQ ID NO: 9 offener Leserahmen ORF-SL-03 aus der Linie SL
SEQ ID NO: 10 das von SEQ ID NO: 9 kodierte Protein
SEQ ID NO: 11 offener Leserahmen ORF-TH-03 aus der Linie TH
SEQ ID NO: 12 das von SEQ ID NO: 11 kodierte Protein
SEQ ID NO: 13 offener Leserahmen ORF-SL-04 aus der Linie SL
SEQ ID NO: 14 das von SEQ ID NO: 13 kodierte Protein
SEQ ID NO: 15 offener Leserahmen ORF-TH-04 aus der Linie TH
SEQ ID NO: 16 das von SEQ ID NO: 15 kodierte Protein
SEQ ID NO: 17 offener Leserahmen ORF-SL-05 aus der Linie SL
SEQ ID NO: 18 das von SEQ ID NO: 17 kodierte Protein
SEQ ID NO: 19 offener Leserahmen ORF-SL-06 aus der Linie SL
SEQ ID NO: 20 das von SEQ ID NO: 19 kodierte Protein
SEQ ID NO: 21 offener Leserahmen ORF-SL-12 aus der Linie SL
SEQ ID NO: 22 das von SEQ ID NO: 21 kodierte Protein
SEQ ID NO 23 offener Leserahmen ORF-SL-08 aus der Linie SL
SEQ ID NO: 24 das von SEQ ID NO: 23 kodierte Protein
SEQ ID NO: 25 offener Leserahmen ORF-TH-08 aus der Linie TH, welcher identisch ist mit ORF-SL-08 aus der Linie SL
SEQ ID NO: 26 das von SEQ ID NO: 25 kodierte Protein
SEQ ID NO: 27 offener Leserahmen ORF-SL-09 aus der Linie SL, welcher dem Gen SAUR 31 entspricht.
SEQ ID NO: 28 das von SEQ ID NO: 27 kodierte Protein
SEQ ID NO: 29 offener Leserahmen ORF-TH-09 aus der Linie TH, welcher dem Gen SAUR 31 entspricht, wobei das Gen in einer allelischen Variation vorliegt, welche maßgeblich an der Ausprägung der Kühletoleranz beteiligt ist.
SEQ ID NO: 30 das von SEQ ID NO: 29 kodierte Protein
SEQ ID NO: 31 offener Leserahmen ORF-B73-09 aus der Maisgenom-Referenzlinie B73, welcher dem Gen SAUR 31 entspricht.
SEQ ID NO: 32 das von SEQ ID NO: 31 kodierte Protein
SEQ ID NO: 33 Promotorbereich des Gens SAUR31 entsprechend der allelischen Variation ORF-TH-09, wobei der Promotorbereich in einer allelischen Variation vorliegt, welche maßgeblich an der Ausprägung der Kühletoleranz beteiligt ist.
SEQ ID NO: 34 Promotorbereich des Gens SAUR31 entsprechend der allelischen Variation ORF-SL-09
SEQ ID NO: 35 offener Leserahmen ORF-TH-11 aus der Linie TH
SEQ ID NO: 36 Primer
SEQ ID NO: 37 Primer
SEQ ID NO: 38 Mutierte Version von SAUR31 mit Basenaustausch von Adenin gegen ein Guanin an Position -25 (relativ zum Translationsstart), dargestellt ist der codogene Strang in 5' zu 3' Richtung

Beispiele:
Eine QTL Kartierungsstudie wurde in einer bi-parentalen Kartierungspopulation der durch Inzucht erzeugten Linien SL und TH durchgeführt. Die Inzucht-Linie SL ist sensitiv gegenüber kühlen Temperaturen während der frühen Pflanzenentwicklung im Feld, während TH die tolerante Elternlinie ist.

Feldexperimente wurden mit 720 DH (doppelt haploiden) Linien an 8 Standorten durchgeführt (Presterl et al., 2007). Die 720 DH Linien wurden mit 188 SSR Markern über das Genom hinweg genotypisiert. Eine Phänotypisierung der Pflanzenentwicklung wurde in einem frühen Stadium (sechs bis acht Blätter voll entwickelt) durchgeführt, und Gesamtausbeute von frischem Pflanzenmaterial und Zahl der Pflanzen wurde als Maß der Kühletoleranz im Feld bestimmt.

Die QTL Kartierung wurde auf dem Niveau der Linie *per se* und Testkreuzungen berechnet. Im Ergebnis konnten 7 QTL Regionen auf 6 Chromosomen bestimmt werden, wobei der stärkste QTL auf Chromosom 4 in einem 4 cM Intervall mit 33,7% der erklärten Phänotyp-Varianz nachgewiesen wurde. In der ersten QTL Kartierung deckten nur 3 SSR Marker die Genomregion ab (Presterl et al. 2007). Auf der B73 AGPv01 physikalischen Karte umfasst diese Region 155 Mb. Die QTL Kartierung wurde später in dieser Population verifiziert. Weitere Feinkartierung dieser Region wurde durchgeführt. 23 Marker für die QTL Region wurden entwickelt und Near lsogenic Linien (NILs) für die große QTL Region, und weitere rekombinante Pflanzen abgeleitet von Kreuzungen zwischen NILs und den sensitive Eltern SL wurden genotypisiert, um NILs mit kleineren Chromosomensegmenten zu entwickeln. Zwei flankierende polymorphe Marker der QTL Region konnten bei 36,7 Mb (Zm4-5) und 156,4 Mb (Zm4-6) auf der physikalischen Karte von B73 AGPv01 kartiert werden (siehe Figur 3). Die neu entwickelten Marker konnten bei 37,1 Mb kartiert werden. Die neuen Marker engten die QTL Region auf 119,7 Mb ein, aber aufgrund einer geringen Rekombinationsfrequenz in dieser Region (perizentromere Region) und nicht ausreichenden genomischen Ressourcen konnte kein kleineres Intervall bestimmt werden (Baliashvili, 2011).

Ein phänotypischer Test zur Bestimmung der Kühlesensitivität wurde etabliert, wobei die Pflanzen in einer Wachstumskammer für 14 Tage (Dreiblattstadium) tagsüber bei 25°C und bei 22°C in der Nacht kultiviert wurden. Danach wurde die Temperatur auf 8°C am Tag und 6°C in der Nacht für eine Woche verringert. Die Ausbeute bei 25°C am Tag und 22°C in der Nacht nach Kühle-Behandlung ergibt eine chlorotische Läsion am vierten und fünften Blatt, wenn die Pflanze gegenüber der Kühle-Behandlung empfindlich ist (Linie SL). Die toleranten Pflanzen bleiben grün (Linie TH).

Molekulare Analyse und genomische Ressourcen: Um die QTL Region mit den neuen molekularen Markern anzureichern, wurden neue genomische Ressourcen erzeugt. So ergab ein Sequenz-Capture-Ansatz mit den Linien SL, TH und der NIL TH-N4-32-Linie 28 neue polymorphe SNP-Marker im Vergleich zwischen SL und TH. BAC-Sequenzen von zwei BAC-Bibliotheken, die von einer Linie abstammten, die das sensitive SL Allel an dem QTL trug und einer Linie, die das tolerante TH Allel an dem QTL trug, wurden zudem angefertigt. BAC Bibliotheken-Screening, -Sequenzierung und - Scaffold Konstruktion wurde durchgeführt. Die BAC Bibliotheken wurden mit den bekannten Markern der QTL Region in beiden Bibliotheken gescreent. Drei BAC Klone der SL-BAC-Bibliotheken und vier BAC Klone der TH-BAC-Bibliotheken wurden mit drei verschiedenen Next Generation Techniken sequenziert. Für das SL-BAC-Scaffold konnte eine Gesamtgröße von 284 kb und für das TH-BAC-Scaffold eine Gesamtgröße von 356 kb zusammengesetzt werden, wobei beide die Zielregion zwischen ma59778s31 und ma59778119 einschließlich flankierenden Regionen enthalten. Das Fehlen polymorpher Marker in Richtung auf das Zentromer konnte durch Vergleich der BAC-Sequenzen von beiden Bibliotheken bestätigt werden. Hinter 38729663 bp auf der B73 AGPv02 Karte konnte kein Polymorphismus zwischen den zwei Linien nachgewiesen werden. An Position 37297901 bp konnte als letzter funktioneller Marker ma59778119 etabliert werden. Dieser Marker ermöglicht die 3' Bestimmung der QTL Region, da von Position 38729663 an keine weiteren Polymorphismen zwischen SL und TH beobachtet wurden. Ohne diese Marker wäre es nicht möglich gewesen, so kleine Introgressionen wie 35 kb zu identifizieren (von Marker ma59778s31 bis ma59778119) (siehe Figur 3).

Identifizierung der Kandidatengene: Die BAC Scaffolds beider BAC-Bibliotheken wurden annotiert. Kandidatengene/-regionen wurden zugewiesen, wenn sie in Übereinstimmung mit den Ergebnisse des rekombinanten Screenings waren, ihrer funktionellen Annotierung, den Ergebnissen der Expressionsanalysen, und ob sie zwischen den Linien SL und TH Polymorphismen zeigten. Insgesamt neun offene Leseraster (ORFs) konnten auf dem SL-BAC-Scaffold annotiert werden, und sieben ORFs konnten auf dem TH-BAC-Scaffold zwischen ma59778s31 und 7202707 annotiert werden. ORFs konnten nachgewiesen werden, wobei die meisten für vollständige oder verkürzte transponierbare Elemente (Tabelle 1a bis c) kodierten. Es ist bekannt, dass solche Elemente, wenn in der Nähe von Genen lokalisiert sind, deren Expression beeinflussen können (Butelli, Eugenio, et al. "Retrotransposons control fruit-specific, cold-dependent accumulation of anthocyanins in blood oranges." The Plant Cell 24.3 (2012): 1242-1255; Meihls, Lisa N., et al. "Natural variation in maize aphid resistance is associated with 2, 4-dihydroxy-7-methoxy-1, 4-benzoxazin-3-one glucoside methyltransferase activity." The Plant Cell 25.6 (2013): 2341-2355.).

Zwei annotierte ORFs (ORF-SL-03/0RF-TH-03, ORF-SL-08/ORF-TH-08) zeigten zwischen SL und TH keine genomischen Sequenzunterscheide. Vier annotierte ORFs (ORF-SL-02/ORF-TH-02, ORF-SL-04/0RF-TH-04, ORF-SL-09/ORF-TH-09, ORF-TH-11/Region-SL-11, ORF-SL-12/Region-TH-12) zeigten Polymorphismen entweder von einzelnen Nukleotiden oder Insertionen/Deletionen, ein ORF (ORF-SL-01/ORF-TH-01) kartierte nur teilweise zwischen den Genotypen. Zwei ORFs (ORF-SL-05, ORF-SL-06), die in SL identifiziert und annotiert wurden, fehlen in TH. Im Ergebnis sind alle ORFs, die polymorph sind, zwischen den zwei Genotypen fehlen oder eine unterschiedliche Expression zeigen, geeignete Kandidatengene für die beobachtete Eigenschaft der Kühletoleranz. Von besonderem Interesse ist ORF-09, der als SAUR31 in der Mais-Datenbank identifiziert wurde. SAUR-Gene (small auxin upregulated RNA) reagieren auf Auxin.

Validierung von Kandidatengenen: Das Screening einer Tilling-Population mit dem toleranten Allel von TH für die Kühletoleranz-vermittelnde QTL Region auf Chromosom 4) wurde für die Kandidatengene insbesondere ORF-SL-09, ORF-TH-09 (B73: GRMZM2G420812) begonnen. Zwei Aminosäureaustausche konnten in den Mutanten identifiziert werden, und zwei Mutanten wiesen Polymorphismen in der 5'-Region des Gens auf.

Die Expression von ausgewählten Kandidatengenen wurde durch qRT-PCR in beiden Elternlinien und in zwei NILs, die sich in der Kühletoleranz unterschieden, analysiert. Pflanzen wurden in einer Wachstumskammer unter den oben erwähnten Bedingungen angezogen, und die Expression der Kandidatengene wurde zu drei Zeitpunkten der Kühlebehandlung analysiert:
1. Vor der Kühlebehandlung (t0),
2. Vier Stunden nach Beginn der Kühlebehandlung (t4) und
3. 24 Stunden nach Beginn der Kühlebehandlung (t24).

Die Expression des SAUR31 Gens (ORF-09) war höher in den kühlesensitiven Linien und nahm mit jedem Messzeitpunkt kontinuierlich ab. Zwei der analysierten transponierbaren Elemente (ORF-08 und ORF-02) zeigten ebenfalls eine unterschiedliche Expression zwischen sensitiven und toleranten Linien (Fig. 6). Tabelle 1a bis c fasst die Kandidatengene, ihre Annotierung, die beobachteten Polymorphismen und die Ergebnisse der Expressionsanalyse zusammen. Die Unterschiede der Expression können als die Ursache der Wachstumsretardation unter kühlen Bedingungen angenommen werden.

Entwicklung einer SAUR31-Mutante und deren Analyse im Feldversuch: Aufgrund der besonderen Einflussnahme von SAUR 31 auf die phänotypische Ausprägung der Kühletoleranz wurde eine funktionale Validierung dieses Gens angestrebt. Als Ansatz wurde eine ungerichtete EMS Mutagenese von Mais-Pollen (nach Neuffer und Coe, 1978; Paraffin oil technique for treating mature corn pollen with chemical mutagens. Maydica 23: 21-28) gewählt. Nach Mutagenese einer Originallinie (KWS279) wurde M1 Saatgut ausgebracht und anschließend eine Blatternte der entsprechenden Einzelpflanzen durchgeführt. Die anschließende DNA-Extraktion aus den geernteten Blattproben gestattet mittels spezifischer Primer (SEQ ID NOs 36 und 37), ein DNA-Fragment des SAUR31 Genes zu amplifizieren. Durch Sequenzierung dieser DNA Fragmente können Abweichungen zur Originalsequenz des SAUR31 Gens gezielt detektiert und auf die entsprechende Einzelpflanze zurückgeführt werden. Mittels dieses Verfahrens konnte erfolgreich eine solche Mutante identifiziert werden, die eine Mutation des SAUR31 Gens im 5'-UTR (untranslatierter Bereich) aufweist. Hierbei ist an Position (-25), ausgehend vom Start ATG, ein Austausch von G/C nach A/T zur Originalsequenz zu beobachten. Die zugehörige Sequenz ist als SEQ ID NO: 38 dargestellt. Die in der M1 Generation identifizierte heterozygote Mutation wurde durch Selbstung der entsprechenden Einzelpflanze in der folgenden M2 Generation fixiert.

In Feldversuchen an Standort A wurde die Mutante in Reihen mit jeweils 20 Pflanzen in 5 Wiederholungen angebaut. Die nicht-mutagenisierte Originallinie wurde jeweils in direkter Nachbarschaft der Mutante angebaut, um einen bestmöglichen Vergleich zu gewährleisten. Die statistische Auswertung der Mittelwerte von Mutante zu Originallinie zeigt ein signifikant schlechteres Wachstum der Mutante unter kühlen Frühjahrsbedingungen im Vergleich zur Originallinie (Fig. 4). Fig. 4 zeigt die Mittelwerte des Merkmals Pflanzenwuchshöhe der Mutante zum Mittel der nicht-mutagenisierten Ausgangslinie an zwei unterschiedlichen Messzeitpunkten (zwei unterschiedlichen Stufen der Pflanzenentwicklung): Die in der Figur 4 rechts dargestellte zweite Messung erfolgte 27 Tage nach der links dargestellten ersten Messung. Der Feldaufgang erfolgte unter kühlen Frühjahrsbedingungen.

Entwicklung von rekombinanten NILs: Mit Hilfe der neuen molekularen Marker wurden weiterhin rekombinante NILs, die von den NILs TH-N4-8X, TH-N4-56X und TH-N4-32 stammten, entwickelt. Sehr kleine Rekombinationsereignisse konnten identifiziert werden, die 34,729 kb auf der B73AGPv02 physikalischen Karte, 32,731 kb auf dem SL-BAC Scaffold und 25,662 kb auf dem TH-BAC-Scaffold umfassen (Die Ränder werden von Marker ma59778s31 bis Marker ma59778119 vorgegeben) (Tabelle 2). Eine Übersicht der NILs, die in den verschiedenen Ansätzen verwendet wurden, ist in Tabelle 2 gezeigt.

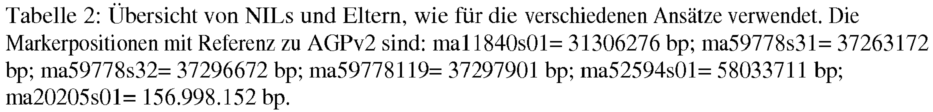

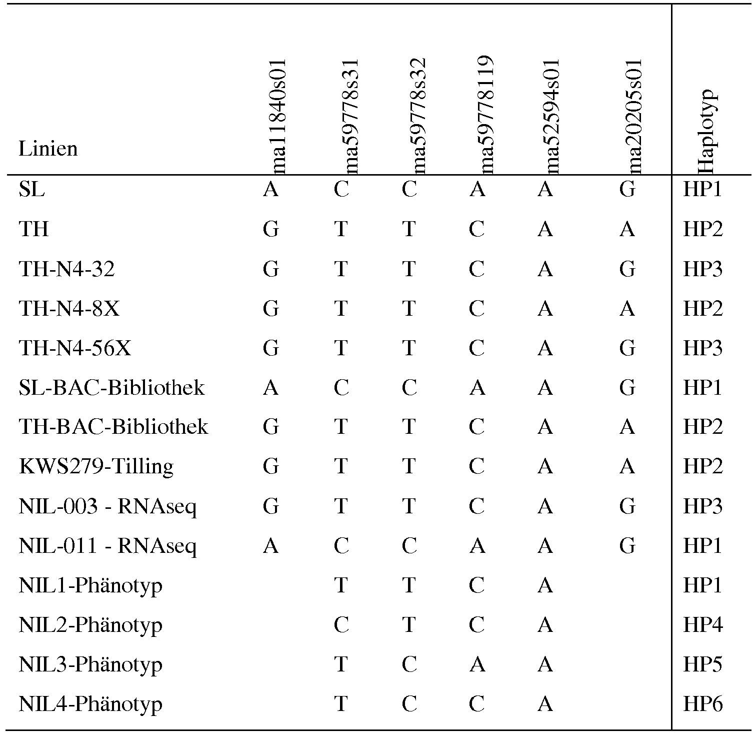

Phänotypische Evaluierung: Die NILs, welche das Donorsegment bei Marker ma59778s32 enthielten, jedoch das SL Allel bei Marker ma59778s31 enthielten, und umgekehrt, wurden im Feld und in der Wachstumskammer phänotypisiert.

NILs und die Elternlinien wurden auf Pflanzentwicklung in einem frühen Stadium an zwei Standorten A und B in Norddeutschland evaluiert, die für niedrige Temperaturen während früher Wachstumsperioden von Mais bekannt sind. Experiment 1 wurde an zwei Standorten mit 27 rekombinanten Pflanzen in 20 Replikationen durchgeführt, und Experiment 2 bestand aus 38 rekombinanten Pflanzen in 10 Replikationen, evaluiert an Standort A. In beiden Experimenten wurden NILs in einer Reihe mit je 20 Pflanzen gepflanzt. Die Pflanzenentwicklung wurde als Pflanzenhöhe zum Beginn der Verlängerungsphase des Stängels bemessen.

**Tabelle 3: Mittel der Vererbbarkeit in Abhängigkeit vom Versuchsstandort für frühe Pflanzenhöhe für Experimente 1 und 2**

| Parameter | Experiment 1 | | | Experiment 2 |
|---|---|---|---|---|
| | Mehrere Standorte | Standort A | Standort B | Standort A |
| Mittel [cm] | 67,4 | 61,0 | 73,8 | 58,0 |
| Mittel TH [cm] | 83,0 | 76,2 | 89,8 | 79,9 |
| Mittel SL [cm] | 64,8 | 59,0 | 70,6 | 58,7 |
| LSD5% [cm] | 1,9 | 2,4 | 3,0 | 2,6 |
| Vererbbarkeit [%] | 98,5 | 97,2 | 97,2 | 97,4 |

Frühe Pflanzenhöhe zeigte eine sehr hohe Vererbbarkeit (> 97%, Tabelle 3). Die zwei Elternlinien SL und TH wurden in beide Experimente eingeschlossen und unterschieden sich deutlich in der frühen Pflanzenhöhe.

Frühe Pflanzenhöhe von NILs wurde als Prozent des empfindlichen Eltern SL (Tabelle 3) berechnet. RecNILs mit identischen Genotypen in dem chromosomalen Intervall zwischen Markern ma59778s31 und ma59778119 wurden als Haplotypen zusammengefasst (Tabelle 4). RecNILs, die den SL Genotyp an Markern ma59778s32, ma59778119 und ma59778116 zeigten, hatten klar eine geringere frühe Pflanzenhöhe verglichen mit dem jeweiligen TH Genotypen. Die TH-Variante hatte überraschenderweise eine um etwa 35% gesteigerte Pflanzenlänge, welche absolut betrachtet etwa 21 zusätzliche cm ausmachte. Zwei NILs mit dem SL Genotyp an Marker ma59778s32 und TH an Markern ma59778119 und ma59778116 wiesen auch eine ähnlich geringe frühe Pflanzenhöhe auf. Zusätzlich wurden die NILs in der Klimakammer unter der Verwendung des oben angegebenen Phänotyp-Tests phänotypisiert. Die Linien wurden zwei Wochen im Gewächshaus (25/22°C) angezogen und für eine Woche in die Klimakammer bei 8/6°C transferiert (kühle Bedingungen), nach einer Woche Erholung im Gewächshaus (25/22 °C) wurde die Grünfärbung der Blätter vier und fünf zwischen 0 (gelb) und 100 % (grün) evaluiert. Hierbei steht ein Wert von 100% für vollständige Erhaltung der grünen Blattfarbe und ein Wert von 0% für eine vollständige Gelbfärbung (Chlorose). Es zeigte sich, dass die TH-Variante der SL-Variante auch bei der Erhaltung des Chlorophylls unter Kühlestress überlegen war. Insgesamt wurden Werte von 10 bis 85% gemessen. Der Blattgrünverlust der TH-Varianten war dabei um 19 bis zu 75% gegenüber den SL-Varianten reduziert.

**Tabelle 4: Tabellarische Übersicht von NIL Haplotypen, dargestellt mit unterschiedlicher Zahl (N) an einzelnen NILs und deren Phänotyp-Ergebnissen für die Eigenschaften frühe Pflanzenhöhe (SL = 100%) und Grüne der Blätter.**

| Linien | Haplotyp | Frühe Pflanzenhöhe [% SL] | N | | Grünfärbung [%] | N |
|---|---|---|---|---|---|---|
| NIL1-Phänotyp | HP2 | 107,6 | 22 | | 83,1 | 10 |
| NIL2-Phänotyp | HP4 | 106,1 | 6 | | 63,1 | 7 |
| NIL3-Phänotyp | HP5 | 92,8 | 16 | | 34,2 | 5 |
| NIL4-Phänotyp | HP6 | 94,3 | 2 | | 15,0 | 2 |
| SL | HP1 | 100 | 1 | | 10 | 1 |

Zusätzlich wurden die Effekte der Marker in dem Zielintervall für die Ergebnisse aus dem Feld und den Klimakammer-Versuchen unter der Verwendung eines Einzelmarker-Regressionsansatzes (Figur 1) berechnet. Die Marker, die physikalisch nah bei oder in dem Gen ORF-09 (ma59778120-ma59778119) sind, zeigten die größten Effekte und waren signifikant mit den zwei phänotypischen Messungen assoziiert. Im Feldversuch entspricht 6% zusätzlicher Effekt einem Unterscheid zwischen den zwei homozygoten Markerklassen von 12%. In absoluten Werten bezieht sich dies auf 7,8 cM, was 40% des phänotypischen Unterschieds zwischen SL und TH ist. Für die Wachstumskammer bezieht sich der additive Effekt auf 22,7% der zwei dichtesten flankierenden Marker.

**Tabelle 5: LOD Werte (logarithmic odds ratio, statistische Abschätzung der Wahrscheinlichkeit, ob Marker- und Merkmalsausprägung gekoppelt vererbt werden. LOD=3 wird in der Regel als Signifikanzschwelle gedeutet.) und additive Marker-Effekte (entspricht der halben Differenz der beiden homozygoten Markerausprägungen) für die Phänotypen frühe Pflanzenhöhe und Grünfärbung der Blätter der untersuchten rekombinanten Pflanzen im Feld und den Klimakammerexperimenten**

| Marker | Position | Frühe Pflanzenhöhe | | Grünfärbung | |
|---|---|---|---|---|---|
| | | LOD | Effekt [%] | LOD | Effekt [%] |
| zm00139s01 | 37227335 | 0 | 0,52 | 0 | 0,29 |
| ma59778s17 | 37250743 | 0,3 | 1,09 | 0 | 2,27 |
| ma59778s20 | 37255740 | 0,2 | 1,02 | 0 | 2,27 |
| ma59778s21 | 37255778 | 0,2 | 0,94 | 0 | 1,45 |
| ma59778s22 | 37257777 | 0,1 | 0,79 | 0 | 1,67 |
| ma59778s24 | 37258325 | 0,2 | 0,89 | 0 | 2,27 |
| ma35241s01 | 37258811 | 0,3 | 1,09 | 0,1 | 4,10 |
| ma59778s25 | 37258935 | 0,3 | 1,09 | 0 | 2,27 |
| ma59778s26 | 37260907 | 0,3 | 1,09 | 0,1 | 5,32 |
| ma59778s27 | 37260916 | 0,3 | 1,09 | 0,1 | 4,10 |
| ma59778s30 | 37263151 | 0,2 | 0,89 | 0,1 | 4,10 |
| ma59778s31 | 37263172 | 0,2 | 1,00 | 0 | 1,69 |
| ma59778s32 | 37296672 | 19,5 | 5,99 | 4,4 | 24,20 |
| ma59778119 | 37297901 | 14,1 | 5,37 | 3,2 | 22,70 |

Relevanz der entwickelten Marker für die Kühletoleranz: Die Identifikation des Kandidatengen-Haplotyps und die Entwicklung von neuen Markern ermöglichen, mithilfe von markergestützter Selektion das entsprechende Kandidatengen innerhalb eines stark eingegrenzten Intervalls in kühlesensitives Züchtungsmaterial einzukreuzen. Die auf diese Weise erzeugten Pflanzen erhalten eine gesteigerte Kühletoleranz bei gleichzeitig weitestgehend erhaltener Biodiversität.

Eine Analyse von 5.598 Genotypen aus dem züchterisch verwendeten Dent-Genpool hat gezeigt, dass durch 86% der Genotypen das Kandidatengen in der gewünschten allelischen Variante besitzen und somit den gewünschten Haplotypen aufweisen.

## Patentansprüche

1. Kühletolerante Maispflanze oder ein Teil davon, umfassend ein erstes chromosomales Intervall eines Donors auf Chromosom 4 zwischen den Markerpositionen ma59778s31 und ma59778119, welches eine endogene Kühletoleranz-vermittelnde Nukleinsäure aufweist, wobei die Kühletoleranz-vermittelnde Nukleinsäure eine Nukleinsäuresequenz umfasst, ausgewählt aus der Gruppe bestehend aus
a) einer Nukleinsäuresequenz gemäß SEQ ID NO: 29,
b) einer Nukleinsäuresequenz, die zu mindestens 98% identisch ist mit einer Sequenz aus a),
c) einer Nukleinsäuresequenz, die sich entsprechend des degenerierten genetischen Codes von einer Nukleinsäuresequenz gemäß a) ableitet,
d) einer Nukleinsäuresequenz, die für ein Protein gemäß SEQ ID NO 30 kodiert,
wobei an der Markerposition ma59778s31 ein T, an einer Markerposition ma59778s32 ein T, an der Markerposition ma59778119 ein C, an einer Markerposition ma52594s01 ein A und einer Markerposition ma20205s01 ein G ist, oder
wobei an der Markerposition ma59778s31 ein C, an einer Markerposition ma59778s32 ein T, an der Markerposition ma59778119 ein C und an einer Markerposition ma52594s01 ein A ist;
und wobei die Markerpositionen mit Referenz zur Maislinie B73 AGPv2 die folgenden sind: ma59778s31 = 37263172 bp, ma59778s32 = 37296672 bp, ma59778119 = 37297901 bp, ma52594s01 = 58033711 bp und ma20205s01 = 156.998.152 bp.

2. Maispflanze oder ein Teil davon gemäß Anspruch 1, **dadurch gekennzeichnet, dass** der Bereich auf Chromosom 4 flankiert durch die Markerpositionen ma59778119 und ma20205s01 zumindest in Teilen eine erhöhte Allelfrequenz aufweist.

3. Maispflanze oder ein Teil davon gemäß Anspruch 1, **dadurch gekennzeichnet, dass** die Nukleinsäuresequenz operativ verknüpft mit einem Promotor, der die Nukleotidsequenz gemäß SEQ ID NO: 33 umfasst, oder mit einer allelischen Variante, welche eine vergleichbare Expressionsrate oder Expressionshöhe wie der Promotor, der die Nukleotidsequenz gemäß SEQ ID NO: 33 umfasst, erzeugt, oder dass die Nukleinsäuresequenz operativ verknüpft mit einer modifizierten Form eines Promotors, der die Nukleotidsequenz gemäß SEQ ID NO: 34 umfasst, wobei die modifizierte Form eine vergleichbare Expressionsrate oder Expressionshöhe wie der Promotor, der die Nukleotidsequenz gemäß SEQ ID NO: 33 umfasst, erzeugt.

4. Verfahren zur Selektion einer kühletoleranten Maispflanze umfassend die Schritte:
i. Identifizieren einer kühletoleranten Maispflanze umfassend ein erstes chromosomales Intervall eines Donors auf Chromosom 4 zwischen den Markerpositionen ma59778s31 und ma59778119, welches eine endogene Kühletoleranz-vermittelnde Nukleinsäure aufweist, umfassend die Schritte
A) Isolierung von DNA aus dem Genom einer Maispflanze,
B) Nachweis der Allele an Marker-Positionen ma59778s31, ma59778s32 und ma59778119, welches diagnostisch für die Kühletoleranz-vermittelnde Nukleinsäure ist, in einem chromosomalen Intervall flankiert durch die Markerpositionen ma59778s31 und ma59778119,
wobei an Markerposition ma59778s31 ein T, an Markerposition ma59778s32 ein T und an Markerposition ma59778119 ein C ist, oder
wobei an Markerposition ma59778s31 ein C, an Markerposition ma59778s32 ein T und an Markerposition ma59778119 ein C ist; und
ii. Selektieren der kühletoleranten Maispflanze auf Grundlage des Nachweises in Schritt i. B);
wobei die endogene Kühletoleranz-vermittelnde Nukleinsäure eine Nukleinsäuresequenz umfasst, ausgewählt aus der Gruppe bestehend aus
a) einer Nukleinsäuresequenz gemäß SEQ ID NO 29,
b) einer Nukleinsäuresequenz, die zu mindestens 98% identisch ist mit einer Sequenz aus a),
c) einer Nukleinsäuresequenz, die sich entsprechend des degenerierten genetischen Codes von einer Nukleinsäuresequenz gemäß a) ableitet,
d) einer Nukleinsäuresequenz, die für ein Protein gemäß SEQ ID NO 30 kodiert;
wobei die Markerpositionen mit Referenz zur Maislinie B73 AGPv2 die folgenden sind: ma59778s31 = 37263172 bp, ma59778s32 = 37296672 bp und ma59778119 = 37297901 bp.

5. Verfahren zur Selektion gemäß Anspruch 4, **dadurch gekennzeichnet, dass** die Nukleinsäuresequenz operativ verknüpft mit einem Promotor, der die Nukleotidsequenz gemäß SEQ ID NO: 33 umfasst, oder mit einer allelischen Variante, welche eine vergleichbare Expressionsrate oder Expressionshöhe wie der Promotor, der die Nukleotidsequenz gemäß SEQ ID NO: 33 umfasst, erzeugt, oder dass die Nukleinsäuresequenz operativ verknüpft mit einer modifizierten Form eines Promotors, der die Nukleotidsequenz gemäß SEQ ID NO: 34 umfasst, wobei die modifizierte Form eine vergleichbare Expressionsrate oder Expressionshöhe wie der Promotor, der die Nukleotidsequenz gemäß SEQ ID NO: 33 umfasst, erzeugt.

6. Verfahren zur Selektion gemäß Anspruch 4, **dadurch gekennzeichnet, dass** der Bereich auf Chromosom 4 flankiert durch die Markerpositionen ma59778119 und ma20205s01 zumindest in Teilen eine erhöhte Allelfrequenz aufweist.

## Claims

1. A chill-tolerant maize plant or a portion thereof, comprising a first chromosomal interval from a donor on chromosome 4 between the marker positions ma59778s31 and ma59778119, which comprises an endogenous chill tolerance-conferring nucleic acid, wherein the chill tolerance-conferring nucleic acid comprises a nucleic acid sequence selected from the group consisting of
a) a nucleic acid sequence with SEQ ID NO: 29,
b) a nucleic acid sequence which has at least 98% identity with a sequence from a),
c) a nucleic acid sequence which differs from a nucleic acid sequence according to a) in accordance with the degeneracy of the genetic code,
d) a nucleic acid sequence which codes for a protein with SEQ ID NO 30,
wherein a T is at the marker position ma59778s31, a T is at a marker position ma59778s32, a C is at the marker position ma59778119, an A is at a marker position ma52594s01 and a G is at a marker position ma20205s01, or
wherein a C is at the marker position ma59778s31, a T is at a marker position ma59778s32, a C is at the marker position ma59778119 and an A is at a marker position ma52594s01;
and wherein the marker positions with reference to the maize line B73 AGPv2 are as follows: ma59778s31 = 37263172 bp, ma59778s32 = 37296672 bp, ma59778119 = 37297901 bp, ma52594s01 = 58033711 bp and ma20205s01 = 156998152 bp.

2. The maize plant or a portion thereof as claimed in claim 1, **characterized in that** the region on chromosome 4 flanked by the marker positions ma59778119 and ma20205s01 has an enhanced allele frequency, at least in parts.

3. The maize plant or a portion thereof as claimed in claim 1, **characterized in that** the nucleic acid sequence is operatively connected to a promoter which comprises the nucleotide sequence with SEQ ID NO: 33, or with an allele variant which produces a comparable expression rate or level of expression to the promoter which comprises the nucleotide sequence with SEQ ID NO: 33, or **in that** the nucleic acid sequence is operatively connected to a modified form of a promoter which comprises the nucleotide sequence with SEQ ID NO: 34, wherein the modified form has a comparable expression rate or level of expression to the promoter which comprises the nucleotide sequence with SEQ ID NO: 33.

4. A method for the selection of a chill-tolerant maize plant, comprising the steps of:
i. identification of a chill-tolerant maize plant comprising a first chromosomal interval from a donor on chromosome 4 between the marker positions ma59778s31 and ma59778119, which has an endogenous chill tolerance-conferring nucleic acid, comprising the steps of
A) isolating DNA from the genome of a maize plant,
B) detecting the alleles at the marker positions ma59778s31, ma59778s32 and ma59778119, which is diagnostic for the chill tolerance-conferring nucleic acid, in a chromosomal interval flanked by the marker positions ma59778s31 and ma59778119,
wherein a T is at the marker position ma59778s31, a T is at the marker position ma59778s32 and a C is at the marker position ma59778119, or
wherein a C is at the marker position ma59778s31, a T is at the marker position ma59778s32 and a C is at the marker position ma59778119; and
ii. selection of the chill-tolerant maize plant on the basis of the detection in step i. B);
wherein the endogenous chill tolerance-conferring nucleic acid comprises a nucleic acid sequence selected from
a) a nucleic acid sequence with SEQ ID NO 29,
b) a nucleic acid sequence which has at least 98% identity with a sequence from a),
c) a nucleic acid sequence which differs from a nucleic acid sequence according to a) in accordance with the degeneracy of the genetic code,
d) a nucleic acid sequence which codes for a protein with SEQ ID NO 30;
wherein the marker positions with reference to the maize line B73 AGPv2 are as follows: ma59778s31 = 37263172 bp, ma59778s32 = 37296672 bp and ma59778119 = 37297901 bp.

5. The selection method as claimed in claim 4, **characterized in that** the nucleic acid sequence is operatively connected to a promoter which comprises the nucleotide sequence with SEQ ID NO: 33, or with an allele variant which produces a comparable expression rate or level of expression to the promoter which comprises the nucleotide sequence with SEQ ID NO: 33, or **in that** the nucleic acid sequence is operatively connected to a modified form of a promoter which comprises the nucleotide sequence with SEQ ID NO: 34, wherein the modified form has a comparable expression rate or level of expression to the promoter which comprises the nucleotide sequence with SEQ ID NO: 33.

6. The selection method as claimed in claim 4, **characterized in that** the region on chromosome 4 flanked by the marker positions ma59778119 and ma20205s01 has an enhanced allele frequency, at least in parts.

## Revendications

1. Plant de maïs tolérant le froid ou une partie de celui-ci, comprenant un premier intervalle chromosome d'un donneur sur le chromosome 4, entre les positions des marqueurs ma59778s31 et ma59778119, lequel comporte un acide nucléique transmettant une tolérance au froid endogène, l'acide nucléique transmettant une tolérance au froid comprenant une séquence d'acide nucléique, sélectionnée dans le groupe comprenant :
a) une séquence d'acide nucléique selon SEQ ID NO : 29,
b) une séquence d'acide nucléique, qui est identique à au moins 98% avec une séquence selon a),
c) une séquence d'acide nucléique, qui se déduit en fonction du code générique dégénéré d'une séquence d'acide nucléique selon a),
d) une séquence d'acide nucléique, codant pour une protéine selon SEQ ID NO 30,
sur la position des marqueurs ma59778s32 étant un T, sur une position des marqueurs ma59778s32 étant un T et sur la position des marqueurs ma59778119 étant un C, sur une position des marqueurs ma52594s01 étant un A, et sur une position des marqueurs ma20205s01 étant un G ou
sur la position des marqueurs, ma59778s31 étant un C, sur une position des marqueurs, ma59778s32 étant un T, sur la position des marqueurs, ma59778119 étant un C et sur une position des marqueurs, ma52594s01 étant un A ;
et les positions des marqueurs en référence à la lignée de maïs B73 AGPv2 étant les suivantes : ma59778s31 = 37263172 bp, ma59778s32 = 37296672 bp, ma59778119 = 37297901 bp, ma52594s01 = 58033711 bp et ma20205s01 = 156.998.152 bp.

2. Plant de maïs ou une partie de celui-ci selon la revendication 1, **caractérisé en ce que** la zone encadrée par le chromosome 4 présente au moins partiellement par les positions des marqueurs ma59778119 et ma20205s01 une fréquence allélique augmentée.

3. Plant de maïs ou une partie de celui-ci selon la revendication 1, **caractérisé en ce que** la séquence d'acide nucléique liée de façon opérationnelle avec un promoteur qui comprend la séquence nucléotidique selon SEQ ID NO: 33 ou avec une variante allélique qui génère un taux d'expression ou un niveau d'expression comparable à celui du promoteur qui comprend la séquence nucléotidique selon SEQ ID NO: 33, ou que la séquence d'acide nucléique liée de façon opérationnelle avec une forme modifiée d'un promoteur, qui comprend la séquence nucléotidique selon SEQ ID NO: 34, la forme modifiée générant un taux d'expression ou un niveau d'expression comparable à celui du promoteur qui comprend la séquence nucléotidique selon SEQ ID NO: 33.

4. Procédé de sélection d'un plant de maïs tolérant le froid, comprenant les étapes consistant à :
i. identifier un plant de maïs tolérant le froid, comprenant un premier intervalle chromosome d'un donneur sur le chromosome 4, entre les positions des marqueurs ma59778s31 et ma59778119, lequel comporte un acide nucléique transmettant une tolérance au froid endogène, comprenant les étapes consistant à :
A) identifier l'ADN sur le génome d'un plant de maïs,
B) détecter les allèles sur des positions des marqueurs ma59778s31, ma59778s32 et ma59778119, lesquelles ont des propriétés diagnostiques pour l'acide nucléique transmettant la tolérance au froid, encadré dans un intervalle chromosome par les positions des marqueurs ma59778s31 et ma59778119,
Sur la position des marqueurs, ma59778s31 étant un T, sur la position des marqueurs ma59778s32 étant un T et sur la position des marqueurs ma59778119 étant un C, ou
Sur la position des marqueurs ma59778s3 1 étant un C, sur la position des marqueurs ma59778s32 étant un T et sur la position des marqueurs ma59778119 étant un C ; et
ii. sélectionner le plant de maïs tolérant au froid sur la base de la détection opérée dans l'étape B);
l'acide nucléique transmettant une tolérance au froid comprenant une séquence d'acide nucléique, sélectionnée dans le groupe comprenant
a) une séquence d'acide nucléique selon SEQ ID NO : 29,
b) une séquence d'acide nucléique, qui est identique à au moins 98% avec une séquence selon a),
c) une séquence d'acide nucléique, qui se déduit en fonction du code générique dégénéré d'une séquence d'acide nucléique selon a),
d) une séquence d'acide nucléique, codant pour une protéine selon SEQ ID NO 30,
les positions des marqueurs en référence à la lignée de maïs B73 AGPv2 étant les suivantes : ma59778s31 = 37263172 bp, ma59778s32 = 37296672 bp et ma59778119 = 37297901 bp.

5. Procédé de sélection selon la revendication 4, **caractérisé en ce que** la séquence d'acide nucléique liée de façon opérationnelle avec un promoteur qui comprend la séquence nucléotidique selon SEQ ID NO: 33 ou avec une variante allélique, qui génère un taux d'expression ou un niveau d'expression comparable à celui du promoteur qui comprend la séquence nucléotidique selon SEQ ID NO: 33, ou **en ce que** la séquence d'acide nucléique liée de façon opérationnelle avec une forme modifiée d'un promoteur qui comprend la séquence nucléotidique selon SEQ ID NO: 34 la forme modifiée générant un taux d'expression ou un niveau d'expression comparable à celui du promoteur qui comprend la séquence nucléotidique selon SEQ ID NO: 33.

6. Procédé de sélection selon la revendication 4, **caractérisé en ce que** la zone encadrée par le chromosome 4 présente au moins partiellement par les positions des marqueurs ma59778119 et ma20205s01 une fréquence allélique augmentée.


### SEQUENCE LISTING

<110> KWS SAAT SE
<120> Kühletolerante Pflanze
<130> KWS0225PCT
<150> EP15196721.3
   <151> 2015-11-27
<160> 38
<170> PatentIn version 3.5
<210> 1
   <211> 2016
   <212> DNA
   <213> Zea mays
<400> 1
   atggcgcccc cgagagtgga agaccccatg gcgctgttgt tggccaaaat cgatgaaggc      60
   agtaaggaaa cctgccgacg catggaggca atccattcta caatggagaa gatggagatc     120
   acagtgcaag gactggtctc caatcagagc gacttcaaga agtggcagcc cgagatcgag     180
   aggaaggtgg tggagatggc ggaaacccta gtgaagatcc aaacgaagat aagtaatacg     240
   accccgtcta ccacttcttc gggagcagta ccagcggtcg ccaacgtctc gatgtcggaa     300
   acagcttcgg tggtggtggg ggagaagatg gcgggttcta ccctgcaccg tacggcggat     360
   cccttccgac gacctgctgc cgaatcgaag gtgaaccaga tgtcgctgcc cttgggaggt     420
   atggccacgc ctaatcctca tgctccttgg ttattcggcc aaacctctgt gagtcccttt     480
   gcatctccaa cctggtcaca aggattggga ggaaacatgc caccgatgaa ttttccagtg     540
   tttgatgcat ccaatcctaa gctatggaaa aatcggtgtg aaacttattt tgagtactat     600
   gctgtcctag tggatatgtg gattcgattg gctatcatgc actttgaggg gccgactcta     660
   ttttggctgc agtatatgga aggtagaacg agggaaatga attggggtga actttgtgca     720
   gctctgctca ccagattcgg tcgtgaccag cataatttgc tcactagaca attttaccat     780
   atattccaga caggatcagt atcagattat attgaacaat ttgatttgtt attgcatcag     840
   ttgttggctc atgaaaatca tctcaccact accatggtta ctgcccgttt tgttgatgga     900
   ctgaaagacg aactaagggc agcggtaatc atacagcggc cagctgattt ggatacaaca     960
   tgttctctaa cattattaca agaagaggtc atgagtactt ccggacgtag agaactgaga    1020
   aaagtggata ctaactccat tgtcagagtt ccaaacaaac ccaatgcctt gcctatgttg    1080
   tcaggtagtc ggatatcagg ggtacaggat gaacggaggt ctatggcaac agtgggtgct    1140
   aaaggtgaaa cgagtaaaat ggaagccctc aaggcatatc ggaaggctaa gggactgtgt    1200
   tttaagtgtg gagaaagatg ggtcaacttt cacacgtgct ctaacacagt gcctttacat     1260
   ctggttgaag aaatgtgggc tctaacaatg ggtgcatctg agccggagat ggactctgaa      1320
   gagcctgcaa ctgagactag cctcgagagt gtgcttgcta tttctgttgc agcagtatct      1380
   ggcagcgaag ggagcaaaac tatcagactg tgggcatcca tttattgcca acaggttttg      1440
   gtgttagtgg attctggtag ctccgcgagt tttatggata accatctcac aggagtaatg      1500
   tccacaatga agccattacc aatgcctttg caagtgaagg ttgtcgatgg aaggacacta      1560
   tggagtactc actttgttcc tgattgccag tggctatgtg ggggacatac tttcatccat      1620
   gacttcaaaa tattaccatt gagtgggtat gatctgattc ttagtatgga ctggctggaa      1680
   aaatatagcc caatgtctat acactggggg gaaaagtggt tccaatttat atataaaggg      1740
   aagtcagtat ggctgcaagg tgttttaccc aatactcggt cttgtttttc cctaaattgt      1800
   ctccagtttg attcccttgt caaacaagat gcaattgagc agttactgga gttgcaagtc      1860
   gtaccactta gtgaacccac tgacatgcct atggtggtgg ctgatttggt taaccagttt      1920
   aaccatctgt tcgatgagcc aaaagagtta ccgcccaaga ggtggattga tcatgctata      1980
   ccactaatcc caggagctca accttttcga ctctga                                2016
<210> 2
   <211> 671
   <212> **PRT**
   <213> Zea mays
<400> 2
   Met Ala Pro Pro Arg Val Glu Asp Pro Met Ala Leu Leu Leu Ala Lys
   1               5                   10                  15
   Ile Asp Glu Gly Ser Lys Glu Thr Cys Arg Arg Met Glu Ala Ile His
               20                  25                  30
   Ser Thr Met Glu Lys Met Glu Ile Thr Val Gln Gly Leu Val Ser Asn
           35                  40                  45
   Gln Ser Asp Phe Lys Lys Trp Gln Pro Glu Ile Glu Arg Lys Val Val
       50                  55                  60
   Glu Met Ala Glu Thr Leu Val Lys Ile Gln Thr Lys Ile Ser Asn Thr
   65                  70                  75                  80
   Thr Pro Ser Thr Thr Ser Ser Gly Ala Val Pro Ala Val Ala Asn Val
                   85                  90                  95
   Ser Met Ser Glu Thr Ala Ser Val Val Val Gly Glu Lys Met Ala Gly
               100                 105                 110
   Ser Thr Leu His Arg Thr Ala Asp Pro Phe Arg Arg Pro Ala Ala Glu
           115                 120                 125
   Ser Lys Val Asn Gln Met Ser Leu Pro Leu Gly Gly Met Ala Thr Pro
       130                 135                 140
   Asn Pro His Ala Pro Trp Leu Phe Gly Gln Thr Ser Val Ser Pro Phe
   145                 150                 155                 160
   Ala Ser Pro Thr Trp Ser Gln Gly Leu Gly Gly Asn Met Pro Pro Met
                   165                 170                 175
   Asn Phe Pro Val Phe Asp Ala Ser Asn Pro Lys Leu Trp Lys Asn Arg
               180                 185                 190
   Cys Glu Thr Tyr Phe Glu Tyr Tyr Ala Val Leu Val Asp Met Trp Ile
           195                 200                 205
   Arg Leu Ala Ile Met His Phe Glu Gly Pro Thr Leu Phe Trp Leu Gln
       210                 215                 220
   Tyr Met Glu Gly Arg Thr Arg Glu Met Asn Trp Gly Glu Leu Cys Ala
   225                 230                 235                 240
   Ala Leu Leu Thr Arg Phe Gly Arg Asp Gln His Asn Leu Leu Thr Arg
                   245                 250                 255
   Gln Phe Tyr His Ile Phe Gln Thr Gly Ser Val Ser Asp Tyr Ile Glu
               260                 265                 270
   Gln Phe Asp Leu Leu Leu His Gln Leu Leu Ala His Glu Asn His Leu
           275                 280                 285
   Thr Thr Thr Met Val Thr Ala Arg Phe Val Asp Gly Leu Lys Asp Glu
       290                 295                 300
   Leu Arg Ala Ala Val Ile Ile Gln Arg Pro Ala Asp Leu Asp Thr Thr
   305                 310                 315                 320
   Cys Ser Leu Thr Leu Leu Gln Glu Glu Val Met Ser Thr Ser Gly Arg
                   325                 330                 335
   Arg Glu Leu Arg Lys Val Asp Thr Asn Ser Ile Val Arg Val Pro Asn
               340                 345                 350
   Lys Pro Asn Ala Leu Pro Met Leu Ser Gly Ser Arg Ile Ser Gly Val
           355                 360                 365
   Gln Asp Glu Arg Arg Ser Met Ala Thr Val Gly Ala Lys Gly Glu Thr
       370                 375                 380
   Ser Lys Met Glu Ala Leu Lys Ala Tyr Arg Lys Ala Lys Gly Leu Cys
   385                 390                 395                 400
   Phe Lys Cys Gly Glu Arg Trp Gly Gln Leu His Thr Cys Ser Asn Thr
                   405                 410                 415
   Val Pro Leu His Leu Val Glu Glu Met Trp Ala Leu Thr Met Gly Ala
               420                 425                 430
   Ser Glu Pro Glu Met Asp Ser Glu Glu Pro Ala Thr Glu Thr Ser Leu
           435                 440                 445
   Glu Ser Val Leu Ala Ile Ser Val Ala Ala Val Ser Gly Ser Glu Gly
       450                 455                 460
   Ser Lys Thr Ile Arg Leu Trp Ala Ser Ile Tyr Cys Gln Gln Val Leu
   465                 470                 475                 480
   Val Leu Val Asp Ser Gly Ser Ser Ala Ser Phe Met Asp Asn His Leu
                   485                 490                 495
   Thr Gly Val Met Ser Thr Met Lys Pro Leu Pro Met Pro Leu Gln Val
               500                 505                 510
   Lys Val Val Asp Gly Arg Thr Leu Trp Ser Thr His Phe Val Pro Asp
           515                 520                 525
   Cys Gln Trp Leu Cys Gly Gly His Thr Phe Ile His Asp Phe Lys Ile
       530                 535                 540
   Leu Pro Leu Ser Gly Tyr Asp Leu Ile Leu Ser Met Asp Trp Leu Glu
   545                 550                 555                 560
   Lys Tyr Ser Pro Met Ser Ile His Trp Gly Glu Lys Trp Phe Gln Phe
                   565                 570                 575
   Ile Tyr Lys Gly Lys Ser Val Trp Leu Gln Gly Val Leu Pro Asn Thr
               580                 585                 590
   Arg Ser Cys Phe Ser Leu Asn Cys Leu Gln Phe Asp Ser Leu Val Lys
           595                 600                 605
   Gln Asp Ala Ile Glu Gln Leu Leu Glu Leu Gln Val Val Pro Leu Ser
       610                 615                 620
   Glu Pro Thr Asp Met Pro Met Val Val Ala Asp Leu Val Asn Gln Phe
   625                 630                 635                 640
   Asn His Leu Phe Asp Glu Pro Lys Glu Leu Pro Pro Lys Arg Trp Ile
                   645                 650                 655
   Asp His Ala Ile Pro Leu Ile Pro Gly Ala Gln Pro Phe Arg Leu
               660                 665                 670
<210> 3
   <211> 2568
   <212> DNA
   <213> Zea mays
<400> 3
   atggataaaa atagggaccc tcaaaacaga agtggggaac agagtgaaga gggtaatggc      60
   agaggcaagt ggggggctga ccagagagtt tccggggacc gtagcgtagt ccttggttgc      120
   agctctgtta acaactctaa tggtggaccg gacctcgaag ggacaggaga tgaagctgtt     180
   ggagatgaag ggaagccgga agctgtccag aaactaagga gagcctacac tggatcctca     240
   tcacgagtac aacacgagat tgcaggccag agcgggagcg atggcgcccc gagagtggaa     300
   gaccccatgg cactgttgtt agccaaaatc gatgaaggca ataaggaaac ctgccgacgc     360
   atggaggcaa tccagtctac aatggagaag atggagatca cagtgcaagg actggtctcc     420
   gatcggagcg acttcaagaa gtggcggccc gagatcgaga ggaaggtggt ggagatggcg     480
   gaaaccctag tgaagatcca aacaaagata agtaatacga ccccgtctac cacttcttca     540
   ggagcagtac cagcggtcac caacgtctcg atgtcggcaa caacttcggt ggtggcgggg     600
   gagaagatgg cgggttctac cctgcaccgt acgacggatc ccttccgacg acctgctacc     660
   gaatcgaagg tgaaccggat gtcgctgccc ttgggaggta tggccacgcc taatcctcat     720
   gctccttggt tattcggcca aacctctgtg agtccctttg catctccaac ctggtcacaa     780
   ggattgggag gaaacatgcc accgatgaat tttccagtgt ttgatgcatc caatcctaag     840
   ctgtggaaaa atcggtgtga aacttatttt gagtactatg ctgtcctagt ggagatgtgg     900
   attcgattgg ctatcatgca ctttgagggg ccgactctat tttggctgca gtctatggaa     960
   ggtagaacga gggaaatgaa ttggggtgaa ctttgtgcag ctctgctcac cagattcagt    1020
   cgtgaccagc ataatttgct cactagacaa ttttaccata tattccagac aggatcagta    1080
   tcagattata ttgaacaatt tgatttgtta ttgcatcagt tgttggctca tgaaaatcat    1140
   ctcaccacta ccatggttac tgcccgtttt gttgatggac tgaaagacga actaagggca    1200
   acgataatca tacagcggcc agctgatttg gatacaacat gttctctagc attattacaa    1260
   gaagaggtca tgagtacttc cggacgtaga gaactgagaa aagtggatac taactccatt    1320
   gtcagagttc caaacaaacc caatgccttg cctatgttgt caggtagtcg gatatcaggg 1380
   gtacaggatg aacggaggtc tatggcaaca gtgggtgata aaggtgaaac gagtaaaatg 1440
   gaagccctca aggcatatcg gaaggctaag ggactgtgtt ttaagtgtgg agaaagatgg 1500
   ggtcaacttc acacgtgctc taacacagtg cctttacatc tggttgaaga aatgtgggct 1560
   ctaacaatgg gtgcatctga gccggagatg gactctgaag agcctgcaac tgagactagc 1620
   cttgagagtg tgcttgctat ttctgttgca gcagtatccg acagcgaagg gagcaaaact 1680
   atcagactgt gggcatccat ttattgccaa caggttttgg tgttagtgga ttctagtagc 1740
   tccgcgagtt ttatggataa ccatcttaca ggagtaatgt ccacagtgaa gccattacca 1800
   atgcctttgc aagtgaaggt tgtcgatgga aggacactat ggagtactca ctttgttcct 1860
   gattgccagt ggctatgtgg gggacatact ttcatccatg acttcaaaat attaccattg 1920
   agtgggtatg atctgattct tgtgttgtat ggccactcac ctatacattt tgggcttgtt 1980
   gacaatggtc agtgtacagt acctaatctt caagagctgt tggtagagag acagctgatg 2040
   ctccagcaag ttaagttgca tctcaatcgt gcccagcaac gtatgaaaaa acaatcggat 2100
   aaagggagga cagatcatgt ttttgaagaa ggacagcaag tgtttctcaa acttcaacct 2160
   tattgtcaat cttccgtagc ttcacgtcct tatcccaaat tggcttttaa gttctttggt 2220
   ccatttacca ttgctcgcaa ggttaatgtt gtagcttatg agttggctct tccaccaggt 2280
   tttggtattc atccggtatt ccatgtttct cagttgaagc ctcaagttgg ttccaataca 2340
   cctgttagct cattagtacc tgatatgtcg actggtttgc aagtgcctga acaaatatta 2400
   gactccaagt tggtttggcg tggaggcaaa gcactttccc atgtgttggt taaatggttg 2460
   gattgggatg tctatctagc tacgtgggaa gatgaagcag tgctgaagca acaattccct 2520
   gcagcaccag cttggggacc agctgtatct ccagggggaa tatgttaa 2568
<210> 4
   <211> 855
   <212> PRT
   <213> Zea mays
<400> 4
   Met Asp Lys Asn Arg Asp Pro Gln Asn Arg Ser Gly Glu Gln Ser Glu
   1               5                   10                  15
   Glu Gly Asn Gly Arg Gly Lys Trp Gly Ala Asp Gln Arg Val Ser Gly
               20                  25                  30
   Asp Arg Ser Val Val Leu Gly Cys Ser Ser Val Asn Asn Ser Asn Gly
           35                  40                  45
   Gly Pro Asp Leu Glu Gly Thr Gly Asp Glu Ala Val Gly Asp Glu Gly
       50                  55                  60
   Lys Pro Glu Ala Val Gln Lys Leu Arg Arg Ala Tyr Thr Gly Ser Ser
   65                  70                  75                  80
   Ser Arg Val Gln His Glu Ile Ala Gly Gln Ser Gly Ser Asp Gly Ala
                   85                  90                  95
   Pro Arg Val Glu Asp Pro Met Ala Leu Leu Leu Ala Lys Ile Asp Glu
               100                 105                 110
   Gly Asn Lys Glu Thr Cys Arg Arg Met Glu Ala Ile Gln Ser Thr Met
           115                 120                 125
   Glu Lys Met Glu Ile Thr Val Gln Gly Leu Val Ser Asp Arg Ser Asp
       130                 135                 140
   Phe Lys Lys Trp Arg Pro Glu Ile Glu Arg Lys Val Val Glu Met Ala
   145                 150                 155                 160
   Glu Thr Leu Val Lys Ile Gln Thr Lys Ile Ser Asn Thr Thr Pro Ser
                   165                 170                 175
   Thr Thr Ser Ser Gly Ala Val Pro Ala Val Thr Asn Val Ser Met Ser
               180                 185                 190
   Ala Thr Thr Ser Val Val Ala Gly Glu Lys Met Ala Gly Ser Thr Leu
           195                 200                 205
   His Arg Thr Thr Asp Pro Phe Arg Arg Pro Ala Thr Glu Ser Lys Val
       210                 215                 220
   Asn Arg Met Ser Leu Pro Leu Gly Gly Met Ala Thr Pro Asn Pro His
   225                 230                 235                 240
   Ala Pro Trp Leu Phe Gly Gln Thr Ser Val Ser Pro Phe Ala Ser Pro
                   245                 250                 255
   Thr Trp Ser Gln Gly Leu Gly Gly Asn Met Pro Pro Met Asn Phe Pro
               260                 265                 270
   Val Phe Asp Ala Ser Asn Pro Lys Leu Trp Lys Asn Arg Cys Glu Thr
           275                 280                 285
   Tyr Phe Glu Tyr Tyr Ala Val Leu Val Glu Met Trp Ile Arg Leu Ala
       290                 295                 300
   Ile Met His Phe Glu Gly Pro Thr Leu Phe Trp Leu Gln Ser Met Glu
   305                 310                 315                 320
   Gly Arg Thr Arg Glu Met Asn Trp Gly Glu Leu Cys Ala Ala Leu Leu
                   325                 330                 335
   Thr Arg Phe Ser Arg Asp Gln His Asn Leu Leu Thr Arg Gln Phe Tyr
               340                 345                 350
   His Ile Phe Gln Thr Gly Ser Val Ser Asp Tyr Ile Glu Gln Phe Asp
           355                 360                 365
   Leu Leu Leu His Gln Leu Leu Ala His Glu Asn His Leu Thr Thr Thr
       370                 375                 380
   Met Val Thr Ala Arg Phe Val Asp Gly Leu Lys Asp Glu Leu Arg Ala
   385                 390                 395                 400
   Thr Ile Ile Ile Gln Arg Pro Ala Asp Leu Asp Thr Thr Cys Ser Leu
                   405                 410                 415
   Ala Leu Leu Gln Glu Glu Val Met Ser Thr Ser Gly Arg Arg Glu Leu
               420                 425                 430
   Arg Lys Val Asp Thr Asn Ser Ile Val Arg Val Pro Asn Lys Pro Asn
           435                 440                 445
   Ala Leu Pro Met Leu Ser Gly Ser Arg Ile Ser Gly Val Gln Asp Glu
       450                 455                 460
   Arg Arg Ser Met Ala Thr Val Gly Asp Lys Gly Glu Thr Ser Lys Met
   465                 470                 475                 480
   Glu Ala Leu Lys Ala Tyr Arg Lys Ala Lys Gly Leu Cys Phe Lys Cys
                   485                 490                 495
   Gly Glu Arg Trp Gly Gln Leu His Thr Cys Ser Asn Thr Val Pro Leu
               500                 505                 510
   His Leu Val Glu Glu Met Trp Ala Leu Thr Met Gly Ala Ser Glu Pro
           515                 520                 525
   Glu Met Asp Ser Glu Glu Pro Ala Thr Glu Thr Ser Leu Glu Ser Val
       530                 535                 540
   Leu Ala Ile Ser Val Ala Ala Val Ser Asp Ser Glu Gly Ser Lys Thr
   545                 550                 555                 560
   Ile Arg Leu Trp Ala Ser Ile Tyr Cys Gln Gln Val Leu Val Leu Val
                   565                 570                 575
   Asp Ser Ser Ser Ser Ala Ser Phe Met Asp Asn His Leu Thr Gly Val
               580                 585                 590
   Met Ser Thr Val Lys Pro Leu Pro Met Pro Leu Gln Val Lys Val Val
           595                 600                 605
   Asp Gly Arg Thr Leu Trp Ser Thr His Phe Val Pro Asp Cys Gln Trp
       610                 615                 620
   Leu Cys Gly Gly His Thr Phe Ile His Asp Phe Lys Ile Leu Pro Leu
   625                 630                 635                 640
   Ser Gly Tyr Asp Leu Ile Leu Val Leu Tyr Gly His Ser Pro Ile His
                   645                 650                 655
   Phe Gly Leu Val Asp Asn Gly Gln Cys Thr Val Pro Asn Leu Gln Glu
               660                 665                 670
   Leu Leu Val Glu Arg Gln Leu Met Leu Gln Gln Val Lys Leu His Leu
           675                 680                 685
   Asn Arg Ala Gln Gln Arg Met Lys Lys Gln Ser Asp Lys Gly Arg Thr
       690                 695                 700
   Asp His Val Phe Glu Glu Gly Gln Gln Val Phe Leu Lys Leu Gln Pro
   705                 710                 715                 720
   Tyr Cys Gln Ser Ser Val Ala Ser Arg Pro Tyr Pro Lys Leu Ala Phe
                   725                 730                 735
   Lys Phe Phe Gly Pro Phe Thr Ile Ala Arg Lys Val Asn Val Val Ala
               740                 745                 750
   Tyr Glu Leu Ala Leu Pro Pro Gly Phe Gly Ile His Pro Val Phe His
           755                 760                 765
   Val Ser Gln Leu Lys Pro Gln Val Gly Ser Asn Thr Pro Val Ser Ser
       770                 775                 780
   Leu Val Pro Asp Met Ser Thr Gly Leu Gln Val Pro Glu Gln Ile Leu
   785                 790                 795                 800
   Asp Ser Lys Leu Val Trp Arg Gly Gly Lys Ala Leu Ser His Val Leu
                   805                 810                 815
   Val Lys Trp Leu Asp Trp Asp Val Tyr Leu Ala Thr Trp Glu Asp Glu
   820 825 830
   Ala Val Leu Lys Gln Gln Phe Pro Ala Ala Pro Ala Trp Gly Pro Ala
   835 840 845
   Val Ser Pro Gly Gly Ile Cys
   850 855
<210> 5
   <211> 705
   <212> DNA
   <213> Zea mays
<400> 5
   atggtgtgcc atctgagatc tgcgagcgtg ccttcgagcc ctcgctctaa tgagatccat 60
   gttgaggaac agctgcagag cctgaaggca gccatctcat caccgtcagt gaccatcaaa 120
   accatggtcg atggtctgag caagctcggg agcatctacg accgcattga tgtgctcaca 180
   tgcttgccca ccagccagag gaaggcggtg gaggaagagc tcgagcgctc cctcgtcctg 240
   cttgacctct gcagcgcctt gcaagagagc ttcgtggagc tcaaggccag tgttcaagag 300
   atgcagttgg ctctcaaaag aggagacgac gcggctctcc agaccagggt tcagtgctac 360
   gcgcgcttgg tcaagaaggc acagaagctg ttcaagaagt tcaacaagaa gactgcttct 420
   gacatcgaaa gttgcagggt gatcaacctt gttgctgaag cgagggagat tgctgtgtca 480
   accctagaat caacattgca tctcctgtca aagcaaattg caatgccaag ttgtagcaag 540
   tggtcacttg tctctaagtc tttccaaaag aagagagtca tgtgcgaggc ggatcaattg 600
   caagggctgg agctcggctt cgttgatctt gagaacagag ttgggacatt gttcaggaaa 660
   ttggtccaga acagagtgtc ttttctgaat attcttagct tgtag 705
<210> 6
   <211> 234
   <212> **PRT**
   <213> Zea mays
<400> 6
   Met Val Cys His Leu Arg Ser Ala Ser Val Pro Ser Ser Pro Arg Ser
   1 5 10 15
   Asn Glu Ile His Val Glu Glu Gln Leu Gln Ser Leu Lys Ala Ala Ile
   20 25 30
   Ser Ser Pro Ser Val Thr Ile Lys Thr Met Val Asp Gly Leu Ser Lys
   35 40 45
   Leu Gly Ser Ile Tyr Asp Arg Ile Asp Val Leu Thr Cys Leu Pro Thr
       50                  55                  60
   Ser Gln Arg Lys Ala Val Glu Glu Glu Leu Glu Arg Ser Leu Val Leu
   65                  70                  75                  80
   Leu Asp Leu Cys Ser Ala Leu Gln Glu Ser Phe Val Glu Leu Lys Ala
                   85                  90                  95
   Ser Val Gln Glu Met Gln Leu Ala Leu Lys Arg Gly Asp Asp Ala Ala
               100                 105                 110
   Leu Gln Thr Arg Val Gln Cys Tyr Ala Arg Leu Val Lys Lys Ala Gln
           115                 120                 125
   Lys Leu Phe Lys Lys Phe Asn Lys Lys Thr Ala Ser Asp Ile Glu Ser
       130                 135                 140
   Cys Arg Val Ile Asn Leu Val Ala Glu Ala Arg Glu Ile Ala Val Ser
   145                 150                 155                 160
   Thr Leu Glu Ser Thr Leu His Leu Leu Ser Lys Gln Ile Ala Met Pro
                   165                 170                 175
   Ser Cys Ser Lys Trp Ser Leu Val Ser Lys Ser Phe Gln Lys Lys Arg
               180                 185                 190
   Val Met Cys Glu Ala Asp Gln Leu Gln Gly Leu Glu Leu Gly Phe Val
           195                 200                 205
   Asp Leu Glu Asn Arg Val Gly Thr Leu Phe Arg Lys Leu Val Gln Asn
       210                 215                 220
   Arg Val Ser Phe Leu Asn Ile Leu Ser Leu
   225                 230
<210> 7
   <211> 705
   <212> DNA
   <213> Zea mays
<400> 7
   atggtgtgcc atctgagatc tgcgagcgtg ccttcgagcc ctcgctctaa tgagatccat      60
   gttgaggaac agctgcagag cctgaaggca gccatctcat caccgtcagt gaccatcaaa     120
   accatggtcg atggtctgag caagctcggg agcatctacg accgcattga tgtgctcaca     180
   tgcttgccca ccagccagag gaaggcggtg gaggaagagc tcgagcgctc cctcgtcctg     240
   cttgacctct gcagcgcctt gcaagagagc ttcgtggagc tcaaggccag tgttcaagag      300
   atgcagttgg ctctcaaaag aggagacgac gcggctctcc agaccagggt tcagtgctac      360
   gcgcgcttgg tcaagaaggc acagaagctg ttcaagaagt tcaacaagaa gactgcttct      420
   gacatcgaaa gttgcagggt gatcaacctt gttgctgaag cgagggagat tgccgtgtca      480
   accctagaat caacattgca tctcctgtca aagcaaattg caatgccaag ttgtagcaag      540
   tggtcacttg tctctaagtc tttccaaaag aagagagtca tgtgcgaggc ggatcaattg      600
   caagggctgg agctcggctt cattgatctt gagaacagag ttgggacatt gttcaggaaa      660
   ttggtccaga acagagtgtc ttttctgaat attcttagct tgtag                      705
<210> 8
   <211> 234
   <212> **PRT**
   <213> Zea mays
<400> 8
   Met Val Cys His Leu Arg Ser Ala Ser Val Pro Ser Ser Pro Arg Ser
   1               5                   10                  15
   Asn Glu Ile His Val Glu Glu Gln Leu Gln Ser Leu Lys Ala Ala Ile
               20                  25                  30
   Ser Ser Pro Ser Val Thr Ile Lys Thr Met Val Asp Gly Leu Ser Lys
           35                  40                  45
   Leu Gly Ser Ile Tyr Asp Arg Ile Asp Val Leu Thr Cys Leu Pro Thr
       50                  55                  60
   Ser Gln Arg Lys Ala Val Glu Glu Glu Leu Glu Arg Ser Leu Val Leu
   65                  70                  75                  80
   Leu Asp Leu Cys Ser Ala Leu Gln Glu Ser Phe Val Glu Leu Lys Ala
                   85                  90                  95
   Ser Val Gln Glu Met Gln Leu Ala Leu Lys Arg Gly Asp Asp Ala Ala
               100                 105                 110
   Leu Gln Thr Arg Val Gln Cys Tyr Ala Arg Leu Val Lys Lys Ala Gln
           115                 120                 125
   Lys Leu Phe Lys Lys Phe Asn Lys Lys Thr Ala Ser Asp Ile Glu Ser
       130                 135                 140
   Cys Arg Val Ile Asn Leu Val Ala Glu Ala Arg Glu Ile Ala Val Ser
   145                 150                 155                 160
   Thr Leu Glu Ser Thr Leu His Leu Leu Ser Lys Gln Ile Ala Met Pro
                   165                 170                 175
   Ser Cys Ser Lys Trp Ser Leu Val Ser Lys Ser Phe Gln Lys Lys Arg
               180                 185                 190
   Val Met Cys Glu Ala Asp Gln Leu Gln Gly Leu Glu Leu Gly Phe Ile
           195                 200                 205
   Asp Leu Glu Asn Arg Val Gly Thr Leu Phe Arg Lys Leu Val Gln Asn
       210                 215                 220
   Arg Val Ser Phe Leu Asn Ile Leu Ser Leu
   225                 230
<210> 9
   <211> 678
   <212> DNA
   <213> Zea mays
<400> 9
   atgccttcga gccctcattc cagggagacc aatgttgagg aacagattct atgcctgaaa      60
   gcagccatct ctctgccttc agtgactgtc gaaaccgtat tcgatgatct gagcaagctc     120
   gggagcatct acaaccacat cgacgcactc acatgcttgc ccaggagcca gaggaaggca     180
   gtggaggagg aggttgagca ctccctcgtc ctgctcgacc tctgcagcat tgtgcaagag     240
   agctttgttg aactcaaggc ctgtgtccag gagatacagt tggctctgaa acgaggtgat     300
   cacacagctg cccataccaa gattcagtgc tatgtgcgct cggccaagaa ggcacagaag     360
   ctgttcaaga aggtcaacaa gaagactgtc tctgacatcg aaggatgctg ggtgatcaat     420
   ctggttgctg gagcgaggga gattgctgcg ttgatccttg aatcgacatt gcatctcctg     480
   tcaaagcaaa ttgtggtcac aagttctagc aagtggtcac ttgtttccaa gtcattccga     540
   aagaagtgtg tcatatgtga ggcagaacaa ttgcaagggt tggagctgga cattgttgaa     600
   cttgagagca gagtagggac attgttcagg aagttgatcc aaagcagagt gtctcttctt     660
   aatgctctta gcttgtag                                                   678
<210> 10
   <211> 225
   <212> PRT
   <213> Zea mays
<400> 10
   Met Pro Ser Ser Pro His Ser Arg Glu Thr Asn Val Glu Glu Gln Ile
   1               5                   10                  15
   Leu Cys Leu Lys Ala Ala Ile Ser Leu Pro Ser Val Thr Val Glu Thr
               20                  25                  30
   Val Phe Asp Asp Leu Ser Lys Leu Gly Ser Ile Tyr Asn His Ile Asp
           35                  40                  45
   Ala Leu Thr Cys Leu Pro Arg Ser Gln Arg Lys Ala Val Glu Glu Glu
       50                  55                  60
   Val Glu His Ser Leu Val Leu Leu Asp Leu Cys Ser Ile Val Gln Glu
   65                  70                  75                  80
   Ser Phe Val Glu Leu Lys Ala Cys Val Gln Glu Ile Gln Leu Ala Leu
                   85                  90                  95
   Lys Arg Gly Asp His Thr Ala Ala His Thr Lys Ile Gln Cys Tyr Val
               100                 105                 110
   Arg Ser Ala Lys Lys Ala Gln Lys Leu Phe Lys Lys Val Asn Lys Lys
           115                 120                 125
   Thr Val Ser Asp Ile Glu Gly Cys Trp Val Ile Asn Leu Val Ala Gly
       130                 135                 140
   Ala Arg Glu Ile Ala Ala Leu Ile Leu Glu Ser Thr Leu His Leu Leu
   145                 150                 155                 160
   Ser Lys Gln Ile Val Val Thr Ser Ser Ser Lys Trp Ser Leu Val Ser
                   165                 170                 175
   Lys Ser Phe Arg Lys Lys Cys Val Ile Cys Glu Ala Glu Gln Leu Gln
               180                 185                 190
   Gly Leu Glu Leu Asp Ile Val Glu Leu Glu Ser Arg Val Gly Thr Leu
           195                 200                 205
   Phe Arg Lys Leu Ile Gln Ser Arg Val Ser Leu Leu Asn Ala Leu Ser
       210                 215                 220
   Leu
   225
<210> 11
   <211> 678
   <212> DNA
   <213> Zea mays
<400> 11
   atgccttcga gccctcattc cagggagacc aatgttgagg aacagattct atgcctgaaa        60
   gcagccatct ctctgccttc agtgactgtc gaaaccgtat tcgatgatct gagcaagctc       120
   gggagcatct acaaccacat cgacgcactc acatgcttgc ccaggagcca gaggaaggca       180
   gtggaggagg aggttgagca ctccctcgtc ctgctcgacc tctgcagcat tgtgcaagag       240
   agctttgttg aactcaaggc ctgtgtccag gagatacagt tggctctgaa acgaggtgat       300
   cacacagctg cccataccaa gattcagtgc tatgtgcgct cggccaagaa ggcacagaag       360
   ctgttcaaga aggtcaacaa gaagactgtc tctgacatcg aaggatgctg ggtgatcaat       420
   ctggttgctg gagcgaggga gattgctgcg ttgatccttg aatcgacatt gcatctcctg       480
   tcaaagcaaa ttgtggtcac aagttctagc aagtggtcac ttgtttccaa gtcattccga       540
   aagaagtgtg tcatatgtga ggcagaacaa ttgcaagggt tggagctgga cattgttgaa       600
   cttgagagca gagtagggac attgttcagg aagttgatcc aaagcagagt gtctcttctt       660
   aatgctctta gcttgtag                                                     678
<210> 12
   <211> 225
   <212> PRT
   <213> Zea mays
<400> 12
   Met Pro Ser Ser Pro His Ser Arg Glu Thr Asn Val Glu Glu Gln Ile
   1               5                   10                  15
   Leu Cys Leu Lys Ala Ala Ile Ser Leu Pro Ser Val Thr Val Glu Thr
               20                  25                  30
   Val Phe Asp Asp Leu Ser Lys Leu Gly Ser Ile Tyr Asn His Ile Asp
           35                  40                  45
   Ala Leu Thr Cys Leu Pro Arg Ser Gln Arg Lys Ala Val Glu Glu Glu
       50                  55                  60
   Val Glu His Ser Leu Val Leu Leu Asp Leu Cys Ser Ile Val Gln Glu
   65                  70                  75                  80
   Ser Phe Val Glu Leu Lys Ala Cys Val Gln Glu Ile Gln Leu Ala Leu
                   85                  90                  95
   Lys Arg Gly Asp His Thr Ala Ala His Thr Lys Ile Gln Cys Tyr Val
               100                 105                 110
   Arg Ser Ala Lys Lys Ala Gln Lys Leu Phe Lys Lys Val Asn Lys Lys
           115                 120                 125
   Thr Val Ser Asp Ile Glu Gly Cys Trp Val Ile Asn Leu Val Ala Gly
       130                 135                 140
   Ala Arg Glu Ile Ala Ala Leu Ile Leu Glu Ser Thr Leu His Leu Leu
   145                 150                 155                 160
   Ser Lys Gln Ile Val Val Thr Ser Ser Ser Lys Trp Ser Leu Val Ser
                   165                 170                 175
   Lys Ser Phe Arg Lys Lys Cys Val Ile Cys Glu Ala Glu Gln Leu Gln
               180                 185                 190
   Gly Leu Glu Leu Asp Ile Val Glu Leu Glu Ser Arg Val Gly Thr Leu
           195                 200                 205
   Phe Arg Lys Leu Ile Gln Ser Arg Val Ser Leu Leu Asn Ala Leu Ser
       210                 215                 220
   Leu
   225
<210> 13
   <211> 681
   <212> DNA
   <213> Zea mays
<400> 13
   atgccttcga gctctcgctc cagtgagacc tctattgacg aacagattct gagcctgaaa        60
   gcagccatct ctctgccttc agtgtccatc aaaaccatgg tggatagtct gagcaagctc       120
   ggcagcatct acaaccacat cgacgcactc acatgcttgc ccaggagcca gaggaaggca       180
   gtggaggagg agctcgagca ctccctggtc ctgctcgatc tctgcagcgc tgtgcaagag       240
   agctttgttg agcttaaggc cagtgtccag gaggtgcagt tggctctgga acgaggtgac       300
   cacacggctg cccataccaa gattcagtgc tatgtgcgct cggccaagaa ggcacagaag       360
   ctgttcaaga aggtcaacaa gaagactgcc tctgacatcg aaggatgctg ggtgattaat       420
   ctggttgctg aagcgagaga gattgccgtg ttgatccttg aatcgacatt gcatctcatg       480
   ttgaagcaaa ttgtgattcc aagctctagc aagtggtccc ttgtttccaa gtcattccga       540
   aagaagtgtg ttgtatcatg cgatgcggaa caattgcaag ggttggagct ggacgttgtt       600
   gatcttgaga gcagagttgg gacattgttc aggacgttga tccagagcag agtgtctctt       660
   cttaatgctc ttagcttgta g                                                 681
<210> 14
   <211> 226
   <212> PRT
   <213> Zea mays
<400> 14
   Met Pro Ser Ser Ser Arg Ser Ser Glu Thr Ser Ile Asp Glu Gln Ile
   1               5                   10                  15
   Leu Ser Leu Lys Ala Ala Ile Ser Leu Pro Ser Val Ser Ile Lys Thr
               20                  25                  30
   Met Val Asp Ser Leu Ser Lys Leu Gly Ser Ile Tyr Asn His Ile Asp
           35                  40                  45
   Ala Leu Thr Cys Leu Pro Arg Ser Gln Arg Lys Ala Val Glu Glu Glu
       50                  55                  60
   Leu Glu His Ser Leu Val Leu Leu Asp Leu Cys Ser Ala Val Gln Glu
   65                  70                  75                  80
   Ser Phe Val Glu Leu Lys Ala Ser Val Gln Glu Val Gln Leu Ala Leu
                   85                  90                  95
   Glu Arg Gly Asp His Thr Ala Ala His Thr Lys Ile Gln Cys Tyr Val
               100                 105                 110
   Arg Ser Ala Lys Lys Ala Gln Lys Leu Phe Lys Lys Val Asn Lys Lys
           115                 120                 125
   Thr Ala Ser Asp Ile Glu Gly Cys Trp Val Ile Asn Leu Val Ala Glu
       130                 135                 140
   Ala Arg Glu Ile Ala Val Leu Ile Leu Glu Ser Thr Leu His Leu Met
   145                 150                 155                 160
   Leu Lys Gln Ile Val Ile Pro Ser Ser Ser Lys Trp Ser Leu Val Ser
                   165                 170                 175
   Lys Ser Phe Arg Lys Lys Cys Val Val Ser Cys Asp Ala Glu Gln Leu
               180                 185                 190
   Gln Gly Leu Glu Leu Asp Val Val Asp Leu Glu Ser Arg Val Gly Thr
           195                 200                 205
   Leu Phe Arg Thr Leu Ile Gln Ser Arg Val Ser Leu Leu Asn Ala Leu
       210                 215                 220
   Ser Leu
   225
<210> 15
   <211> 681
   <212> DNA
   <213> Zea mays
<400> 15
   atgccttcga gctctcgctc cagtgagacc tctattgacg aacagattct gagcctgaaa      60
   gcagccatct ctctgccttc agtgtccatc aaaaccatgg tggatagtct gagcaagctc     120
   ggcagcatct acaaccacat cgacgcactc acatgcttgc ccaggagcca gaggaaggca     180
   gtggaggagg agctcgagca ctccctggtc ctgctcgatc tctgcagcgc tgtgcaagag     240
   agctttgttg agcttaaggc cagtgtccag gaggtgcagt tggctctgga acgaggtgac     300
   cacacggctg cccataccaa gattcagtgc tatgtgcgct cggccaagaa ggcacagaag     360
   ctgttcaaga aggtcaacaa gaagactgcc tctgacatcg aaggatgctg ggtgattaat     420
   ctggttgctg aagcgagaga gattgccgtg ttgatccttg aatcgacatt gcatctcatg     480
   ttgaagcaaa ttgtgattcc aagctctagc aagtggtccc ttgtttccaa gtcattccga     540
   aagaagtgtg ttgtatcatg cgatgcggaa caattgcaag ggttggagct ggacgttgtt     600
   gatcttgaga gcagagttgg gacattgttc aggacgttga tccagagcag agtgtctctt     660
   cttaatgctc ttagcttgta g                                               681
<210> 16
   <211> 226
   <212> PRT
   <213> Zea mays
<400> 16
   Met Pro Ser Ser Ser Arg Ser Ser Glu Thr Ser Ile Asp Glu Gln Ile
   1               5                   10                  15
   Leu Ser Leu Lys Ala Ala Ile Ser Leu Pro Ser Val Ser Ile Lys Thr
               20                  25                  30
   Met Val Asp Ser Leu Ser Lys Leu Gly Ser Ile Tyr Asn His Ile Asp
           35                  40                  45
   Ala Leu Thr Cys Leu Pro Arg Ser Gln Arg Lys Ala Val Glu Glu Glu
       50                  55                  60
   Leu Glu His Ser Leu Val Leu Leu Asp Leu Cys Ser Ala Val Gln Glu
   65                  70                  75                  80
   Ser Phe Val Glu Leu Lys Ala Ser Val Gln Glu Val Gln Leu Ala Leu
                   85                  90                  95
   Glu Arg Gly Asp His Thr Ala Ala His Thr Lys Ile Gln Cys Tyr Val
               100                 105                 110
   Arg Ser Ala Lys Lys Ala Gln Lys Leu Phe Lys Lys Val Asn Lys Lys
           115                 120                 125
   Thr Ala Ser Asp Ile Glu Gly Cys Trp Val Ile Asn Leu Val Ala Glu
       130                 135                 140
   Ala Arg Glu Ile Ala Val Leu Ile Leu Glu Ser Thr Leu His Leu Met
   145                 150                 155                 160
   Leu Lys Gln Ile Val Ile Pro Ser Ser Ser Lys Trp Ser Leu Val Ser
                   165                 170                 175
   Lys Ser Phe Arg Lys Lys Cys Val Val Ser Cys Asp Ala Glu Gln Leu
               180                 185                 190
   Gln Gly Leu Glu Leu Asp Val Val Asp Leu Glu Ser Arg Val Gly Thr
           195                 200                 205
   Leu Phe Arg Thr Leu Ile Gln Ser Arg Val Ser Leu Leu Asn Ala Leu
       210                 215                 220
   Ser Leu
   225
<210> 17
   <211> 1836
   <212> DNA
   <213> Zea mays
<400> 17
   atggagaata cgaggcgtgg gagacgaacg gaacgatcca acgcatcgtc ttcttcatcg      60
   aaacaggctg ctgcagcagc atgcataggt gggcctggcc tgagagaaag taatgcgtcg     120
   gtgggccaga gtggagacgt gctgtggcgt tgtgcagtcc agaacgaacc atacaagccg     180
   cccgtcggtc ttgtggttct ttttgacaga acacacgcgt cgcttcctct ctaccaccga     240
   gcgccaaaga cgaaacgtct ggctttaggg tcccaatcct acgcggactc aaccaaggtg     300
   ccgccgacgc tacccatgga gaaccgcggg gactcccagc aaggagtgca gggagggcga     360
   cctgtcgagc ctggcgcagg gagggctgca ccgaggcagg gattccagta caacggctca     420
   ggtcaattcc gtccaggata cggtggcggt cgtggctacg cccagaaccg ggggaggacc     480
   tggtcgcggg caggacacgg gcgcggaatg cacggcccgg ttggaggccg tggcgcggcg     540
   agacccaacc cagctggccc tggcacgatg actccggcat cgggcatgga tggcacaacg     600
   gggactggtc cggttgcagg tggaagtatt ggcacagctg gggatcaggc aggaatggca     660
   gcggtgttgc tacagcaagc actctcagct ctccagggta tgaatgccga caagggaggg        720
   ggtgctgctc aaccttctgc tgctcaacaa cctgtgatgc cggtacctag tgatgggaag        780
   aaggtacacc cgaaacctgc tgttgtggaa gagaagaaaa agtccgatca agagaaggag        840
   ggttttgtgg atgcgcctaa gaacaacaag agctactgcc ataggtgcta cggcaaggga        900
   catgtcatga gtgagtgttc gacggcgctg ttttgtgaag tatgtggcac tgatacgcac        960
   ataaagcaca aatgcccggt gttcaacgct ccgaaggttt atgcggttcc ggccggcttt       1020
   ggcatcaaca agggcggctt cttccacatt ccctcgaaca agaagctggt gaagacgaag       1080
   caagatgcta ggacagcaat gatacaggtg tcggagggac agatcagctt ggagaatgtt       1140
   aaccgtgagc tggaccgctt gcttcccggg tctgctcctt ggaaggtgga acaagtttcg       1200
   gctagttcct acagaactac ctttccatca gcctcggaac tgcagcgtat ggtggagtgg       1260
   gggccggttc gtgctaaatc acagaaggca gtgctggaat tcatagctag cactagcatg       1320
   gctgaaggac gggtcaaggc aaggctgacg gatgtgtggg tgcagtttga tggactgccg       1380
   gctcagcttt gcacttacca acacatttgg ggagtgggtt cgaaacttgg ggtcacggtt       1440
   gaagtggaca tgcctttttt ccgcaagcat gggatctgta gaatgttggt ggctgtcatt       1500
   gatccagagg caattccatt cgcaggtgat gtggaaatta acaagataat ttacgaggtg       1560
   cactattggg tggaacaagg ccctctggat gatgaaccaa cacctatggt ctctgatctt       1620
   ggcggtgatg accagggtaa cggtgacaac agcaagcaaa ataatgccaa ggaagggaat       1680
   gagcaattca aaatgccggg tgaggaaggg agagatggag aggagaagaa aggcagtaat       1740
   gatgtcgggg agcataagca agtgatggat gctgctccgg tggatgatgg tcaagccttg       1800
   caatgtgatg aggagaatag gctggctgtt ttttga                                 1836
<210> 18
   <211> 611
   <212> PRT
   <213> Zea mays
<400> 18
   Met Glu Asn Thr Arg Arg Gly Arg Arg Thr Glu Arg Ser Asn Ala Ser
   1               5                   10                  15
   Ser Ser Ser Ser Lys Gln Ala Ala Ala Ala Ala Cys Ile Gly Gly Pro
               20                  25                  30
   Gly Leu Arg Glu Ser Asn Ala Ser Val Gly Gln Ser Gly Asp Val Leu
           35                  40                  45
   Trp Arg Cys Ala Val Gln Asn Glu Pro Tyr Lys Pro Pro Val Gly Leu
       50                  55                  60
   Val Val Leu Phe Asp Arg Thr His Ala Ser Leu Pro Leu Tyr His Arg
   65                  70                  75                  80
   Ala Pro Lys Thr Lys Arg Leu Ala Leu Gly Ser Gln Ser Tyr Ala Asp
                   85                  90                  95
   Ser Thr Lys Val Pro Pro Thr Leu Pro Met Glu Asn Arg Gly Asp Ser
               100                 105                 110
   Gln Gln Gly Val Gln Gly Gly Arg Pro Val Glu Pro Gly Ala Gly Arg
           115                 120                 125
   Ala Ala Pro Arg Gln Gly Phe Gln Tyr Asn Gly Ser Gly Gln Phe Arg
       130                 135                 140
   Pro Gly Tyr Gly Gly Gly Arg Gly Tyr Ala Gln Asn Arg Gly Arg Thr
   145                 150                 155                 160
   Trp Ser Arg Ala Gly His Gly Arg Gly Met His Gly Pro Val Gly Gly
                   165                 170                 175
   Arg Gly Ala Ala Arg Pro Asn Pro Ala Gly Pro Gly Thr Met Thr Pro
               180                 185                 190
   Ala Ser Gly Met Asp Gly Thr Thr Gly Thr Gly Pro Val Ala Gly Gly
           195                 200                 205
   Ser Ile Gly Thr Ala Gly Asp Gln Ala Gly Met Ala Ala Val Leu Leu
       210                 215                 220
   Gln Gln Ala Leu Ser Ala Leu Gln Gly Met Asn Ala Asp Lys Gly Gly
   225                 230                 235                 240
   Gly Ala Ala Gln Pro Ser Ala Ala Gln Gln Pro Val Met Pro Val Pro
                   245                 250                 255
   Ser Asp Gly Lys Lys Val His Pro Lys Pro Ala Val Val Glu Glu Lys
               260                 265                 270
   Lys Lys Ser Asp Gln Glu Lys Glu Gly Phe Val Asp Ala Pro Lys Asn
           275                 280                 285
   Asn Lys Ser Tyr Cys His Arg Cys Tyr Gly Lys Gly His Val Met Ser
       290                 295                 300
   Glu Cys Ser Thr Ala Leu Phe Cys Glu Val Cys Gly Thr Asp Thr His
   305                 310                 315                 320
   Ile Lys His Lys Cys Pro Val Phe Asn Ala Pro Lys Val Tyr Ala Val
                   325                 330                 335
   Pro Ala Gly Phe Gly Ile Asn Lys Gly Gly Phe Phe His Ile Pro Ser
               340                 345                 350
   Asn Lys Lys Leu Val Lys Thr Lys Gln Asp Ala Arg Thr Ala Met Ile
           355                 360                 365
   Gln Val Ser Glu Gly Gln Ile Ser Leu Glu Asn Val Asn Arg Glu Leu
       370                 375                 380
   Asp Arg Leu Leu Pro Gly Ser Ala Pro Trp Lys Val Glu Gln Val Ser
   385                 390                 395                 400
   Ala Ser Ser Tyr Arg Thr Thr Phe Pro Ser Ala Ser Glu Leu Gln Arg
                   405                 410                 415
   Met Val Glu Trp Gly Pro Val Arg Ala Lys Ser Gln Lys Ala Val Leu
               420                 425                 430
   Glu Phe Ile Ala Ser Thr Ser Met Ala Glu Gly Arg Val Lys Ala Arg
           435                 440                 445
   Leu Thr Asp Val Trp Val Gln Phe Asp Gly Leu Pro Ala Gln Leu Cys
       450                 455                 460
   Thr Tyr Gln His Ile Trp Gly Val Gly Ser Lys Leu Gly Val Thr Val
   465                 470                 475                 480
   Glu Val Asp Met Pro Phe Phe Arg Lys His Gly Ile Cys Arg Met Leu
                   485                 490                 495
   Val Ala Val Ile Asp Pro Glu Ala Ile Pro Phe Ala Gly Asp Val Glu
               500                 505                 510
   Ile Asn Lys Ile Ile Tyr Glu Val His Tyr Trp Val Glu Gln Gly Pro
           515                 520                 525
   Leu Asp Asp Glu Pro Thr Pro Met Val Ser Asp Leu Gly Gly Asp Asp
       530                 535                 540
   Gln Gly Asn Gly Asp Asn Ser Lys Gln Asn Asn Ala Lys Glu Gly Asn
   545                 550                 555                 560
   Glu Gln Phe Lys Met Pro Gly Glu Glu Gly Arg Asp Gly Glu Glu Lys
   565 570 575
   Lys Gly Ser Asn Asp Val Gly Glu His Lys Gln Val Met Asp Ala Ala
   580 585 590
   Pro Val Asp Asp Gly Gln Ala Leu Gln Cys Asp Glu Glu Asn Arg Leu
   595 600 605
   Ala Val Phe
   610
<210> 19
   <211> 645
   <212> DNA
   <213> Zea mays
<400> 19
   atgaagatgg gaaagagtag gaaagaacca agggggcggt caggaggtat acttatgggt 60
   attgatctca atgtgaactc atcagagaag aataacgaca attttaatgc gagatggcct 120
   tttctattca atgcgcctga attcaagttc gaacttggat ggctgttgcg ggagggattc 180
   tgggagatgg tcactcaaat ttggtcaaag gagtatggtg gagatactgc cattgagaga 240
   tggcagcgaa aaataaggaa gttaagacaa tacttgagag aagtagacat gcgcagtttt 300
   cttcgtaata ggctcgcggc catgttacga gaagaagagg ttaagtggta ccagagagca 360
   aaaactaaag gtttgctgga aggggatgcg aacactaaat atttccatct ggtcgcgaat 420
   ggacgcaata tcatggaagg gatagtgatt agatggtcat ttctaggcaa taacttccaa 480
   actaagaagg ggctacggca aggccttaaa attaacttcc ataaaagtga aatcttctgc 540
   tttggtgcgg ctaaagaaag tgaacattta tactcccaac ttttcggatg tactctttcg 600
   aggaacctac tggtgtcgtt tctggggact actccaaaag cgtga 645
<210> 20
   <211> 214
   <212> PRT
   <213> Zea mays
<400> 20
   Met Lys Met Gly Lys Ser Arg Lys Glu Pro Arg Gly Arg Ser Gly Gly
   1               5                   10                  15
   Ile Leu Met Gly Ile Asp Leu Asn Val Asn Ser Ser Glu Lys Asn Asn
               20                  25                  30
   Asp Asn Phe Asn Ala Arg Trp Pro Phe Leu Phe Asn Ala Pro Glu Phe
           35                  40                  45
   Lys Phe Glu Leu Gly Trp Leu Leu Arg Glu Gly Phe Trp Glu Met Val
       50                  55                  60
   Thr Gln Ile Trp Ser Lys Glu Tyr Gly Gly Asp Thr Ala Ile Glu Arg
   65                  70                  75                  80
   Trp Gln Arg Lys Ile Arg Lys Leu Arg Gln Tyr Leu Arg Glu Val Asp
                   85                  90                  95
   Met Arg Ser Phe Leu Arg Asn Arg Leu Ala Ala Met Leu Arg Glu Glu
               100                 105                 110
   Glu Val Lys Trp Tyr Gln Arg Ala Lys Thr Lys Gly Leu Leu Glu Gly
           115                 120                 125
   Asp Ala Asn Thr Lys Tyr Phe His Leu Val Ala Asn Gly Arg Asn Ile
       130                 135                 140
   Met Glu Gly Ile Val Ile Arg Trp Ser Phe Leu Gly Asn Asn Phe Gln
   145                 150                 155                 160
   Thr Lys Lys Gly Leu Arg Gln Gly Leu Lys Ile Asn Phe His Lys Ser
                   165                 170                 175
   Glu Ile Phe Cys Phe Gly Ala Ala Lys Glu Ser Glu His Leu Tyr Ser
               180                 185                 190
   Gln Leu Phe Gly Cys Thr Leu Ser Arg Asn Leu Leu Val Ser Phe Leu
           195                 200                 205
   Gly Thr Thr Pro Lys Ala
       210
<210> 21
   <211> 234
   <212> DNA
   <213> Zea mays
<400> 21
   atgcgagtca tatgcgttgt tgctagatac atgtctaatc ctgattatgg tggtgattta    60
   gacaggagga gatctctttc agaagctgag tatatggcaa ttgcagaagt tactaaggaa   120
   gccttatggt tgaaagatca gatgattact gagaaatcca aacatattga tattcgttat   180
   cacttcattc gtgatatcat tggagaacgt gtatttgcac agcagtttaa atga         234
<210> 22
   <211> 77
   <212> PRT
   <213> Zea mays
<400> 22
   Met Arg Val Ile Cys Val Val Ala Arg Tyr Met Ser Asn Pro Asp Tyr
   1               5                   10                  15
   Gly Gly Asp Leu Asp Arg Arg Arg Ser Leu Ser Glu Ala Glu Tyr Met
               20                  25                  30
   Ala Ile Ala Glu Val Thr Lys Glu Ala Leu Trp Leu Lys Asp Gln Met
           35                  40                  45
   Ile Thr Glu Lys Ser Lys His Ile Asp Ile Arg Tyr His Phe Ile Arg
       50                  55                  60
   Asp Ile Ile Gly Glu Arg Val Phe Ala Gln Gln Phe Lys
   65                  70                  75
<210> 23
   <211> 693
   <212> DNA
   <213> Zea mays
<400> 23
   atggcgtgcc acctgagatc tgctagcatg ccttcgagcc ctcgctccgt tgaggaacag      60
   attctgagcc tgaaagtagc catctctctg ccttcagtga ccatcgaaac catggtggat     120
   agtctgagca agctcgggag catctacagc cacatagacg cgctcgcatc cctgcccagc     180
   tgccagagga aggcaatgga ggaggagctc gagcgctccg ttgtcctgct tgacctctgc     240
   agcgccatgc aagagagctt tgcagaactc aaggccagtg tccaggagac gcagttggct     300
   ctcaaaagag gagacgacgc ggctcttcat gccaagattc agtgctatgc gcgctcagct     360
   aagaaggcac agaagctgtt caagaaggtc aacaagaaga ctgcctccga catcaaagga     420
   tgcagggtga tcagcctggt cgctgaagcg agggaagttg ccctatcgat cctcgagtcg     480
   acactgcatc tcctggcgaa gcagattgcg gtcccaagtc ccagcaagtg gtcacttgta     540
   tccaaatcgt tccagaagaa gagaatcatg tgtgaggcgg agcagttgca agggttggag     600
   ccggagattg ctggtcttga gagcggagtt gggactttgt tcaggacgtt gatccagagc     660
   agagtttctc ttctcaatgc tcttagtttg tag                                  693
<210> 24
   <211> 214
   <212> PRT
   <213> Zea mays
<400> 24
   Met Lys Met Gly Lys Ser Arg Lys Glu Pro Arg Gly Arg Ser Gly Gly
   1               5                   10                  15
   Ile Leu Met Gly Ile Asp Leu Asn Val Asn Ser Ser Glu Lys Asn Asn
               20                  25                  30
   Asp Asn Phe Asn Ala Arg Trp Pro Phe Leu Phe Asn Ala Pro Glu Phe
           35                  40                  45
   Lys Phe Glu Leu Gly Trp Leu Leu Arg Glu Gly Phe Trp Glu Met Val
       50                  55                  60
   Thr Gln Ile Trp Ser Lys Glu Tyr Gly Gly Asp Thr Ala Ile Glu Arg
   65                  70                  75                  80
   Trp Gln Arg Lys Ile Arg Lys Leu Arg Gln Tyr Leu Arg Glu Val Asp
                   85                  90                  95
   Met Arg Ser Phe Leu Arg Asn Arg Leu Ala Ala Met Leu Arg Glu Glu
               100                 105                 110
   Glu Val Lys Trp Tyr Gln Arg Ala Lys Thr Lys Gly Leu Leu Glu Gly
           115                 120                 125
   Asp Ala Asn Thr Lys Tyr Phe His Leu Val Ala Asn Gly Arg Asn Ile
       130                 135                 140
   Met Glu Gly Ile Val Ile Arg Trp Ser Phe Leu Gly Asn Asn Phe Gln
   145                 150                 155                 160
   Thr Lys Lys Gly Leu Arg Gln Gly Leu Lys Ile Asn Phe His Lys Ser
                   165                 170                 175
   Glu Ile Phe Cys Phe Gly Ala Ala Lys Glu Ser Glu His Leu Tyr Ser
               180                 185                 190
   Gln Leu Phe Gly Cys Thr Leu Ser Arg Asn Leu Leu Val Ser Phe Leu
           195                 200                 205
   Gly Thr Thr Pro Lys Ala
       210
<210> 25
   <211> 693
   <212> DNA
   <213> Zea mays
<400> 25
   atggcgtgcc acctgagatc tgctagcatg ccttcgagcc ctcgctccgt tgaggaacag        60
   attctgagcc tgaaagtagc catctctctg ccttcagtga ccatcgaaac catggtggat       120
   agtctgagca agctcgggag catctacagc cacatagacg cgctcgcatc cctgcccagc       180
   tgccagagga aggcaatgga ggaggagctc gagcgctccg ttgtcctgct tgacctctgc       240
   agcgccatgc aagagagctt tgcagaactc aaggccagtg tccaggagac gcagttggct       300
   ctcaaaagag gagacgacgc ggctcttcat gccaagattc agtgctatgc gcgctcagct       360
   aagaaggcac agaagctgtt caagaaggtc aacaagaaga ctgcctccga catcaaagga       420
   tgcagggtga tcagcctggt cgctgaagcg agggaagttg ccctatcgat cctcgagtcg       480
   acactgcatc tcctggcgaa gcagattgcg gtcccaagtc ccagcaagtg gtcacttgta       540
   tccaaatcgt tccagaagaa gagaatcatg tgtgaggcgg agcagttgca agggttggag       600
   ccggagattg ctggtcttga gagcggagtt gggactttgt tcaggacgtt gatccagagc       660
   agagtttctc ttctcaatgc tcttagtttg tag                                    693
<210> 26
   <211> 230
   <212> PRT
   <213> Zea mays
<400> 26
   Met Ala Cys His Leu Arg Ser Ala Ser Met Pro Ser Ser Pro Arg Ser
   1               5                   10                  15
   Val Glu Glu Gln Ile Leu Ser Leu Lys Val Ala Ile Ser Leu Pro Ser
               20                  25                  30
   Val Thr Ile Glu Thr Met Val Asp Ser Leu Ser Lys Leu Gly Ser Ile
           35                  40                  45
   Tyr Ser His Ile Asp Ala Leu Ala Ser Leu Pro Ser Cys Gln Arg Lys
       50                  55                  60
   Ala Met Glu Glu Glu Leu Glu Arg Ser Val Val Leu Leu Asp Leu Cys
   65                  70                  75                  80
   Ser Ala Met Gln Glu Ser Phe Ala Glu Leu Lys Ala Ser Val Gln Glu
                   85                  90                  95
   Thr Gln Leu Ala Leu Lys Arg Gly Asp Asp Ala Ala Leu His Ala Lys
               100                 105                 110
   Ile Gln Cys Tyr Ala Arg Ser Ala Lys Lys Ala Gln Lys Leu Phe Lys
           115                 120                 125
   Lys Val Asn Lys Lys Thr Ala Ser Asp Ile Lys Gly Cys Arg Val Ile
       130                 135                 140
   Ser Leu Val Ala Glu Ala Arg Glu Val Ala Leu Ser Ile Leu Glu Ser
   145                 150                 155                 160
   Thr Leu His Leu Leu Ala Lys Gln Ile Ala Val Pro Ser Pro Ser Lys
                   165                 170                 175
   Trp Ser Leu Val Ser Lys Ser Phe Gln Lys Lys Arg Ile Met Cys Glu
               180                 185                 190
   Ala Glu Gln Leu Gln Gly Leu Glu Pro Glu Ile Ala Gly Leu Glu Ser
           195                 200                 205
   Gly Val Gly Thr Leu Phe Arg Thr Leu Ile Gln Ser Arg Val Ser Leu
       210                 215                 220
   Leu Asn Ala Leu Ser Leu
   225                 230
<210> 27
   <211> 309
   <212> DNA
   <213> Zea mays
<400> 27
   atggcggcgg gaaagctggg gcagcagctg atgacgaggc tgcacctcgc gaggacccga      60
   tcgtcggcga cggcggacgt gccgcggggc cacctggcgg tgtacgtggg cgaggggcgg     120
   aagcggctgg tcatcccgac ggcgtgcctc agccacccgg ccttcgtcac gctgctgaag     180
   cgggtggagg acgagttcgg cttcgaccac cgctgcggcg gcctcaccat cccctgcgcc     240
   tccgagaccg agttcgctca catcgtcggc gccgccgccg ccgccgggga cgaccaccac     300
   catcactga                                                             309
<210> 28
   <211> 102
   <212> PRT
   <213> Zea mays
<400> 28
   Met Ala Ala Gly Lys Leu Gly Gln Gln Leu Met Thr Arg Leu His Leu
   1               5                   10                  15
   Ala Arg Thr Arg Ser Ser Ala Thr Ala Asp Val Pro Arg Gly His Leu
               20                  25                  30
   Ala Val Tyr Val Gly Glu Gly Arg Lys Arg Leu Val Ile Pro Thr Ala
           35                  40                  45
   Cys Leu Ser His Pro Ala Phe Val Thr Leu Leu Lys Arg Val Glu Asp
       50                  55                  60
   Glu Phe Gly Phe Asp His Arg Cys Gly Gly Leu Thr Ile Pro Cys Ala
   65                  70                  75                  80
   Ser Glu Thr Glu Phe Ala His Ile Val Gly Ala Ala Ala Ala Ala Gly
                   85                  90                  95
   Asp Asp His His His His
               100
<210> 29
   <211> 309
   <212> DNA
   <213> Zea mays
<400> 29
   atggcggcgg gaaagctggg gcagcagctg atgacgaggc tgcacctcgc gaggacccga        60
   tcgtcggcga cggcggacgt gccgcggggc cacctggcgg tgtacgtggg cgaggggcgg       120
   aagcggctgg tcatcccgac ggcgtgcctc agccacccgg ccttcgtcac gctgctcaag       180
   cgggtggagg acgagttcgg cttcgaccac cgctgcggcg gcctcaccat cccctgcgcc       240
   tccgagaccg agttcgctca catcgtcggc gccgccgccg ccgccgggga cgaccaccac       300
   catcactga                                                               309
<210> 30
   <211> 102
   <212> PRT
   <213> Zea mays
<400> 30
   Met Ala Ala Gly Lys Leu Gly Gln Gln Leu Met Thr Arg Leu His Leu
   1               5                   10                  15
   Ala Arg Thr Arg Ser Ser Ala Thr Ala Asp Val Pro Arg Gly His Leu
               20                  25                  30
   Ala Val Tyr Val Gly Glu Gly Arg Lys Arg Leu Val Ile Pro Thr Ala
           35                  40                  45
   Cys Leu Ser His Pro Ala Phe Val Thr Leu Leu Lys Arg Val Glu Asp
       50                  55                  60
   Glu Phe Gly Phe Asp His Arg Cys Gly Gly Leu Thr Ile Pro Cys Ala
   65                  70                  75                  80
   Ser Glu Thr Glu Phe Ala His Ile Val Gly Ala Ala Ala Ala Ala Gly
                   85                  90                  95
   Asp Asp His His His His
               100
<210> 31
   <211> 512
   <212> DNA
   <213> Zea mays
<400> 31
   atggcggcgg gaaagctggg gcagcagctg atgacgaggc tgcacctcgc gaggacccga      60
   ccgtcggcga cggcggacgt gccgcggggc cacctggcgg tgtacgtggg cgaggggcgg     120
   aagcggctgg tcatcccaac ggcgtgcctc agccacccag ccttcgtcac gctgctgaag     180
   cgggtggagg acgagttcgg cttcgaccac cgctgcggcg gcctcaccat cccctgcgcc     240
   tccgagaccg agttcgctca catcgtgggc gccgccgccg ccggggacgg ccaccaccat     300
   cactgacgat cgcgtgcgtg cccgcgccga tcgatcgagt tagagtccgg ccgtgtcgat     360
   agattaattc cgcttccagt tccacctagc taggacaaaa ttattgttct cttttggggt     420
   ggtgtcgatc gtagcagcaa tagtgttggg ttttgcttga cgacactgta aagattgtga     480
   ttgggaattg gaagaataag ctcttctctc ac                                   512
<210> 32
   <211> 101
   <212> PRT
   <213> Zea mays
<400> 32
   Met Ala Ala Gly Lys Leu Gly Gln Gln Leu Met Thr Arg Leu His Leu
   1               5                   10                  15
   Ala Arg Thr Arg Pro Ser Ala Thr Ala Asp Val Pro Arg Gly His Leu
               20                  25                  30
   Ala Val Tyr Val Gly Glu Gly Arg Lys Arg Leu Val Ile Pro Thr Ala
           35                  40                  45
   Cys Leu Ser His Pro Ala Phe Val Thr Leu Leu Lys Arg Val Glu Asp
       50                  55                  60
   Glu Phe Gly Phe Asp His Arg Cys Gly Gly Leu Thr Ile Pro Cys Ala
   65                  70                  75                  80
   Ser Glu Thr Glu Phe Ala His Ile Val Gly Ala Ala Ala Ala Gly Asp
                   85                  90                  95
   Gly His His His His
               100
<210> 33
   <211> 1501
   <212> DNA
   <213> Zea mays
<400> 33
   cctatgcaaa cctagattaa atttatagtg ggatttaacc caaataatgc atgccccgct      60
   aatgggtgat ggatttcccc tcataagttt ttaccatatg gtcatttcct gccatcctaa     120
   caacttctaa ttcatgaatt ggattggttg acataacccc ataaaattgt gggttgggta     180
   aagcagttaa tttgacatgg ggttgaggta gatatgggat ggaatttttg ttttaagagc     240
   aatggcatca gctctactat ttattaattt aaaagggaaa acaaatagtt cataaaattg     300
   tgtagagtag aagctagcta gctagcattg gtcagaataa gcaggacaca cctgggtgag     360
   agaagagagc ttattcttcc aatccccaat cacaatcttt acagtgtcgt caagcaaaaa     420
   cccaatcact attgctgcta cgacaccacc ccaaagagaa caataatttt gtcctagcta     480
   ggtggaactg gaagcggaat taatctatcg acacggccgg actcgatcga tcggcgcggg     540
   cacgcacgcg atcgtcagtg atggtggtgg tcgtccccgg cggcggcggc ggcgccgacg     600
   atgtgagcga actcggtctc ggaggcgcag gggatggtga ggccgccgca gcggtggtcg     660
   aagccgaact cgtcctccac ccgcttgagc agcgtgacga aggccgggtg gctgaggcac     720
   gccgtcggga tgaccagccg cttccgcccc tcgcccacgt acaccgccag gtggccccgc     780
   ggcacgtccg ccgtcgccga cgatcgggtc ctcgcgaggt gcagcctcgt catcagctgc     840
   tgccccagct ttcccgccgc catctctagc tctagctgtg tgtgtcggtg attgttgcac     900
   aaagtcgtgt gtatagctct agcttgctat agctagagtg gtgctgctag atttggagct     960
   caagagcttt gtgtggcgac ctgtgctgtg aggaccaagg ttgcactggg ccggtctttt    1020
   atagcgcctc acaccagcta gctcagtctc aggcagcatg catggagatg gagccaatct    1080
   tgccatggca cccaacaacg cgcgcctacc ggataaatta gaaagaatca tggaagcaca    1140
   gtacggagta gtagtgtagt gtggcacgca ccacttgcag tttcttgttg gtgatatgat    1200
   gatgatcata aagctgggca tatgcatgtc aatcacatgc tgcatgcagc agcactggca    1260
   ctaatgagta gtgatgtctc taaaaagtac acccaccatt cacaaatact aacaccattg    1320
   atttaaaaaa aactttaaac actcgtctta tataaaatat aaaatataaa attttaagtt    1380
   ataacttatt tttctaataa gacgagtgat aaaaaattta aaaagaacgg tgtcatatat    1440
   ttatgaacgg atgaagtata ggacaacatt gatttttttt aaaaaaaaaa ttaatcactc    1500
   a                                                                    1501
<210> 34
   <211> 1501
   <212> DNA
   <213> Zea mays
<400> 34
   ccctatgcaa acctagatta aatttatagt gggatttaac ccaaataatg catgccccgc      60
   taatgggtga tggatttccc ctcataagtt tttaccatat ggtcatttcc tgccatccta     120
   acaacttcta attcatgaat tggattggtt gacataaccc cataaaattg tgggttgggt     180
   aaagcagtta atttgacatg gggttgaggt agatatggga tggaattttt gttttaagag     240
   caatggcatc agctctacta tttattaatt taaaagggaa aacaaatagt tcataaaatt     300
   gtgtagagta gaagctagct agctagcatt ggtcagaata agcaggacac acctgggtga     360
   gagaagagag cttattcttc caatccccaa tcacaatctt tacagtgtcg tcaagcaaaa     420
   acccaatcac tattgctgct acgacaccac cccaaagaga acaataattt tgtcctagct     480
   aggtggaact ggaagcggaa ttaatctatc gacacggccg gactcgatcg atcggcgcgg     540
   gcacgcacgc gatcgtcagt gatggtggtg gtcgtccccg gcggcggcgg cggcgccgac     600
   gatgtgagcg aactcggtct cggaggcgca ggggatggtg aggccgccgc agcggtggtc     660
   gaagccgaac tcgtcctcca cccgcttcag cagcgtgacg aaggccgggt ggctgaggca     720
   cgccgtcggg atgaccagcc gcttccgccc ctcgcccacg tacaccgcca ggtggccccg     780
   cggcacgtcc gccgtcgccg acgatcgggt cctcgcgagg tgcagcctcg tcatcagctg     840
   ctgccccagc tttcccgccg ccatctctag ctctagctgt gtgtgtcggt gattgttgca     900
   caaagtcgtg tgtatagctc tagcttgcta tagctagagt ggtgctgcta gatttggagc     960
   tcaagagctt tgtgtggcga cctgtgctgt gaggaccaag gttgcactgg gccggtcttt    1020
   tatagcgcct cacaccagct agctcagtct caggcagcat gcatggagat ggagccaatc    1080
   ttgccatggc acccaacaac gcgcgcctac cggataaatt agaaataatc atggaagcac    1140
   agtacggagt agtagtgtaa tggcacgcac cacttgcagt ttcttgttgg tgatatgatg    1200
   atgatcataa agctgggcat atgcatgtca atcacatgct gcatgcagca gcactggcac    1260
   taatgagtag tgatgtctct aaaaagtaca cccaccattc acaaatacta acaccattga    1320
   tttaaaaaaa actttaaaca ctcgtcttat ataaaatata aaatataaaa ttttaagtta    1380
   taacttattt ttctaataag acgagtgata aaaaatttaa aaagaacggt gtcatatatt    1440
   tatgaacgga tgaagtatag gacaacattg atttttttta aaaaaaaaat taatcactca    1500
   t                                                                    1501
<210> 35
   <211> 507
   <212> DNA
   <213> Zea mays
<400> 35
   atgcgagtca tatgcgttgt tgctagatac atgtctaatc ctggtaaaga gcattggaaa        60
   gctgttcagt ggattttcag atatctacgt ggttcttcta gtgcttgttt atgttttggt       120
   aaatctggag atggtctgat tggctatgtt gattcagatt atggtggtga tttagacagg       180
   aggagatctc tttcaggtta tgtctttact attggagatt gtgctgtgag ttggaaagct       240
   cgtttacagg atactgttgc tttgtctacc acagaagctg aatatatggc aattgcagaa       300
   gttactaagg aagccttatg gttgaaaggt atatattcag agctatgtgg aattaagtct       360
   tgcattacca tctattgtga tagccagagt gccattcatc tcaccaaaga tcagatgatt       420
   actgagaaat ccaaacatat tgatattcgt tatcacttca ttcgtgatat cattggagaa       480
   cgtgtatttg cacagcagtt taaatga                                           507
<210> 36
   <211> 30
   <212> DNA
   <213> Zea mays
<400> 36
   agctatacac acgactttgt gcaacaatca 30
<210> 37
   <211> 30
   <212> DNA
   <213> Zea mays
<400> 37
   cgacacacac agctagagct agagatggcg 30
<210> 38
   <211> 509
   <212> DNA
   <213> Artificial Sequence
<220>
   <223> SAUR31 mutant
<220>
   <221> 5'UTR
   <222> (310)..(435)
<220>
   <221> mutation
   <222> (334)..(334)
<400> 38
   tcagtgatgg tggtggtcgt ccccggcggc ggcggcggcg ccgacgatgt gagcgaactc        60
   ggtctcggag gcgcagggga tggtgaggcc gccgcagcgg tggtcgaagc cgaactcgtc       120
   ctccacccgc ttgagcagcg tgacgaaggc cgggtggctg aggcacgccg tcgggatgac       180
   cagccgcttc cgcccctcgc ccacgtacac cgccaggtgg ccccgcggca cgtccgccgt      240
   cgccgacgat cgggtcctcg cgaggtgcag cctcgtcatc agctgctgcc ccagctttcc      300
   cgccgccatc tctagctcta gctgtgtgtg tcgatgattg ttgcacaaag tcgtgtgtat      360
   agctctagct tgctatagct agagtggtgc tgctagattt ggagctcaag agctttgtgt      420
   ggcgacctgt gctgtgagga ccaaggttgc actgggccgg tcttttatag cgcctcacac      480
   cagctagctc agtctcaggc agcatgcat                                       509